(19) **Europäisches Patentamt / European Patent Office / Office européen des brevets**

(11) **EP 4 313 149 B1**

(12) # EUROPEAN PATENT SPECIFICATION

(45) Date of publication and mention
of the grant of the patent:
**08.07.2026 Bulletin 2026/28**

(21) Application number: **22720561.4**

(22) Date of filing: **30.03.2022**

(51) International Patent Classification (IPC):
**A61K 41/00** (2020.01)    **A61P 25/00** (2006.01)

(52) Cooperative Patent Classification (CPC):
**A61K 41/0028; A61P 25/00**

(86) International application number:
**PCT/EP2022/058410**

(87) International publication number:
**WO 2022/207704 (06.10.2022 Gazette 2022/40)**

(54) **TREATMENT OF THE CENTRAL NERVOUS SYSTEM**

BEHANDLUNG DES ZENTRALNERVENSYSTEMS

TRAITEMENT DU SYSTÈME NERVEUX CENTRAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2021 GB 202104590**

(43) Date of publication of application:
**07.02.2024 Bulletin 2024/06**

(73) Proprietor: **Act Therapeutics Ltd
Newmarket, Suffolk CB8 8LB (GB)**

(72) Inventors:
• **KVÅLE, Svein**
**1435 Ås (NO)**
• **SONTUM, Per Christian**
**0259 Oslo (NO)**
• **KOTOPOULIS, Spiros**
**2040 Kløfta (NO)**
• **HEALEY, Andrew John**
**1528 Moss (NO)**
• **MÜHLENPFORDT, Melina**
**7040 Trondheim (NO)**

(74) Representative: **Bryn Aarflot AS
Patent
Stortingsgata 8
0161 Oslo (NO)**

(56) References cited:
**WO-A1-2015/047103    WO-A1-2017/080481**

• **SONTUM PER ET AL: "Acoustic Cluster Therapy (ACT) - A novel concept for ultrasound mediated, targeted drug delivery", INTERNATIONAL JOURNAL OF PHARMACEUTICS, ELSEVIER, AMSTERDAM, NL, vol. 495, no. 2, 25 September 2015 (2015-09-25), pages 1019 - 1027, XP029292204, ISSN: 0378-5173, DOI: 10.1016/J.IJPHARM.2015.09.047**
• **BUSH NIGEL ET AL: "Theranostic Attributes of Acoustic Cluster Therapy and Its Use for Enhancing the Effectiveness of Liposomal Doxorubicin Treatment of Human Triple Negative Breast Cancer in Mice", FRONTIERS IN PHARMACOLOGY, vol. 11, 20 February 2020 (2020-02-20), XP055926534, DOI: 10.3389/fphar.2020.00075**
• **BUSH NIGEL ET AL: "Therapeutic Dose Response of Acoustic Cluster Therapy in Combination With Irinotecan for the Treatment of Human Colon Cancer in Mice", FRONTIERS IN PHARMACOLOGY, vol. 10, 19 November 2019 (2019-11-19), XP055944809, DOI: 10.3389/fphar.2019.01299**
• **ÅSLUND ANDREAS K.O. ET AL: "Efficient Enhancement of Blood-Brain Barrier Permeability Using Acoustic Cluster Therapy (ACT)", THERANOSTICS, vol. 7, no. 1, 1 January 2017 (2017-01-01), AU, pages 23 - 30, XP055926530, ISSN: 1838-7640, Retrieved from the Internet <URL:https://www.ncbi.nlm.nih.gov/pmc/articles/PMC5196882/pdf/thnov07p0023.pdf> DOI: 10.7150/thno.16577**

EP 4 313 149 B1

- MAINPRIZE TODD ET AL: "Blood-Brain Barrier Opening in Primary Brain Tumors with Non-invasive MR-Guided Focused Ultrasound: A Clinical Safety and Feasibility Study", SCIENTIFIC REPORTS, vol. 9, no. 1, 1 December 2019 (2019-12-01), XP055944888, Retrieved from the Internet <URL:https://www.nature.com/articles/s41598-018-36340-0.pdf> DOI: 10.1038/s41598-018-36340-0
- GOLDWIRT LAURIANE ET AL: "Enhanced brain distribution of carboplatin in a primate model after blood-brain barrier disruption using an implantable ultrasound device", CANCER CHEMOTHERAPY AND PHARMACOLOGY, SPRINGER VERLAG , BERLIN, DE, vol. 77, no. 1, 8 December 2015 (2015-12-08), pages 211 - 216, XP035872917, ISSN: 0344-5704, [retrieved on 20151208], DOI: 10.1007/S00280-015-2930-5
- WEI KUO-CHEN ET AL: "Focused Ultrasound-Induced Blood-Brain Barrier Opening to Enhance Temozolomide Delivery for Glioblastoma Treatment: A Preclinical Study", PLOS ONE, vol. 8, no. 3, 1 March 2013 (2013-03-01), pages e58995, XP055945229, Retrieved from the Internet <URL:https://journals.plos.org/plosone/article/file?id=10.1371/journal.pone.0058995&type=printable> DOI: 10.1371/journal.pone.0058995
- CARPENTIER ALEXANDRE ET AL: "Clinical trial of blood-brain barrier disruption by pulsed ultrasound", SCIENCE TRANSLATIONAL MEDICINE, vol. 8, no. 343, 15 June 2016 (2016-06-15), XP055945235, ISSN: 1946-6234, DOI: 10.1126/scitranslmed.aaf6086
- KEVIN BECCARIA ET AL: "Ultrasound induced opening of the blood-brain barrier to enhance temozolomide and irinotecan delivery: an experimental study in rabbits", JOURNAL OF NEUROSURGERY, vol. 124, no. 6, 1 June 2016 (2016-06-01), US, pages 1602 - 1610, XP055616200, ISSN: 0022-3085, DOI: 10.3171/2015.4.JNS142893
- JORDÃO JESSICA F. ET AL: "Antibodies Targeted to the Brain with Image-Guided Focused Ultrasound Reduces Amyloid-? Plaque Load in the TgCRND8 Mouse Model of Alzheimer's Disease", PLOS ONE, vol. 5, no. 5, 1 May 2010 (2010-05-01), pages e10549, XP055834525, Retrieved from the Internet <URL:https://www.ncbi.nlm.nih.gov/pmc/articles/PMC2868024/pdf/pone.0010549.pdf> DOI: 10.1371/journal.pone.0010549
- KINOSHITA M ET AL: "Targeted delivery of antibodies through the blood-brain barrier by MRI-guided focused ultrasound", BIOCHEMICAL AND BIOPHYSICAL RESEARCH COMMUNICATIONS, ELSEVIER, AMSTERDAM NL, vol. 340, no. 4, 24 February 2006 (2006-02-24), pages 1085 - 1090, XP024924157, ISSN: 0006-291X, [retrieved on 20060224], DOI: 10.1016/J.BBRC.2005.12.112
- ZHANG HAIRONG ET AL: "Focused-ultrasound Mediated Anti-Alpha-Synuclein Antibody Delivery for the Treatment of Parkinson's Disease", 2018 IEEE INTERNATIONAL ULTRASONICS SYMPOSIUM (IUS), IEEE, 22 October 2018 (2018-10-22), pages 1 - 4, XP033523076, DOI: 10.1109/ULTSYM.2018.8579677
- BRIGHI CATERINA ET AL: "MR-guided focused ultrasound increases antibody delivery to nonenhancing high-grade glioma", NEURO-ONCOLOGY ADVANCES, vol. 2, no. 1, 5 March 2020 (2020-03-05), XP055945442, Retrieved from the Internet <URL:https://watermark.silverchair.com/vdaa030.pdf?token=AQECAHi208BE49Ooan9kkhW_Ercy7Dm3ZL_9Cf3qfKAc485ysgAAAtgwggLUBgkqhkiG9w0BBwagggLFMIICwQIBADCCAroGCSqGSIb3DQEHATAeBglghkgBZQMEAS4wEQQMtlWUCUkQmqiadT6RAgEQgIICi1HsEhZ9x2wZxZDtfcJDK3tM8zQ_GK-dkW50r_PCau3G7DUpatmdkYN2RusDzFFg_zhT8NgrfBn0e-kqEF-5UaKlDW-j> DOI: 10.1093/noajnl/vdaa030
- PIN-YUAN CHEN ET AL: "Focused ultrasound-induced blood?brain barrier opening to enhance interleukin-12 delivery for brain tumor immunotherapy: a preclinical feasibility study", JOURNAL OF TRANSLATIONAL MEDICINE, BIOMED CENTRAL, vol. 13, no. 1, 17 March 2015 (2015-03-17), pages 93, XP021218132, ISSN: 1479-5876, DOI: 10.1186/S12967-015-0451-Y
- CHEN KO-TING ET AL: "Theranostic Strategy of Focused Ultrasound Induced Blood-Brain Barrier Opening for CNS Disease Treatment", FRONTIERS IN PHARMACOLOGY, vol. 10, 7 February 2019 (2019-02-07), XP055944882, Retrieved from the Internet <URL:https://www.frontiersin.org/articles/10.3389/fphar.2019.00086/full> DOI: 10.3389/fphar.2019.00086
- CURLEY COLLEEN T. ET AL: "Focused Ultrasound Immunotherapy for Central Nervous System Pathologies: Challenges and Opportunities", THERANOSTICS, vol. 7, no. 15, 1 January 2017 (2017-01-01), AU, pages 3608 - 3623, XP055945416, ISSN: 1838-7640, Retrieved from the Internet <URL:https://www.thno.org/v07p3608.pdf> DOI: 10.7150/thno.21225

**Description**

**Field of the invention**

The present invention relates to ultrasound (US) mediated delivery of therapeutic agents to the central nervous system (CNS). Thus, the invention provides a cluster composition and a pharmaceutical composition, for use in delivery of therapeutic agents to the brain and spinal cord for treatment. The invention further provides compositions and methods to increase permeability of the blood-brain barrier for passage of therapeutic agents to allow for medical treatment of diseases, disorders and injuries of the CNS.

**Background of the invention**

Blood vessels provide the vital infrastructure for delivery of oxygen and essential nutrients throughout the body, and the term "blood-brain barrier" (BBB) is used for describing the unique characteristics of the blood vessels that vascularize the central nervous system (CNS, the brain and the spinal cord). The BBB is not a single physical entity but rather the combined function of a series of physiological properties possessed by endothelial cells that limit vessel permeability. The BBB is a highly selective semipermeable border of endothelial cells that prevents solutes in the circulating blood from non-selectively crossing into the extracellular fluid of the central nervous system where neurons reside. The blood-brain barrier is formed by endothelial cells of the capillary wall, astrocyte end-feet ensheathing the capillary, and pericytes embedded in the capillary basement membrane. This system allows the passage of some selected, small molecules by passive diffusion, as well as the selective and active transport of various nutrients, ions, organic anions, glucose, water and amino acids that are crucial to neural function. The blood-brain barrier restricts the passage of pathogens, the diffusion of solutes in the blood, and large or hydrophilic molecules into the cerebrospinal fluid, while allowing the diffusion of small hydrophobic molecules and small polar molecules. The barrier also restricts the passage of peripheral immune factors, like signalling molecules, antibodies, and immune cells, into the CNS, thus insulating the brain from damage due to peripheral immune events.

The BBB is a major obstacle in drug delivery to the brain imposing size and biochemical restrictions on the passage of molecules, and currently there are no standardized methods to penetrate it. The BBB maintains the homeostasis of the brain and protects it from unwanted or harmful substances. Unfortunately, the BBB also blocks >98% of small drugs and all larger therapeutic molecules from entering the brain, unless active transport of the substances is possible. For a small-molecule drug to cross the BBB in pharmacologically significant amounts, the molecule must both have a small size and a high lipid solubility. Thus, the presence of an intact BBB limits the distribution of most therapeutic agents, including anti-cancer and anti-viral drugs, as well as novel therapeutic approaches that do not translate to clinical practice because of this biological barrier. For this reason, many diseases, neurological disorders and physical injuries remain untreatable. Various strategies to overcome the BBB have been investigated, including direct intracranial infusion, convection enhanced delivery, diuretic agents, and biomimetics. These approaches have been limited by lack of specificity, safety concerns, and a failure to achieve adequate concentrations of delivered compounds to sufficient volumes of brain tissue. A safe and effective means of bypassing the barrier temporarily could aid in delivering even large molecules, such as antibodies and growth factors, directly to brain pathology.

Since its introduction in 2001, focused ultrasound (FUS) in combination with microbubbles has been explored to increase the permeability of the BBB in various pre-clinical settings. In brief, insonation of the vascular compartment containing administered microbubbles leads to a variety of biomechanical effects that increase the permeability of the endothelial barrier both paracellularly and transcellularly. The increased permeability leads to enhanced extravasation, distribution and uptake of drug molecules in the target tissue. This approach is currently being evaluated in clinical trials (ClinicalTrials.gov identifier NCT02343991, NCT02253212 and NCT03626896). Most studies using FUS for BBB disruption (BBBD) are employing commercial ultrasound contrast agent microbubbles, such as Definity®, Sonovue® or Optison®, all of which are primarily designed and optimized for diagnostic purposes. To improve treatment strategies, incorporation of drugs into custom-made microbubbles has also been investigated. Whereas the concept clearly holds merit, it also has limitations. The microbubbles need to be close to the endothelial wall to maximize biomechanical effects. However, regular contrast microbubbles are small (1-3 $\mu$m), and their average distance to the vessel wall is often too large to induce a significant biomechanical effect. Furthermore, the circulation lifetime of most microbubbles is typically in the order of 2-3 minutes, thus limiting the exposure time. Microbubble and ultrasound (US) mediated delivery mechanisms have been clearly demonstrated *in vivo* [Chen, K-Ting et al., Theranostic Strategy of Focused Ultrasound Induced Blood-Brain Barrier Opening for CNS Disease Treatment, Frontiers in Pharmacology, V10, 2019]. However, the typical need for applying high power US when using regular contrast microbubbles induce bio-effects that raise safety issues for the approach. Rather violent inertial cavitation mechanisms, where the microbubbles implode are involved, and in particular micro-haemorrhage and irreversible vascular damage has been observed. These processes may also lead to vascular shut down, i.e. blood vessels collapsing (transiently or permanently), effectively stopping blood perfusion with potentially significant clinical safety consequences as well as no uptake of drugs. Although conventional microbubbles have shown promise for opening the BBB and for drug delivery to the brain, these microbubbles were developed for diagnostic imaging, not for therapy. Microbubble formulations designed for therapy are likely to be needed to enable optimal treatment regimens.

In WO2015/047103, a concept for ultrasound mediated, targeted delivery is proposed, wherein a microbubble-microdroplet cluster composition is administered alongside a therapeutic agent and where ultrasound insonation of a targeted pathology may lead to an increase in the therapeutic effect versus the therapeutic agent alone. This concept, termed as Acoustic Cluster Therapy (ACT Sonoporation or ACT), has later been investigated in a series of pre-clinical proof of principal and proof of concept studies. For example, Åslund et al. (2017) Efficient enhancement of blood-brain barrier permeability using Acoustic Cluster Therapy (ACT), Theranostics 2017; 7:23, which is considered to be the closest prior art, provides results from a small pilot study in healthy rats showing increased BBB permeability after ACT, leading to increased accumulation of molecular model drugs. Within this study, Åslund's ultrasound procedure comprises applying US at a single frequency (1MHz) at two different mechanical indices (MIs; 0.28 for an Activation phase and 0.09 for a subsequent Enhancement phase).

There is hence a need for new and alternative compositions and methods for delivery of therapeutic agents to the brain, and for treatment of subjects with CNS diseases, disorders or injuries. The inventors have investigated further the potential of ACT for treating brain diseases and disorders and identified a specific use of cluster compositions for delivery and treatment of CNS diseases and disorders.

## Brief summary of the invention

The invention relates to compositions and method for ultrasound mediated delivery of therapeutic agents to the central nervous system (CNS) and to treatment of diseases, disorders and injuries of the CNS. The inventors have discovered that Acoustic Cluster Therapy (ACT®) can be used to increase the permeability of the BBB to allow for safe passage of therapeutic agents to the CNS. ACT, presented in WO2015/047103, is a concept for ultrasound mediated, targeted delivery, wherein a microbubble-microdroplet cluster composition is administered with a therapeutic agent and wherein ultrasound insonation of a targeted pathology may lead to an increase in the therapeutic effect versus using just the therapeutic agent alone. Contrasting the available treatment methods for the CNS, the compositions and methods of the invention have shown to increase BBB permeability safely and transiently, and to provide increased drug delivery across the BBB, likely to improve therapeutic efficacy. To achieve an optimal therapeutic outcome using microbubbles and ultrasound (US), with regards to frequency and power (described as the mechanical index, MI), the US field needs to be precisely regulated. However, controlled US insonation of the CNS is severely hampered by bone structures, hence, the invention discloses and details several options for configuring US devices to enable necessary control with US targeting and dosimetry. Furthermore, it has been identified that a specific use wherein the ultrasound insonation frequencies of the method are carefully selected, have provided an increased BBB permeability of the co-administered therapeutic agent, fully exploiting the potential of ACT. Further, the inventors have combined the ACT technology with clinically relevant drug carriers and identified that with the compositions and methods of the invention, even large therapeutic agents can be used in CNS therapy, as a crossing of the BBB is enabled. The potential of ACT to increase the permeability of the BBB and improve delivery of clinically relevant drug carriers across the BBB is demonstrated herein.

Accordingly, the present invention provides a microbubble-microdroplet cluster composition for use in treatment of CNS diseases, disorders or injuries of a subject. Likewise, the invention provides a method for treatment of CNS diseases, disorder or injuries comprising the administration of a microbubble-microdroplet cluster composition to a subject. The present disclosure demonstrates that a two component, microbubble-microdroplet cluster composition, wherein microbubbles as a first component are physically attached to microdroplets, as a second component, in clusters, can be used in a method to increase the BBB permeability, and provide increased delivery of a therapeutic agent across the BBB providing improved therapeutic efficacy of the therapeutic agent.

In one aspect, the invention provides a pharmaceutical composition comprising a microbubble-microdroplet cluster composition and at least one therapeutic agent for use in a method of treatment of diseases, disorders or injuries of the central nervous system (CNS) of a subject, wherein the use comprises Acoustic Cluster Therapy (ACT) treatment wherein at least one step of ultrasound insonation is performed either non-invasively towards the CNS, invasively towards the CNS or towards the heart or a spinal or carotid artery outside the CNS, comprising the steps of:

(i) administering the pharmaceutical composition to the subject; wherein the at least one therapeutic agent is pre-, and/or co- and/or post administered separate to the cluster composition;
(ii) optionally imaging the clusters of the pharmaceutical composition using ultrasound imaging to identify a region of interest in the CNS for treatment within said subject;
(iii) activating a phase shift of a diffusible component of the microdroplet of the cluster composition from step (i) by ultrasound insonation at a first frequency and a first mechanical index; wherein

a) for the non-invasive insonation, this takes place from at least one transducer positioned outside the brain skull or vertebral column of the subject for insonation through this; and

b) for the invasive insonation, this takes place from at least one surgically implanted transducer in the subject's brain skull or vertebral column; and

c) for the insonation towards the heart or a spinal or carotid artery, this takes place in the heart or in a spinal or carotid artery outside the CNS;

(iv) further insonating with ultrasound at a second frequency which is lower than the first frequency, and a second mechanical index, facilitating extravasation of the at least one therapeutic agent administered in step (i) at the region of interest in the CNS; wherein

a) for the non-invasive insonation, this takes place from at least one transducer positioned outside the brain skull or vertebral column of the subject for insonation through this; and

b) for the invasive insonation, this takes place from at least one surgically implanted transducer in the subject's brain skull or vertebral column;

c) when activating according to c) in step (iii), further insonation in this step takes place either non-invasively or invasively according to step (iv) a) or step (iv) b).

Further disclosed herein but not encompassed by the wording of the claims, is a method of treatment of CNS diseases, disorders or injuries of a subject, wherein the method comprises Acoustic Cluster Therapy (ACT) treatment wherein at least one step of ultrasound insonation is performed either non-invasively towards the CNS, invasively towards the CNS or towards the heart or a spinal or carotid artery outside the CNS, wherein the method comprises the steps of:

(i) administering a pharmaceutical composition comprising a microbubble-microdroplet cluster composition to the subject; wherein at least one therapeutic agent is pre-, and/or co- and/or post administered separate to the cluster composition;

(ii) optionally imaging the clusters of the pharmaceutical composition using ultrasound imaging to identify a region of interest in the CNS for treatment within said subject;

(iii) activating a phase shift of a diffusible component of the microdroplet of the cluster composition from step (i) by ultrasound insonation at a first frequency and a first mechanical index; wherein

a) for the non-invasive insonation, this takes place from at least one transducer positioned outside the brain skull or vertebral column of the subject for insonation through this; and

b) for the invasive insonation, this takes place from at least one surgically implanted transducer in the subject's brain skull or vertebral column; and

c) for the insonation towards the heart or a spinal or carotid artery, this takes place in the heart or in a spinal or carotid artery outside the CNS;

(iv) further insonating with ultrasound at a second frequency and a second mechanical index facilitating extravasation of the at least one therapeutic agent administered in step (i) at the region of interest in the CNS; wherein

a) for the non-invasive insonation, this takes place from at least one transducer positioned outside the brain skull or vertebral column of the subject for insonation through this; and

b) for the invasive insonation, this takes place from at least one surgically implanted transducer in the subject's brain skull or vertebral column;

c) when activating according to c) in step (iii), further insonation in this step takes place either non-invasively or invasively according to step (iv) a) or step (iv) b).

## Brief description of the drawings

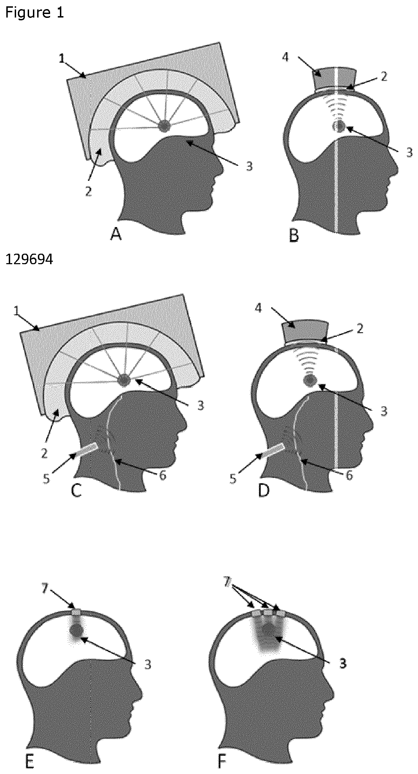

Figure 1 provides visualisations of various ultrasound devices and set-ups for activation and enhancement insonation pertaining to ACT treatment of the CNS. The exemplified transducers may also be used for imaging. Panels A: Extracranial hemispheric (non-invasive) focused ultrasound arrays (such as ExAblate® and NaviFUS®) for both activation and enhancement insonation, B: Extracranial (non-invasive) mono-element focused device for both activation and enhancement insonation, C: Activation insonation performed by external transducer towards the heart or carotid or spinal arteries, combined with extracranial hemispheric focused ultrasound arrays for enhancement insonation, D: Activation insonation performed by external transducer towards the heart or carotid or spinal arteries, combined with extracranial mono-element focused device for enhancement insonation, E: Implantable (invasive), unfocused single-emitter US device (such as SonoCloud®-1) and F: Implantable, unfocused 9-emitter US device (such as SonoCloud®-9). Numbers 1: Helmet with multiple ultrasound transducers, 2: water or gel for optimal coupling of ultrasound field, 3: target/region of interest to be insonated, 4: single element or array ultrasound transducer, 5: single element or array ultrasound transducer for activation of cluster composition in e.g. carotid artery, 6: carotid arteries to the brain.7: single element or array ultrasound transducer.

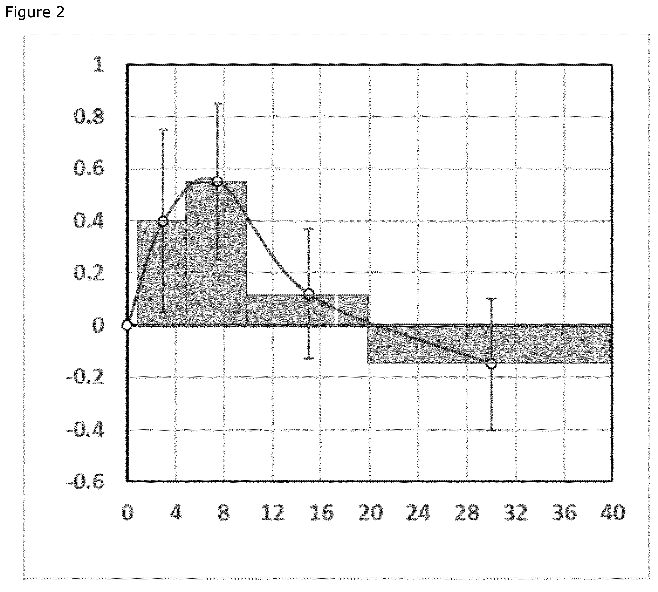

Figure 2 provides a visualization of cluster size versus in-vivo product efficacy, wherein the Y-axis shows the calculated correlation coefficient for Grey Scale enhancement from US imaging (i.e. amount of bubbles deposited after activation) and the X-axis shows cluster diameter in $\mu$m.

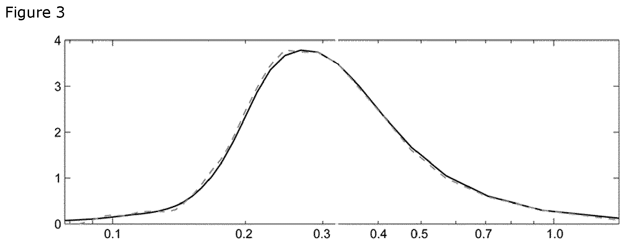

Figure 3 provides the attenuation spectrum of a population of large bubbles after ultrasound activation of the cluster composition. Y-axis shows attenuation in dB/cm. X-axis shows frequency in MHz.

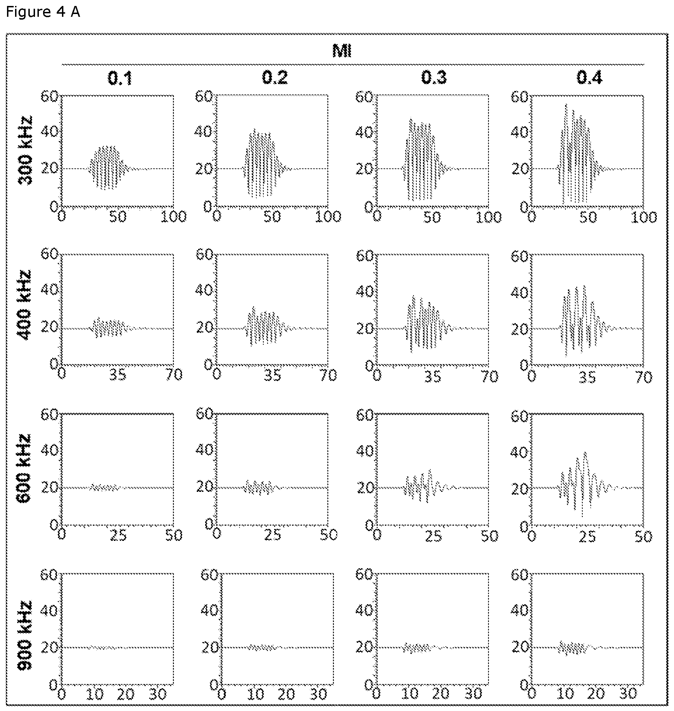

Figure 4 A provides results from modelling of activated bubble response to the low frequency Enhancement insonation field for various frequencies and mechanical indices (MIs) of the incident US field. Bubble diameter at rest is 20 $\mu$m and the incident field consisted of 8 cycles with a frequency and MI as stated in each panel. Y-axis shows the diameter of the activated bubble in $\mu$m. X-axis shows time in $\mu$-seconds.

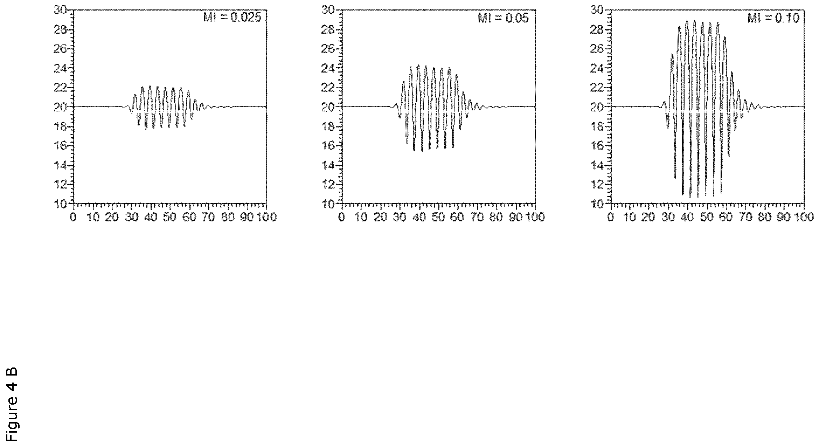

Figure 4 B provides results from modelling of activated bubble response at a low frequency Enhancement insonation field of 0.25 MHz with various mechanical indices (MIs). Bubble diameter at rest is 20 $\mu$m and the incident field consisted of 8 cycles with a frequency and MI as stated in each panel. Y-axis shows the diameter of the activated bubble in $\mu$m. X-axis shows time in $\mu$-seconds.

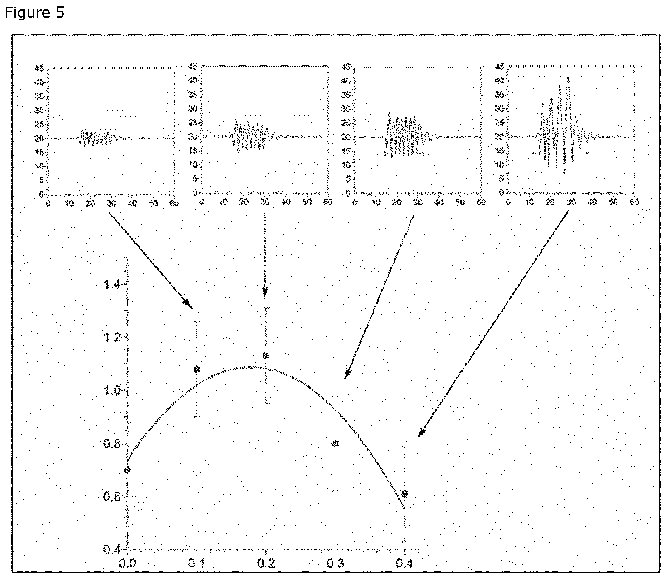

Figure 5 provides results of tumour specific uptake of a fluorescent dye (Evans Blue) upon ACT treatment with an Enhancement step insonation field at 0.500 MHz, with mechanical indices (MIs) at 0, 0.1, 0.2, 0.3 and 0.4 (lower panel). Y-axis shows tumour specific uptake in mg Evans Blue/mg tumour tissue. X-axis shows mechanical index. The four upper panels show results from modelling of activated bubble response to the incident US field at the different MIs investigated. Y-axis shows the diameter of the activated bubble in $\mu$m. X-axis shows time in $\mu$-seconds.

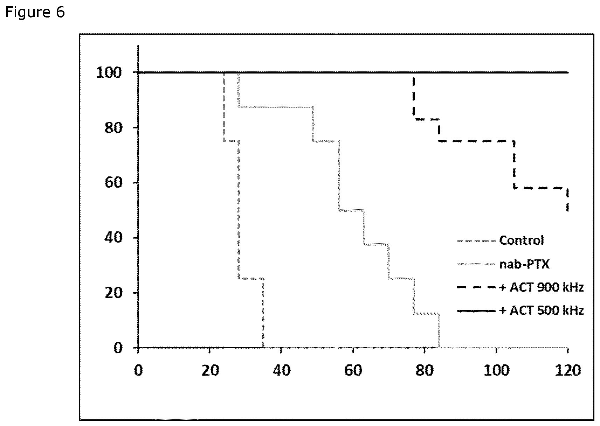

Figure 6 provides results for the therapeutic efficacy of nab-paclitaxel (nab-PTX) $\pm$ ACT for treatment of prostate cancer in mice. Y-axis show overall survival in % of all treated animals. X-axis shows time in days after study start. Groups: saline control (dotted grey line),: nab-PTX alone,: nab-PTX + ACT with Enhancement field 0.5 MHz (MI 0.2) (dotted black line) and,: nab-PTX + ACT with Enhancement field 0.9 MHz (MI 0.2) (solid grey line).

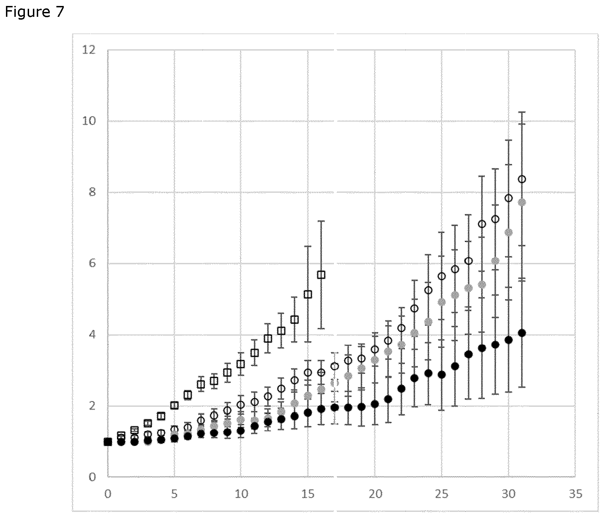

Figure 7 provides results of therapeutic efficacy of nab-paclitaxel $\pm$ ACT for treatment of breast cancer in mice. Y-axis show normalized tumour diameter. X-axis shows time after study start, in days. Groups: saline control (black, open squares), nab-PTX alone (black, open circles), nab-PTX + ACT with Enhancement field MI 0.1 (0.5 MHz) (grey, closed circles) and nab-PTX + ACT with Enhancement field MI 0.2 (0.5 MHz) (black, closed circles).

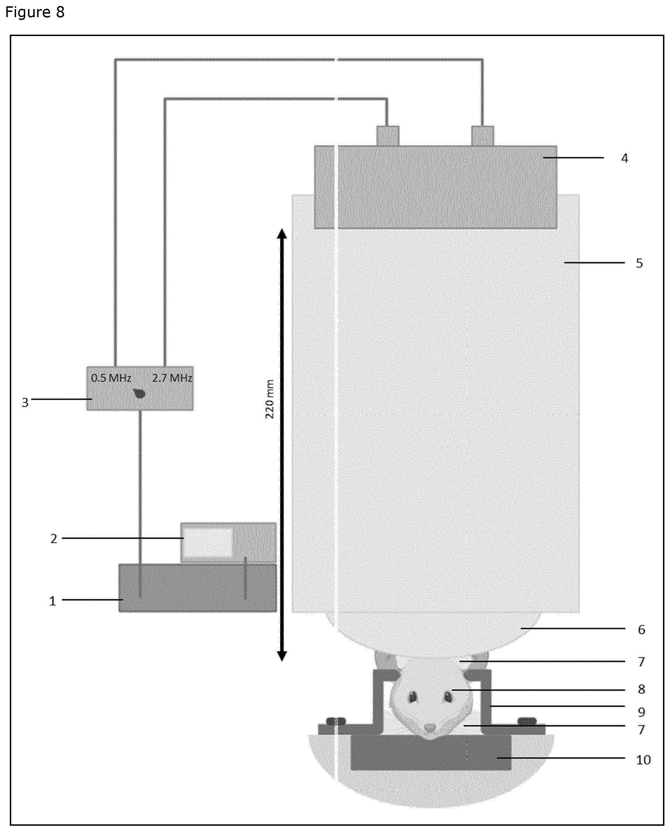

Figure 8 provides a set up sketch of the apparatus used in the study of Example 4 for application of the ACT Sonoporation procedure comprising ultrasound activation and enhancement. The numbers denote the following: 1 - Amplifier, 2 - Signal generator, 3 - Switch box for frequencies (between 0.5 and 2.7 MHz), 4 - Dual frequency transducer, 5 - Water filled cone, 6 - Water filled bag, 7 - Ultrasound gel, 8 - Mouse in prone position, 9 - Ear bar, 10 - Acoustic absorber pad.

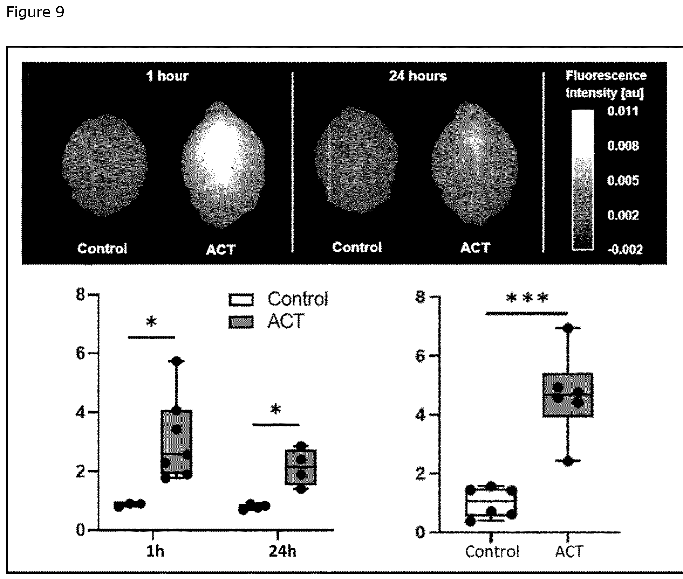

Figure 9 provides a box and whiskers plot of the results from the study of Example 4: ACT induced delivery of nanoparticles across the Blood-brain Barrier. Upper panel: representative pictures from near infrared fluorescence (NIRF) imaging of uptake of core-crosslinked polymeric micelles (CCPM) to brain tissue. Control and ACT treated brains at 1 and 24 hours after ACT treatment. Lower left panel: Y-axis shows uptake as measured by NIRF as percent of injected dose per grams of brain tissue for control and ACT groups, at 1 and 24 hours after ACT treatment. Black, filled circles represent individual observations. Line and asterisk indicate statistical significance (***$p < 0.001$) between groups derived from a t-test. Lower right panel: Y-axis shows uptake as measured by confocal microscopy as percent of brain area containing CCPMs, for control and ACT groups, 1 hour after ACT treatment. Black, filled circles represent individual observations. Line and asterisk indicate statistical significance (*$p < 0.05$) between groups derived from a Mann-Whitney Rank sum test.

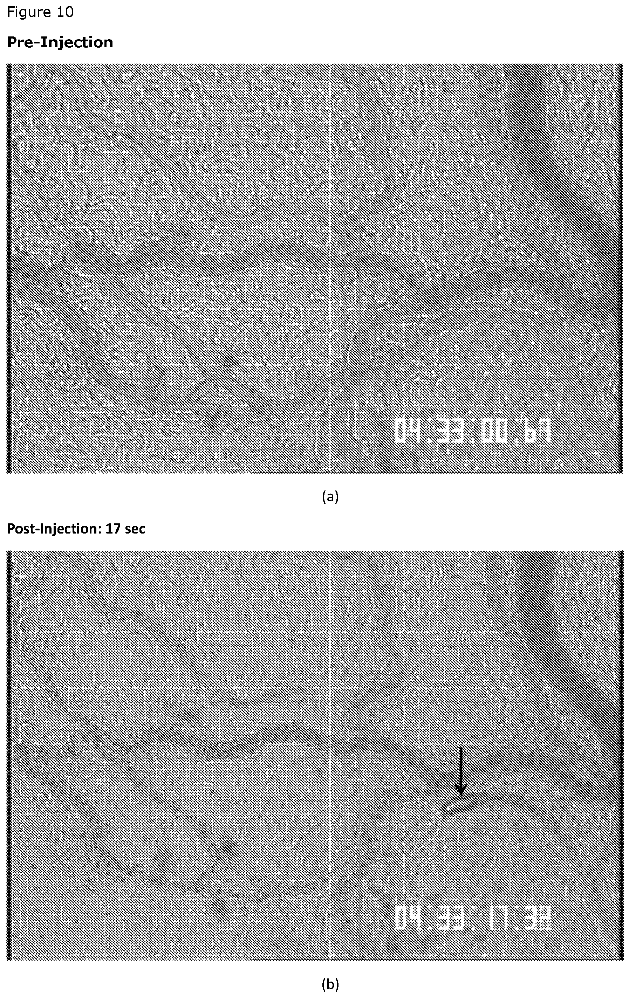

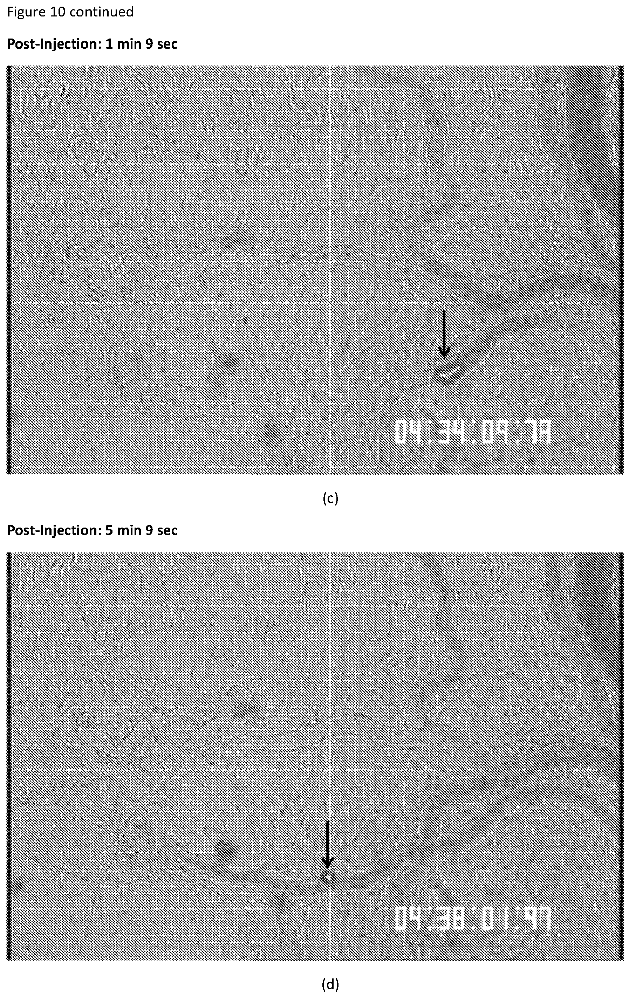

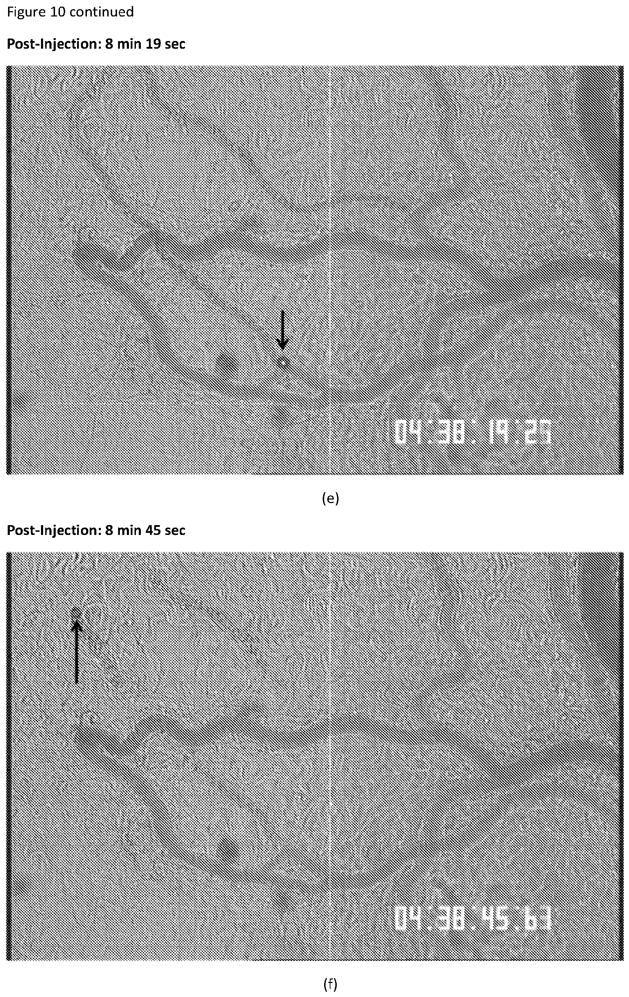

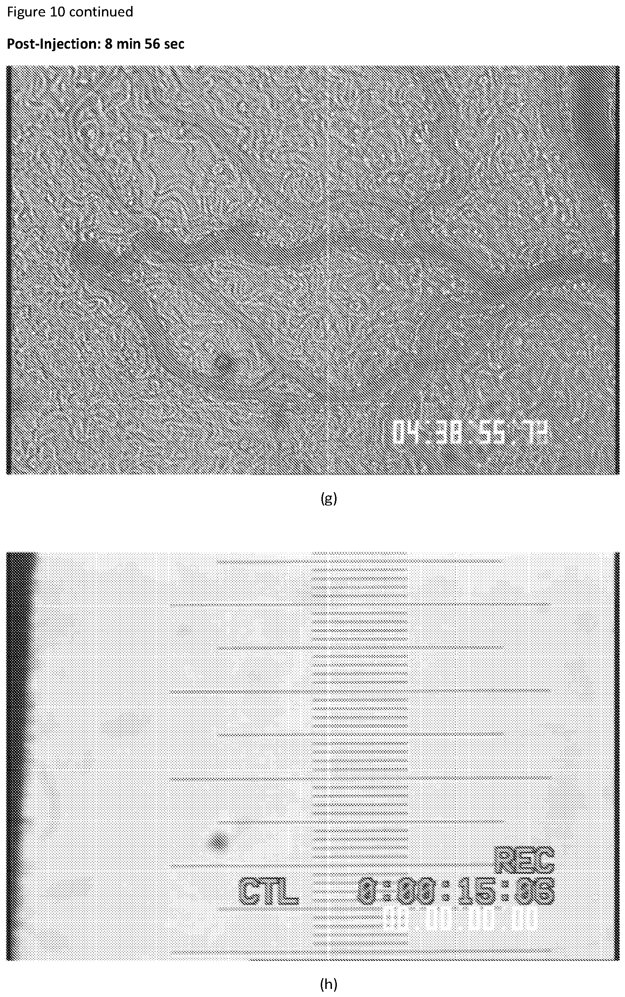

Figure 10 provides results from Example 4: intravital microscopy of activated bubbles in a rat mesentery artery. Video frames of an activated phase shift bubble in the mesentery (indicated by black arrows) at; (a) pre-injection, (b) 17 seconds post-injection in a micro vessel, blocking blood flow; (c) at 1 minutes and 9 seconds; (d) at 5 minutes and 9 seconds; (e) at 8 minutes and 19 seconds; (f) at 8 minutes and 45 seconds and (g) at 8 minutes and 56 seconds, respectively. The activated phase shift bubble (indicated by the arrow) gradually shrinks and advances in the micro vessel by intermittent lodging and dislodging, before it has cleared completely after 8 minutes and 56 seconds. Figure 10 (h) shows a scale bar with minor units of 10 $\mu$m.

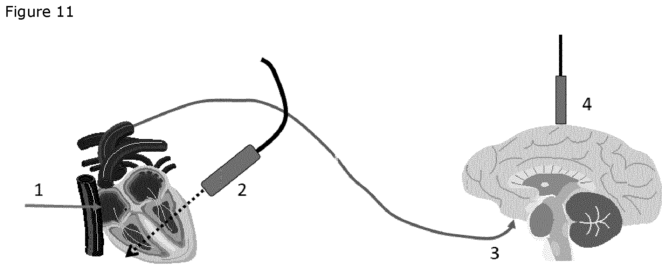

Figure 11 provides a schematic representation of the experimental setup for Example 5: deposition of activated large bubbles in the brain after activation of clusters in the heart. Numbers 1: i.v. injection of cluster composition, 2: transducer for activation in the heart, 3: carotid artery for transport of activated, large bubbles to the brain and 4: imaging transducer for detection of deposition of activated bubbles.

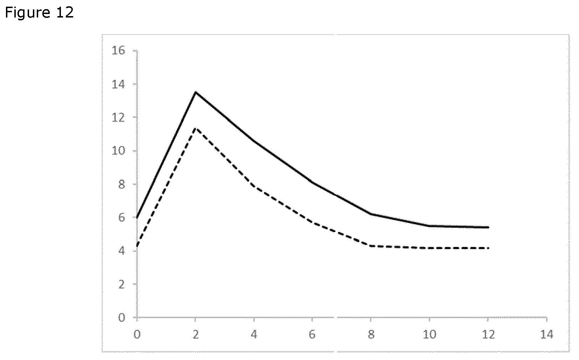

Figure 12 provides results from Example 5: deposition of activated, large bubbles in the brain after activation of clusters in the heart, for two animals (dotted and solid line). Y-axis shows contrast enhancement in the brain (dB). X-axis shows minutes after activation in the heart.

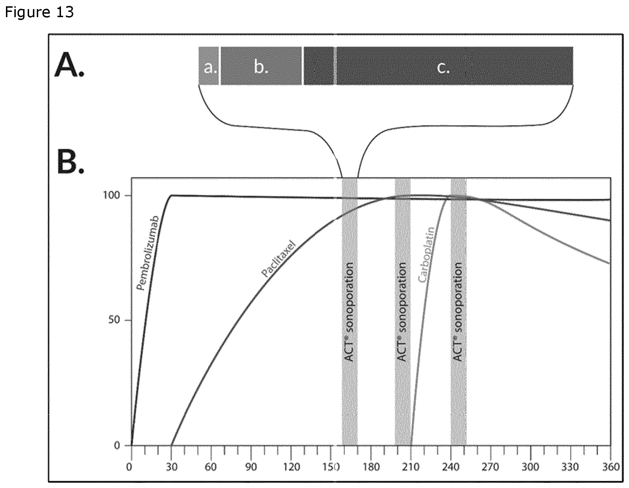

Figure 13 provides a graph of possible ACT treatments performed during treatment with the combination regimen comprising the Standard of Care combination immunotherapy plus chemotherapy regimen for treatment of cancer; pembrolizumab followed by paclitaxel and cisplatin. Panel A: ACT procedure comprising a.: injection of the cluster composition, b.: activation step with e.g. 60 second of ultrasound insonation, and c.: enhancement step with e.g. 5 minutes of ultrasound insonation. Panel B: y-axis showing plasma concentration of the administered therapeutic agents in percent of peak and x-axis showing time in minutes. In this example three ACT procedures are performed at approximately 160 minutes, 200 minutes and 240 minutes in order to cover all three drugs and provide treatment of the entire region of interest.

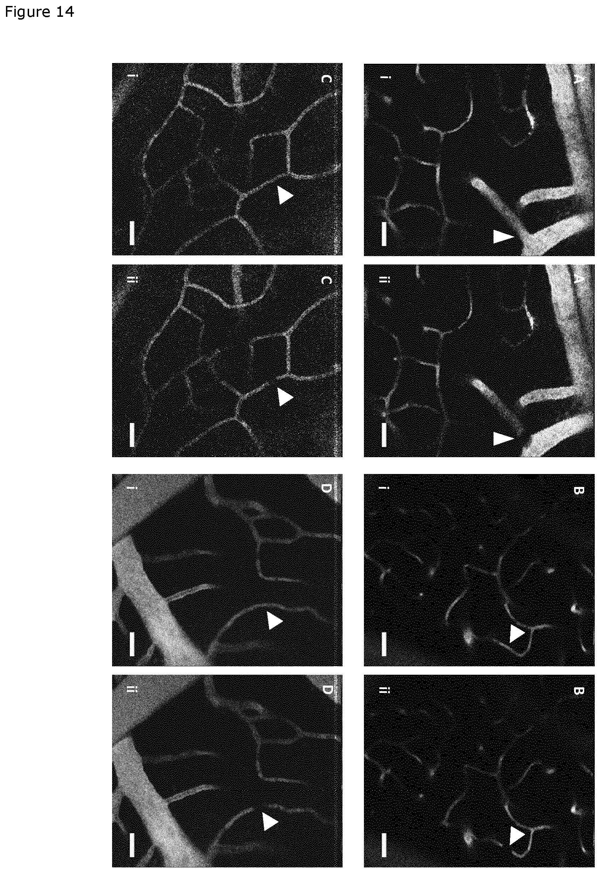

Figure 14 provides snapshots of acquired videos of the vasculature during ACT treatment, for four (A-D) examples of ACT® bubble observations, (i) without and (ii) with an anticipated ACT® bubble. Scale bar represents 50 $\mu$m.

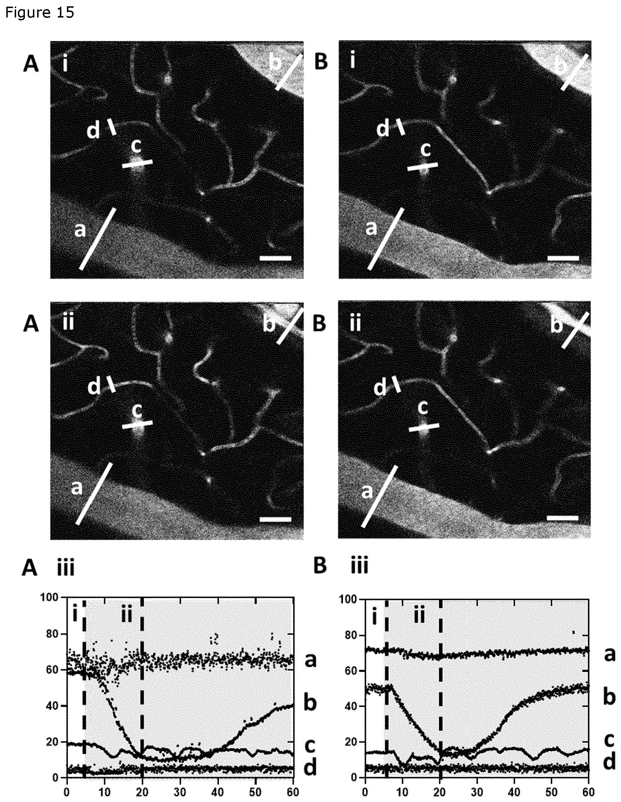

Figure 15 provides snapshots from videos of transient vaso-modulation seen as a reduction and extension of the blood vessel diameter in an animal during a consecutive (A) first and (B) second ACT® treatment. Per panel, the images (i) and (ii) represent two snapshots while the (iii) graph indicates the diameters of the blood vessels of interest over time in the corresponding video. The two vertical dashed lines indicate the timepoint of when the snapshots were taken. Scalebar represents 50 $\mu$m.

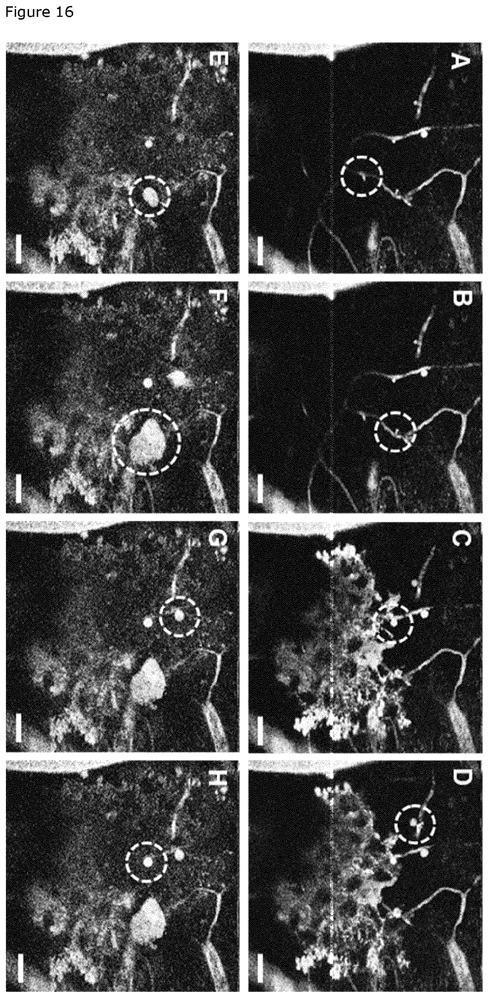

Figure 16 provides video frames of outpouchings as they grow during ultrasound exposure, showing video frames from which the maximum size of each of the (A-H) outpouchings was determined. Dashed circle indicates the outpouchings of interest. Scalebar presents 50 $\mu$m.

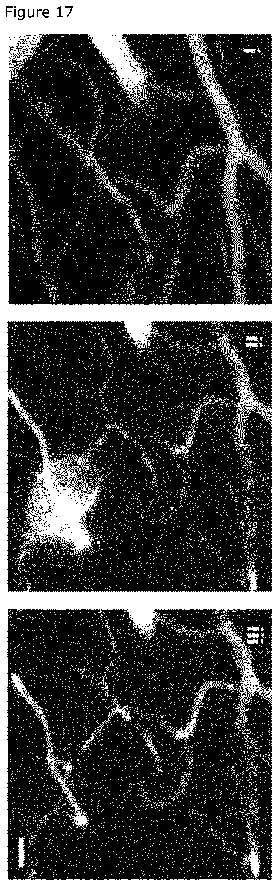

Figure 17 provides maximum projection images of an ACT®-induced structure, as Z-stacks, to compare and assess for ACT-induced effects at different depths. (i) In the pre Z-stack the elliptical structure was not observed. (ii) In the post ACT® treatment Z-stack an elliptical structure appeared including an increased fluorescence intensity in the blood vessel. (iii) Approximately 6 minutes post ACT® treatment the elliptical structure had disappeared. Scalebar represents 25 $\mu$m.

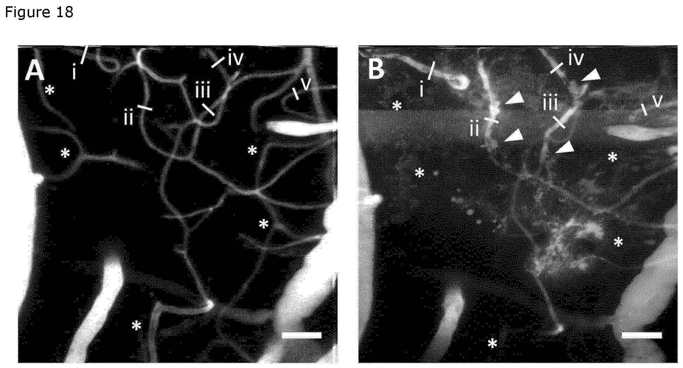

Figure 18 provides pre and post Z-stack maximum projection images of outpouchings taken (A) before treatment and (B) after treatment. Scale bar represents 50 $\mu$m. Short lines mark blood vessels of which the diameter is measured pre and post ACT® treatment. Arrows mark the remaining structures of the outpouchings. The asterisks mark blood vessels which were observed in the pre XYZ-stack but not in the post XYZ-stack.

## Detailed description of the invention

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

**Definitions:**

As used herein, Acoustic Cluster Therapy (ACT), which is further defined below, comprises the administration of a cluster composition (cf. definition below) in conjunction with at least one therapeutic agent and subsequent application of ultrasound to a targeted region of interest.

A used herein, the CNS refer to either of the brain and the spinal cord.

As used herein, "subject" means any human, or non-human animal individual selected for treatment or therapy, and encompasses, and may be limited to, a patient, particularly to a human patient diagnosed with a CNS disease, disorder or injury.

The phrase "therapeutically effective amount" as used herein means the amount of therapeutic agent which is effective for producing the desired therapeutic effect in a subject at a reasonable benefit/risk ratio applicable to any treatment.

The term 'microbubble' or 'regular, contrast microbubble' is used in this text to describe microbubbles with a diameter in the range from 0.2 to 10 microns, typically with a mean diameter between 2 to 3 $\mu$m. 'Regular, contrast microbubbles' include commercially available agents such as Sonazoid™ (GE Healthcare), Optison™ (GE Healthcare), Sonovue™ (Bracco Spa.), Definity™ (Lantheus Medical Imagin), Micromarker™ (VisualSonics Inc.) and Polyson L™ (Miltenyi Biotec GmbH).

The term HEPS/PFB microbubble is used in this text to describe the microbubbles formed by reconstituting a 1st component (as provided in Example 1) with water, such as with 2 mL of water.

The terms 'phase shift bubbles', 'large, phase shift bubbles, 'large, activated bubbles' and 'activated bubbles' in this text is used to describe the large (> 10 $\mu$m) bubbles that form after ultrasound (US) induced activation of the cluster composition.

The term 'microdroplet' is used in this text to describe emulsion microdroplets with a diameter in the range from 0.2 to 10 microns.

'Insonation' or 'US insonation' are terms used to describe exposure to, or treatment with, ultrasound.

The term "regular medical imaging ultrasound" is used to describe ultrasound from of the shelf US scanners and probes intended for medical imaging. I.e. at a frequency between 1 to 10 MHz and an MI of < 1.9, preferably < 0.7 and more preferably < 0.4.

The term 'deposit tracer' is used in this text in relation to the activated phase shift bubbles, in the sense that the temporary mechanical trapping of the large bubbles in the microcirculation implies that the regional deposition of phase shift bubbles in the tissue will reflect the amount of blood that flowed through the microcirculation of the tissue at the time of activated bubble deposition. Thus, the number of trapped 'deposited' phase shift bubbles will be linearly dependent on the tissue perfusion at the time of deposition.

The term 'phase shift (process)' is used in this text to describe the phase transition from the liquid to gaseous states of matter. Specifically, the transition (process) of the change of state from liquid to gas of the oil component of the microdroplets of the cluster composition upon US insonation.

In this text the terms "therapy delivery/therapeutic agent(s)" and "drug delivery/drug(s)" are both understood to include the delivery of drug molecules, nanoparticles and nanoparticle delivery systems/nano carriers, and liposomal delivery systems, including at least one therapeutically active agent, the term also including stem cell delivery.

The term '1st component' (or first component, or C1) is used in this text to describe the dispersed gas (microbubble) component. The term '2nd component' (or second component, or C2) is used in this text to describe the dispersed oil phase (microdroplet) component comprising a diffusible component.

The term 'cluster composition' is used in this text to describe a composition resulting from a combination, such as mixing, of the 1st (microbubble) component and the 2nd (microdroplet) component. Hence, the cluster composition, with characteristics as further described herein, refers to the formulated composition ready for administration to a subject, and for use in the Acoustic Cluster Therapy.

The term "diffusible component" is used in this text to describe a chemical component of the oil phase of the 2nd component that is capable of diffusion *in vivo* into the microbubbles in the 1st component, transiently increasing its size.

The term "pharmaceutical composition" used in this text has its conventional meaning, and in particular is in a form suitable for mammalian administration. The composition preferably comprises two separate compositions; The cluster composition (a), and the therapeutic agent (b), which are both suitable for mammalian administration such as via parenteral injection, intraperitoneal injection or intramuscular injection, either by the same or different administration routes. By the phrase "in a form suitable for mammalian administration" is meant a composition that is sterile, pyrogen-free, lacks compounds which produce excessive toxic or adverse effects, and is formulated at a biocompatible pH (approximately pH 4.0 to 10.5). Such a composition is formulated so that precipitation does not occur on contact with biological fluids (e.g. blood), contain only biologically compatible excipients, and is preferably isotonic.

The term 'Sonometry (system)' in this text refers to an in-vitro measurement system to size and count activated phase shift bubbles dynamically using an acoustic technique.

The term 'Reactivity' is used in this text to describe the ability of the microbubbles in the 1st component and the microdroplets in the 2nd component to form microbubble-microdroplet clusters upon mixing. Coulter counting is suitable for quantification of microbubble and microdroplet concentration and size distribution in C1 and C2, and for characterization of particles in the cluster composition (drug product, DP). Reactivity (R) of the cluster composition defined as;

$$R = (C_{C1} + C_{C2} - C_{DP}) \cdot 100 / (C_{C1} + C_{C2})$$

Where $C_{C1}$, $C_{C2}$ and $C_{DP}$ are the number concentration observed in C1, C2 and DP, respectively. The Reactivity is hence a measure of how many of the individual microbubbles and microdroplets in C1 and C2 that are contained in cluster form in the DP. The Reactivity is also correlated to how large these clusters are (i.e. how many individual microbubbles and microdroplets comprises a single cluster). From Coulter analysis of C1, C2 and DP, the Reactivity can easily be calculated.

The terms "microbubble-microdroplet cluster" or "cluster" in this text refers to groups of microbubbles and microdroplets permanently held together by electrostatic attractive forces, in a single particle, agglomerated entity. The term 'clustering' in this text refers to the process where microbubbles in the 1st component and microdroplets of the 2nd component form clusters.

Within medical ultrasound, acoustic power is normally described by "the Mechanical Index" (MI). This parameter is defined as the peak negative pressure in the ultrasound field (PNP) divided be the square root of the centre frequency of the ultrasound field in MHz ($F_c$) [American Institute of Ultrasound in Medicine. Acoustic Output Measurement Standard for Diagnostic Ultrasound Equipment. 1st ed. 2nd ed. Laurel, MD: American Institute of Ultrasound in Medicine; 1998, 2003].

$$MI = \frac{PNP}{\sqrt{F_c}}.$$

Regulatory requirements during medical US imaging are to use a MI less than 1.9. During US imaging with microbubble contrast agents, an MI below 0.7 is recommended to avoid detrimental bio-effects such as micro-haemorrhage and irreversible vascular damage and using an MI below 0.4 is considered "best practise". It should be understood that when referring to MI in the text, this reflects the in-situ MI, i.e. the MI applied to the targeted region of interest.

The term 'activation' or "activation step", step (iii) of the method, in the context of the ACT procedure in this text, refers to the induction of a phase shift of microbubble-microdroplet clusters by ultrasound (US) insonation, i.e. the generation of large, activated bubbles.

The term frequency is defined as number of (ultrasound) cycles per second (Hz). When used herein the term designates the centre frequency of the applied sound field.

The term "enhancement" or "enhancement step", step (iv) of the method, in the context of the ACT procedure in this text, refers to the induction of volume oscillations of the large, activated bubbles and ensuing biomechanical effects, by US insonation. The term "resonance frequency" or "microbubble resonance frequency", when used in this text, is meant to describe the acoustic resonance frequency of a single bubble in an infinite matrix domain (neglecting the effects of surface tension and viscous attenuation). The resonant frequency is given by:

$$f = \frac{1}{2\pi a}\left(\frac{3\gamma p_A}{\rho}\right)^{1/2}$$

where a is the radius of the bubble, $\gamma$ is the polytropic coefficient, $p_A$ is the ambient pressure, and $\rho$ is the density of the matrix.

**Details of the Invention:**

Hence, the invention provides a cluster composition for use in a method of treatment of diseases, disorders or injuries of the CNS. The invention uses ACT technology to generate large phase shift bubbles *in vivo* from an administered pharmaceutical composition comprising microbubble-microdroplet clusters, and which facilitates delivery and uptake of separate pre-, and/or co- and/or post administered therapeutic agent(s). The therapeutic effect of the therapeutic agent is considerably increased compared to administration of the agent alone, due to biomechanical mechanisms in the microvasculature, as further explained below. The composition for use and the method of the invention potentiates the therapeutic effect of the separately co-administered therapeutic agent, providing an improved therapeutic outcome, compared to treatment without the use of the compositions of the invention. Whereas WO2015/047103 briefly notes the possibility of using ACT for opening the BBB, it does not disclose how such a procedure should be performed. In a small pilot study in rats reported by Åslund, using a simple preclinical setup, the application of ACT is shown to be able to increase uptake of a small MRI contrast agent and a fluorescent imaging agent. However, Åslund fails to understand and disclose the importance of applying a lower frequency, targeted to the resonance frequency of the large, activated bubbles. He also fails to disclose the particular benefit the ACT concept could have in delivery of larger drug constructs such as nanoparticulate drugs. Finally, he does not disclose insonation procedures that are applicable to treatment of humans. The present disclosure details several options for configuring US devices to enable necessary control with US targeting and dosimetry within the CNS in a human, clinically relevant situation. Furthermore, it demonstrates that a specific use of the ACT technology comprising two steps of ultrasound insonation at different frequencies enables an increased BBB permeability of separately administered therapeutic agents. Finally, the present invention is applicable for delivery of large construct therapeutical agents such as nano-drugs.

In one aspect, the invention provides a pharmaceutical composition comprising a microbubble-microdroplet cluster composition and at least one therapeutic agent for use in a method of treatment of diseases, disorders or injuries of the central nervous system (CNS) of a subject, wherein the use comprises Acoustic Cluster Therapy (ACT) treatment wherein at least one step of ultrasound insonation is performed either non-invasively towards the CNS, invasively towards the CNS or towards the heart or a spinal or carotid artery outside the CNS, comprising the steps of:

(i) administering the pharmaceutical composition to the subject; wherein the at least one therapeutic agent is pre-, and/or co- and/or post administered separate to the cluster composition;
(ii) optionally imaging the clusters of the pharmaceutical composition using ultrasound imaging to identify a region of interest in the CNS for treatment within said subject;
(iii) activating a phase shift of a diffusible component of the microdroplet of the cluster composition from step (i) by ultrasound insonation at a first frequency and a first mechanical index; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column; and
c) for the insonation towards the heart or a spinal or carotid artery, this takes place in the heart or in a spinal or carotid artery outside the CNS;

(iv) further insonating with ultrasound at a second frequency which is lower than the first frequency, and a second mechanical index, facilitating extravasation of the at least one therapeutic agent administered in step (i) at the region of interest in the CNS; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column;
c) when activating according to c) in step (iii), further insonation in this step takes place either non-invasively or invasively according to step (iv) a) or step (iv) b).

In the text the term Activation or Activation step is used for describing step (iii) above and the term Enhancement or Enhancement step is used for describing step (iv) above.

The Acoustic Cluster Therapy (ACT) used in the invention is an ultrasound-mediated targeted drug delivery platform that utilizes microbubble-microdroplet clusters, activated by the application of ultrasound, to create biomechanical effects that facilitate extravasation, e.g. by creating localized openings or fenestrations in the vasculature, leading to a transient increase in vascular permeability and thereby allowing drugs to better penetrate the vasculature. The current invention is partly based on findings from a pre-clinical study, wherein the applicant has investigated the ability of ACT in delivery of nanoparticles encapsulating cytotoxic substances in the form of core-crosslinked polymeric micelles (CCPMs) across the blood-brain barrier (BBB) as further described in Example 3. The applicant has found that the ACT concept is an effective way to overcome biological barriers for improved uptake, even of large construct therapeutic agents such as CCPMs. This has been found to be particularly beneficial for treatment of the CNS, providing a safe and effective means of temporarily bypassing the highly selective semipermeable border of endothelial cells of the BBB, aiding in delivering of even large molecules or constructs directly to e.g. a pathological site in the brain. The BBB represents the tightest vascular barrier in the body and is closed to therapeutic agents larger than approximately 4-500 Daltons. Example 3 demonstrates the ability of the compositions and methods of the invention to enable delivery and uptake of large constructs like nano-particles into the brain tissue. In Example 3, the accumulation of 65 nm large CCPM in healthy mouse brains, was studied using near infrared fluorescence (NIRF) imaging. Micro-distribution of the CCPM in brain sections was imaged by confocal laser scanning microscopy (CLSM) and a semi-quantitative analysis on the CLSM images was performed. As demonstrated by Figure 9, ACT clearly increased the permeability of the BBB using rather low acoustic pressures which resulted in an improved accumulation, extravasation and penetration of the large CCPM into the brain parenchyma. In short, Example 3 demonstrates:

- That the compositions and methods herein (ACT[®]) safely and temporarily increased the permeability of the BBB shown by contrast-enhanced magnetic resonance imaging

- Improved brain accumulation of CCPM using Near-infrared fluorescence imaging

- Enhanced extravasation (extravasation in a cloud pattern with an average size of 80863 $\mu m^2$ clouds, size of area) and penetration of core-crosslinked polymeric micelles + macromolecule CW800-PEG using confocal microscopy of fluorescently labelled blood vessels and model drugs

- Histological evaluation showed no evidence of tissue damage or an acute inflammatory response

The study demonstrates the great potential of ACT for improving the delivery of drugs and nanoparticles temporarily and safely across the BBB. Successful delivery of CCPM and other therapeutic agents across the BBB by ACT could be a promising strategy for treating several brain disorders, such as Alzheimer's, Parkinson's disease and cancers of the brain, which are expected to occur more often in the worlds' ageing population. The composition and methods of the invention hence allow for safe opening of the BBB which enables delivery of drugs directly to the brain, paving the way for penetration of disease-modifying therapies.

Several pre-clinical studies in different cancer models have demonstrated the improved therapeutic efficacy when chemotherapeutic agents were combined with ACT. These studies have demonstrated the great potential of ACT to increase tumour vascular permeability and thereby improving drug delivery to solid tumours. However, the potential of the ACT-concept to increase the permeability of the BBB and improve drug delivery to the brain remains largely unexplored. The initial proof of principle study (Åslund et al. 2017) in healthy rats showed increased BBB permeability after ACT, leading to increased accumulation of contrast agents. However, as demonstrated in Example 2 herein, assessing the sensitivity of the ACT procedure towards the frequency and MI of the incident US field during the Enhancement step, these experiments of Åslund were conducted with what has now been found to be sub-optimal ultrasound frequencies, not fully exploiting the potential of ACT. Secondly, combining ACT with a more clinically relevant drug carrier would be of great interest to study the potential of ACT for treating brain diseases, disorders or injuries. The main aim of the study presented herein as Example 3, was therefore to show the potential of the ACT, the cluster compositions and use of these to increase the permeability of the BBB and improve delivery of therapeutic agents such as in clinically relevant drug carriers across the BBB. As shown in Example 3, combining ACT with core-crosslinked polymeric micelles (CCPM) as drug nanocarriers could improve drug delivery across the BBB, demonstrating that even larger therapeutic molecules and drug constructs (e.g. liposomal formulations, nanocarriers and therapeutic viruses) can enter the brain using the compositions and methods of the invention. In this study a custom-built dual frequency ultrasound transducer was used to efficiently and safely increase BBB permeability using ACT.

**Methods of Insonation:**

In Example 2, the sensitivity of the ACT procedure towards the frequency and MI of the incident US field during the Enhancement step is demonstrated. From this, it will be clear that a relatively precise control with the incident US field is required to enable optimal therapeutic effect and avoid adverse effects such as permanent vascular damage and/or haemorrhaging.

However, bone structures represent a significant and frequency dependent barrier for US. For example, even through a thin bone structure like a mouse skull, at a frequency of 1 MHz, 20% of the incident US power is lost passing through the skull. At a frequency of 2.7 MHz, more than 40% is lost. Adding to this, the thickness of the skull varies with age, sex and across ethnic's groups, all in all making precise control with US dosimetry within the CNS extremely challenging.

The method comprises steps of ultrasound insonation towards the brain or spinal cord, or to the heart or spinal or carotid arteries, i.e. steps iii) and iv) described above. Due to the skull protecting the brain this is not trivial. There are basically two ways to administrate ultrasound towards the brain, one way is non-invasive, and the other is from a surgically implanted ultrasound transducers (herein named invasive insonation) in or through the skull bone. The location of the treating area in the brain may be determined using a pre-treatment planning software from patient's CT/MRI images. One example of a non-invasive way to administrate ultrasound is by focused ultrasound using an extracranial, hemispheric focused US array (Figure 1 A), eg. as disclosed by Beccaria, K. et al., Blood-brain barrier disruption with low-intensity pulsed ultrasound for the treatment of paediatric brain tumours: a review and perspectives, Neurosurg Focus, 2020 Jan 1;48(1). This device configuration does not require open surgery and provides fine spatial control over the treatment field using a helmet with multiple small transducers assembled in array, allowing for precise control with the sound field inside the brain. In order to enable unimpeded connectivity, there is a water or ultrasound gel layer between the transducer and the head, and the head should be shaved before treatment. An alternative non-invasive device configuration for use is an extracranial, focused mono-element US transducer (Figure 1 B). Yet further alternatives are nasal and ocular transducers. Hence a nasal cavity ultrasound transducer may be used, potentially with administration of the drug through the olfactory pathway. Focused ultrasound enhanced delivery may hence be achieved, eg.by administration of drugs by the olfactory pathway directly to the brain, allowing for a more homogenous distribution in targeted locations compared to intravenous delivery. alone. Ocular transducers may be worn on the eye as a lens or as a transplanted chip. Alternatively, a transducer may be positioned into the bony orbit (cavity) of the eyeball. In certain embodiments, the eyeball may be removed in order to position the transducer.

In one embodiment of the method, both steps of ultrasound insonation, i.e. both the activation in step iii) and the further insonation (enhancement) of step iv) are performed non-invasively towards the CNS. In one embodiment, the ultrasound insonation is performed either by an extracranial, hemispheric focused US array or an extracranial, focused mono-element US transducer, or by a nasal transducer.

So-called invasive administration of the US field requires that one or more US transducers have been surgically implanted through the skull or spinal cord bone of the subject, as visualised in Figure 1 E (single transducer) and 1 F (multiple transducers). At least one surgically implanted transducer in the subject's skull or vertebral column have been implanted prior to the method of the invention, and hence the "invasive insonation" of the method does not represent surgery in itself. These configurations bypass the issues of variable US attenuation going through a bony structure, simplifying control with dosimetry, but represents an invasive solution. In one embodiment, the transducer is an ultrasound chip implanted into the brain, such as in form of a self-controlled, biodegradable piezoelectric nanofiber chip. In one embodiment of the method, both steps of ultrasound insonation, i.e. both the activation in step iii) and the further insonation (enhancement) of step iv) are performed invasively towards the CNS. In one embodiment, the ultrasound insonation is performed by one or more US transducers positioned in or through the skull or spinal cord bone.

ACT represents a deposit tracer; the activated bubbles will transiently deposit in the nearest capillary bed downstream of the site of activation, in an amount correlated to the blood perfusion of the tissue. As demonstrated in Example 4, this attribute of the technology may be utilized to bypass the US dosimetry issues for the activation step by activating in a feeding vessel outside the CNS, such as in the carotid or spinal arteries. Such a procedure would allow for precise control with the US activation field and deposit activated bubbles throughout the CNS capillary beds getting their blood supply from the artery in question. These procedures are visualized in Figure 1 C and Figure 1 D. If the Activation step is performed according to this procedure, the Enhancement step is still performed according to either the non-invasive or the invasive procedure described above. Hence, in one embodiment of the method the first step of ultrasound insonation, i.e. the activation in step iii) takes place in the heart or in a spinal or carotid artery outside the CNS, while the further insonation (enhancement) of step iv) is performed either non-invasively or invasively, hence step iv) is performed either by an extracranial, hemispheric focused US array or an extracranial, focused mono-element US transducer, or by one or more surgically implanted US transducers in or through the skull bone or spinal cord bone. In one embodiment, when the activation is performed towards the heart or in a spinal or carotid artery outside the CNS, the enhancement step is preferably performed non-invasively.

**Attributes of the US field:**

Hence, the administered microdroplet-microbubble clusters are triggered by a localized insonation method, comprising ultrasound insonation at different frequencies - one relatively high frequency for the activation step (step iii of the method) and one relatively low frequency for the enhancement step (step iv of the method). When the clusters are insonated with ultrasound (activated) the oscillating microbubbles initiate an instant vaporisation (phase-shift) of the attached microdroplet. The enlarged resulting bubbles have been shown to form in capillary sized vessels *in vivo* and are then further excited by low frequency ultrasound (step iv) to induce biomechanical effects that facilitate extravasation and increase drug penetration in the insonated tissue (enhancement). Contrary to what is disclosed by Åslund, the applicant has identified that the method of treatment should comprise two steps of insonation, comprising one step of activation and one step of enhancement, and that different ultrasound frequencies are to be used in these insonation steps. In one embodiment, the frequency used in the activation and enhancement steps are the same. During the activation step, the microbubbles of the clusters oscillate and transfer energy to the microdroplets inducing droplet vaporization and possible initial biomechanical effects on the vessel wall, forming large ACT bubbles designed to transiently lodge in the microvasculature. Hence, the clusters are activated to produce large bubbles by application of external ultrasound energy, after administration, such as from a clinical ultrasound imaging system, under imaging control, wherein, during the enhancement step, the further insonation at the lower frequency induces biomechanical effects, extravasation and increased drug penetration.

**EP 4 313 149 B1**

The steps of the method of treatment are further described below, and in Example 2 the applicant has investigated attributes of the ultrasound fields applied during the second insonation step (the enhancement step) and their effect on the functionality of the applied procedure. Surprisingly, the applicant has found that the functionality of the concept is quite sensitive to these parameters. Based on these studies, the applicant has found that for step (iv) of the method, insonating with ultrasound at a second frequency range between 0.4 to 0.6 MHz and with the applied second MI kept to more than 0.1, but less than 0.3, is preferred. Alternatively, for applications towards the CNS, as lower frequencies result in lower attenuation through bony structures and allow for better control with the in-situ US field (i.e. the US field in the region of interest), a preferred second frequency range may also be between 0.2 to 0.4 MHz, with an applied second MI of 0.025 to 0.15. With lower frequencies and/or higher MIs, than those disclosed, during the enhancement step, the applicant has surprisingly found that the activated bubble oscillations induced are too strong, leading to a significant loss of efficacy and vascular damage. On the other hand, with higher frequencies and/or lower MIs, the bubble oscillations induced are too small, leading to a lack of sufficient biomechanical effects and hence a significant loss in therapeutic efficacy. It should be understood that these preferred ranges are irrespective of whether the insonation is applied using invasive or non-invasive approaches. As for insonation towards the heart or the spinal and carotid artery, this approach is not applicable to the enhancement insonation, only the activation insonation.

For the activation step, step iii) of the method, the situation is simpler, as the resonance frequency of the microbubble component is typically in the range of 2-5 MHz, the clusters are readily activated by frequencies in the regular medical imaging range of 1-10 MHz with MIs above 0.1. However, in order to avoid inertial cavitation mechanisms of the microbubble components, the MI during the enhancement step should be kept below 0.4. Even though the clusters are activated across the regular imaging spectrum of frequencies, as attenuation through bone is a sharply increasing function of frequency, frequencies towards the lower end of the spectrum are preferred for non-invasive activation insonation. Furthermore, for the same reason, as many commercial devices for non-invasive US insonation of the CNS apply frequencies down to 0.2 MHz, such are included in the preferred range for the current invention.

For non-invasive activation insonation, a preferred embodiment of the invention is to perform the activation step with a frequency between 0.2-3 MHz and at an in-situ MI of 0.1-0.4.

For invasive activation insonation, or activation outside the CNS, a preferred embodiment of the invention is to perform the activation step with a frequency between 1-10 MHz and at an in-situ MI of 0.1-0.4.

Hence, the method comprises the following steps:

Administration, step i): The cluster composition is administered to said mammalian subject parenterally, preferably intravenously, and the therapeutic agent is pre-, and/or co- and/or post administered separate to the cluster composition as a separate composition, as further described under "Administration routes" herein. The term "pre-administered" also encompasses surgically implanted drug depos, e.g. wherein the ultrasound treatment triggers a release of the drug.

Imaging, step ii): The clusters are not activated at low MI, i.e. below the cluster activation threshold of approx. 0.1, allowing standard medical ultrasound contrast agent imaging to be performed, for example to identify tumour micro vascular pathology without activation of the clusters. Hence, in one embodiment the method includes a step of using low MI contrast agent imaging modes (MI < 0.1) to image the microbubble component, i.e. the dispersed gas, without activation of the clusters, to identify the pathological location for treatment (step ii). Hence, as the clusters are not activated at low MI (below the activation threshold) standard medical ultrasound contrast agent imaging may be performed, prior to the activation step, for example to identify tumour microvascular pathology.

Activation, step iii): The acoustic resonance of the microbubble component of the clusters is within the diagnostic frequency range (1-10 MHz). For invasive activation insonation or activation insonation outside the CNS, when the cluster composition has been administered to the subject, activation of the clusters is readily obtained with standard diagnostic ultrasound imaging pulses (1-10 MHz) used for example in conventional medical ultrasound, at low to mid-range mechanical indices, i.e. an MI below 0.4 and preferably below 0.3, but above 0.1 and preferably above 0.15. For non-invasive activation insonation, the preferred frequency range is from 0.2-3 MHz with an MI below 0.4 and preferably below 0.3, but above 0.1 and preferably above 0.15.

In one embodiment, the activation, i.e. the initial US insonation, starts immediately after each administration of the cluster composition, such as within 20 seconds, and preferably lasts for e.g. 60-120 seconds. This applies irrespective of whether the insonation is applied non-invasively, invasively or to the heart or the spinal or carotid arteries.

The activation under medical ultrasound imaging control using the imaging pulses allows spatially targeted activation of the clusters in the tissue region being interrogated by the ultrasound field. After activation, the large phase shift bubbles produced are temporarily trapped in the microvasculature of the targeted pathology due to their size. The resulting large phase shift bubbles are approximately 1000 times the volume of the emulsion microdroplet vaporised (a 20 $\mu$m bubble diameter from a 2 $\mu$m diameter oil microdroplet). The scattering cross sections of these large phase shift bubbles are orders of magnitude greater than the scattering cross sections of the micron sized microbubbles comprised in the clusters before activation. As a result, the large phase shift bubbles produce copious backscatter signal and are readily imaged in fundamental imaging mode with diagnostic imaging systems. The resonance frequencies of the large phase shift bubbles are also an order of magnitude lower (approximately 0.2-0.8 MHz) than the resonance frequencies of the microbubbles comprised in the clusters before activation.

Due to the large size of the activated bubbles, they temporarily lodge in the microvasculature and can be spatially localised in a tissue or organ of interest, such as of the brain or spinal cord, by spatially localised application of the ultrasound energy to activate the clusters. Hence, after administration of the cluster composition, the clusters are activated within, at or near the CNS by application of ultrasound energy towards the CNS and the target site. Alternatively, the clusters may be activated in feeding arteries outside the CNS, such as the spinal or carotid arteries, depositing the activated bubbles in the nearest CNS capillary beds downstream of the activation site.

As demonstrated in Example 5, after activation, large bubbles of typically 20 $\mu$m in diameter are formed and transiently retained in the larger micro capillaries. As shown in Figure 12, the activated phase shift bubble gradually shrinks and advances further down in the capillary three by intermittent lodging and dislodging, before it clears completely after typically 5 to 10 minutes. This process entails that even when activating in the heart or feeding vessels, the intermittent lodging and dislodging assures that capillaries of all sizes less than the bubble diameter are receiving the biomechanical effect and that a single bubble or several bubbles will lead to enhanced permeability at several places in the capillary bed.

Enhancement, step iv): This step preferably uses a lower frequency than the activation step and induces controlled volume oscillations of the ACT-bubbles, thereby exerting biomechanical forces on the capillary wall and enhancing drug delivery locally. This further application of low frequency ultrasound after activation and deposition facilitates enhancement of delivery mechanisms by effectively overcoming biological barriers to increase the efficiency of drug delivery to a spatially targeted tissue. These mechanisms may include the process of sonoporation i.e. a process where insonation, and ensuing volume oscillation, of microbubbles in the vascular compartment increases the permeability of the blood-brain barrier. In other words, the method steps increase the permeability of the endothelial wall and hence enhances the extravasation, distribution and cellular uptake of the co-administered therapeutic agent(s). Other mechanisms, such as generation of cellular signalling for enhanced therapeutic effect, mechanical breaking down of interstitial structures that enhances drug penetration, opening of intracellular junctions to allow for transcytosis, immune modulation, modulation of influx/efflux transporters, and upregulation of receptors, etc. may also be induced. It will be appreciated that for the composition for use and method of the invention, this further insonation of the large, activated bubbles with the application of low frequency ultrasound further enhances the uptake of the therapeutic agent(s). Hence, it has been found that the application of low frequency ultrasound, close to the resonance frequencies of the large, activated bubbles, can be used to produce mechanical and/or thermal bio-effect mechanisms to increase the permeability of the vasculature and/or sonoporation and/or endocytosis of pathological tissue in the CNS and hence increase delivery and retention, such as in the perivascular space and lymphatic system brain (lymph drainage) of the therapeutic agent to the targeted tissue.

Application of acoustic fields that commensurate with the resonance frequencies of the larger phase shift bubbles produces relatively large radial oscillations at MIs within the medical diagnostic range. However, as demonstrated in Example 2, the performance of the procedure with regards to efficacy and adverse effects is quite sensitive to the frequency and MI of the applied US field. Thus, in combination with MIs in the range of 0.1 to 0.3, low frequency ultrasound, in the range of 0.4 to 0.6 MHz, is preferred to produce the bio-effect mechanisms that enhance the uptake of the administered drug, and hence facilitates extravasation. Alternatively, in combination with MIs in the range of 0.025 to 0.15, low frequency ultrasound, in the range of 0.2 to 0.4 MHz is preferred. It has been found that after activation in-vivo, the volume weighted mean diameter of the activated bubbles is approx. 20 $\mu$m. In blood, the resonance frequency of free microbubbles of this size has been calculated to approx. 0.33 MHz. However, due to dampening effects of the vascular wall, it is expected that the resonance frequency of such a bubble is somewhat higher, when trapped in a micro vessel. As demonstrated by Example 2, a most preferred frequency range for the low frequency enhancement step is 0.4-0.6 MHz in combination with an MI of 0.1 to 0.3 or, alternatively a frequency of 0.2-0.4 in combination with an MI of 0.025 to 0.15. Exploiting the resonance effects of the activated bubbles allows better control of initiation of these bio-effects at lower acoustic intensities and at lower frequencies than possible with other technologies. Coupled with the fact that the large phase shift bubbles are activated and deposited in the tissue microvasculature under imaging control (allowing spatial targeting of the large, activated bubbles in tissue), and their prolonged residence time, allows more efficient and controlled implementation of the drug delivery mechanisms.

Hence, in one embodiment, the method comprises an enhancement step (step iv) insonating further with ultrasound at a second frequency in the range of 0.4 to 0.6 MHz, in combination with a second MI of 0.1-0.3, or, a second frequency in the range of 0.2 to 0.4 in combination with a second MI of 0.025 to 0.15. These ranges apply for both non-invasive and invasive insonation approaches.

Surprisingly, a larger therapeutic benefit has been found when the activated bubbles are insonated to induce enhanced uptake by applying ultrasound e.g. in the range of 0.4 to 0.6 MHz, e.g. 500 Hz as used in the Examples. For this frequency range, the MI for this enhancement step is preferably below 0.4, and more preferably below 0.3 but above 0.1, preferably above 0.2. With a frequency of 0.4-0.6 MHz, if the MI applied during the enhancement step is lower than 0.1 it is expected that the biomechanical effects generated will be insufficient and, hence, reduce the therapeutic benefit significantly. This is demonstrated in Example 2, where insonation using 0.9 MHz leads to too small radial oscillations, with an ensuing significant loss of efficacy when compared to 0.5 MHz insonation. On the other hand, if the MI is larger than 0.3, the biomechanical effects may become too strong with ensuing haemorrhaging, vascular damage and/or shut down, with ensuing loss in extravasation and efficacy, as demonstrated in Example 2. Similar considerations apply for the frequency range 0.2-0.4 MHz; the MI should be kept to 0.025 to 0.15 in order to induce sufficient biomechanical effects to produce a therapeutic benefit, but at the same time avoid potential adverse effects and safety issues. Due to their larger size, ACT bubbles will cover larger areas within the blood vessel, have closer contact with the endothelium and stay for a prolonged time. Due to these attributes, ACT-bubbles, as opposed to conventionally used microbubbles, apply a higher magnitude of biomechanical work on the capillary wall, resulting in increased drug delivery across the BBB.

The insonation with low frequency ultrasound (step iv) follows the activation step (step iii) and should typically last for 3 to 10 minutes, such as for about 5 minutes. There is preferably an immediate start of step (iv) after step (iii) for both non-invasive and invasive insonation approaches. Insonation towards the heart and spinal or carotid arteries is only applicable for the activation step.

In some embodiments, the activation insonation is performed invasively towards the CNS using a first frequency in the range of 1-10 MHz with a first MI of 0.1-0.4, and the enhancement insonation is performed invasively towards the CNS using a second frequency in the range 0.2-0.4 MHz with a second MI of 0.025 to 0.15, or using a second frequency in the range of 0.4-0.6 MHz with a second MI of 0.1 to 0.3.

In some embodiments, the activation insonation is performed non-invasively towards the CNS using a first frequency in the range of 0.2-3 MHz, such as 0.2-3.4 MHz, with a first MI of 0.1-0.4, and the enhancement insonation is performed non-invasively towards the CNS using a second frequency in the range 0.2-0.4 MHz with a second MI of 0.025 to 0.15, or using a second frequency in the range of 0.4-0.6 MHz with a second MI of 0.1 to 0.3.

In some embodiments, the activation insonation is performed non-invasively towards the heart or spinal or carotid arteries using a first frequency in the range of 1-10 MHz with a first MI of 0.1-0.4 and the enhancement insonation is performed either non-invasively or invasively towards the CNS using a second frequency in the range 0.2-0.4 MHz with a second MI of 0.025 to 0.15, or using a second frequency in the range of 0.4-0.6 MHz with a second MI of 0.1 to 0.3.

In some embodiment, the method does not require that any invasive steps need to be taken. Hence, in one embodiment the invention provides a pharmaceutical composition comprising a microbubble-microdroplet cluster composition and at least one therapeutic agent for use in a method of treatment of diseases, disorders or injuries of the central nervous system (CNS) of a subject, wherein the use comprises Acoustic Cluster Therapy (ACT) treatment wherein at least one step of ultrasound insonation is performed either non-invasively towards the CNS or towards the heart or a spinal or carotid artery outside the CNS, comprising the steps of:

(i) administering the pharmaceutical composition to the subject; wherein the at least one therapeutic agent is pre-, and/or co- and/or post administered separate to the cluster composition;
(ii) optionally imaging the clusters of the pharmaceutical composition using ultrasound imaging to identify a region of interest in the CNS for treatment within said subject;
(iii) activating a phase shift of a diffusible component of the microdroplet of the cluster composition from step (i) by ultrasound insonation at a first frequency and a first mechanical index; wherein this either

a) takes place from at least one transducer positioned outside the brain skull or vertebral column of the subject for insonation through this; or
c) takes place in the heart or in a spinal or carotid artery outside the CNS;

(iv) further insonating with ultrasound at a second frequency and a second mechanical index, facilitating extravasation of the at least one therapeutic agent administered in step (i), at the region of interest in the CNS; wherein this takes place from at least one transducer positioned outside the brain skull or vertebral column of the subject for insonation through this.

Hence in this procedure, the further insonation (enhancement) of step iv) is performed non-invasively irrespective of insonation approach in the activation step iii).

## Diseases, disorders and injuries of the CNS:

The cluster composition and method of the invention can increase the permeability of the blood-brain barrier to allow for safe passage of medical drugs and for medical treatment of diseases, disorders or injuries of the central nervous system (CNS). For example, these may be used in treatment of neurological diseases or disorders, or of tumours in the brain or in the spinal cord, also including of autoimmune diseases, cerebral aneurysms or drug addictions.

In some embodiments, neurological diseases are e.g. meningitis, encephalitis, infections from virus, bacteria, protozoan, fungal, or worms. Neurological disorders are e.g. essential tremors, bipolar disorder, epilepsy, seizures, trauma, Parkinson's, multiple sclerosis, dementia, schizophrenia, Alzheimer's disease, depression, mononeuropathy, polyneuropathy, chronic pain, myopathy.

In some embodiments, the injury to be treated using a composition or method according to the invention is either of physical injuries to the CNS such as cerebral tumours, cerebrovascular accidents such as thrombosis, embolism, haemorrhage, and vasculitis.

In some embodiments, the disease or disorder to be treated using a composition or method according to the invention is either of CNS cancer, brain stroke, Alzheimer's disease, Parkinson's disease, Multiple Sclerosis (MS) or amyotrophic lateral sclerosis (ALS). Brain stroke is the leading cause of death in the U.S; over 4 million U.S. men and women suffer from Alzheimer's disease; 1 million from Parkinson's disease; 350,000 from multiple sclerosis (MS); and 20,000 from amyotrophic lateral sclerosis (ALS) (2020). Worldwide, these four diseases account for more than 20 million patients. There is hence a need for new methods to treat these diseases.

In some embodiments, the disease to be treated using a composition or method according to the invention is a CNS cancer selected from the group of: Acoustic neuroma; Adenoma; Anaplastic astrocytoma; Astrocytoma; Brain stem glioma; Cerebrospinal fluid (CSF) leak; Chondroma; Chondrosarcoma; Chordoma; Convexity meningioma; Craniopharyngioma; Encephaloceles; Ependymoma; Esthesioneuroblastoma; Fibrillary astrocytoma; Fibrous dysplasia; Giant cell tumour; Germ cell tumour; Gliomas such as Astrocytoma, Brain stem glioma, Ependymoma, Glioblastoma multiforme, Mixed glioma, Oligodendroglioma, Optic nerve glioma; Hemangiopericytoma; Juvenile pilocytic astrocytoma; Meningioma such as Convexity meningioma, Skull base meningioma, Metastatic brain tumours such as metastasis of Breast cancer, Colon cancer, Kidney cancer, Lung cancer and Melanoma (skin cancer); Nasopharyngeal angiofibroma; Neurofibroma; Neurofibromatosis such as Neurofibromatosis 1 (NF1), Neurofibromatosis 2 (NF2), Schwannomatosis; Olfactory neuroblastoma (esthesioneuroblastoma); Oligodendroglioma; Optic nerve glioma; Osteoma; Paranasal sinus cancer; Pediatric brain tumours; Petrous apex lesion; Pituitary tumours such as Craniopharyngioma, Pituitary adenoma, Rathke's cleft cyst; Rhabdomyosarcoma, Schwannomas, Schwannomatosis, Skull base meningioma, Vestibular schwannoma.

In certain embodiments, the subject to be treated is a child. Children have thinner skull bones and are particularly amenable to US insonation of the CNS. CNS cancers of children that may be treated by the composition and method of the invention include, but are not limited to, the cancer types; Astrocytomas such as Anaplastic astrocytoma, Fibrillary astrocytoma, Glioblastoma multiforme, Juvenile pilocytic astrocytoma; Brain stem glioma; Choroid plexus tumour; Craniopharyngioma; Dysembryoplastic neuroepithelial tumour; Ependymoma; Gliomas in children such as Anaplastic astrocytoma, Brain stem glioma, Fibrillary astrocytoma, Glioblastoma multiforme, Juvenile pilocytic astrocytoma, Optic nerve glioma; Medulloblastoma; Optic nerve glioma; Spinal cord tumour. An astrocytoma may be graded as a glioma.

In some embodiments, the disease to be treated is an autoimmune disease, such as autoimmune encephalitis, central nervous system vasculitis, neurosarcoidosis, NMO Spectrum Disease, Myelin Oligodendrocyte Glycoprotein Antibody associated diseases, NMDAR Encephalitis, other rare neurological autoimmune diseases.

The composition and methods of the invention will allow for safe opening of BBB which allows delivery of drugs directly to the brain, paving the way for disease-modifying therapies. The BBB controls the entry of vital nutrients from the circulation to the brain and the removal of waste products as well as preventing the entry of pathological agents. The integrity of the BBB is essential for CNS homeostasis and damage to the capillary endothelium and BBB can contribute to the pathogenesis of various neurological disorders and diseases. Larger drug molecules cannot cross BBB by state-of-the art technology and drugs that might help are thereby prevented to help this patient population. However, with the composition and methods of the invention the BBB can be opened to deliver therapeutic agents at the relevant pathology and may also deliver agents that are too large to cross the BBB without the ACT technology, to treat the diseases or disorders.

**Therapeutic agent:**

The therapeutic agent, also called "the drug", to be delivered to the subject is an agent that may have an effect in treating diseases, disorders or injuries of the CNS. As it so far has been difficult to treat such diseases and disorders due to the BBB, agents that are designed or approved for other indications, e.g. other cancer forms than brain cancers, could as well be useful. The therapeutic agent is administered as a separate composition to the cluster composition. Examples of the therapeutic agent classes, and specific agents, useful in the claimed invention include, but are not limited to, those described herein.

Thus, the cluster composition and method of the invention for delivery and for administration of therapeutic agent increases the permeability of the blood-brain barrier for passage of therapeutic agents to allow for medical treatment of diseases, disorders and injuries of the CNS. Using the method of the invention, even larger drug constructs can cross the BBB and enter the brain and spinal cord. The BBB represents the tightest vascular barrier in the body. Unimpaired, it is completely closed to therapeutic agents larger than approximately 4-500 Daltons. Example 3 demonstrates the ability of ACT to enable uptake of large constructs like nano-particles into the brain tissue and demonstrate the utility of the composition and method of the invention for localized delivery of large molecules and other drug constructs across the BBB. Hence, in on embodiment, the method of the invention enables the delivery of a broad range of types and sizes of therapeutic agent for use in the treatment. In one embodiment, the therapeutic agent, or the formulated form of the therapeutic agent, or the biologically active form of the therapeutic agent (e.g. as in a protein bound form) has a molecular weight of more than 500 Daltons, preferably more than 15.000 Daltons, more preferably more than 50.000 Daltons and most preferably more than 100.000 Daltons.

Hence, in one embodiment, the therapeutic agent is formulated in a vehicle, such as included in the form of liposomes, conjugates, nanoparticles or microspheres, including nano-drug carriers, as vehicles for the therapeutic agent. In further embodiments, the therapeutic agent is selected from the group of genes (gene delivery), antimicrobial peptides, stem cells and aptamers. In some embodiments, the therapeutic agent is in the form of stem cells, such as neural stem cells (NSCs) or mesenchymal stem cells (MSCs), which may be used to deliver drugs or RNA to the brain. In some embodiments, the therapeutic agent is part of a larger drug construct such as nano-drug, e.g. in liposomal or particulate formulations, or as monoclonal antibodies. In recent years, core-crosslinked polymeric micelles (CCPM) have emerged as promising drug nanocarriers to improve therapeutic performance of a range of drug candidates. The CCPMs consist of highly tuneable polymers and biodegradable drug linkers that can be combined with a therapeutic agent of interest, generating a wide range of possible applications. However, as for most other drug nanocarriers, delivery across the BBB is challenging due to the BBB impeding the access of most substances. Combining the compositions and methods of the invention with therapeutic agents formulated in a vehicle, such as CCPMs, improves drug delivery across the BBB, generating a new range of therapeutic applications of these drug nanocarriers. As demonstrated by Example 3, the ATC concept of the invention may be particularly useful for combination with larger drug molecules or constructs, i.e. in a method wherein the therapeutic agent is a larger drug molecule or construct such as a liposomal formulation or a drug nano-carrier

The therapeutic agent that may be used in the composition or the method according to the invention may have any mechanism of action known to the skilled person, and as relevant for the disease or disorder to treat.

In some embodiments, the therapeutic agent is selected from the group of the drug classes chemotherapeutic agents, immunotherapeutic agents, immune oncology agents, immunomodulatory drugs, anti-B cell drugs, anti-inflammatory drugs, antimicrobial drugs, anti-angiogenic drugs, antidepressants, anticonvulsants, cannabinoid drugs, tumour necrosis factor-$\alpha$ (TNF) inhibitors, dopamine precursors, catechol-o-methyl transferase inhibitors, dopamine agonists, monoamine oxidase B inhibitors, mantadine, anticholinergics, anticoagulants, anti-platelet drugs, tissue plasminogen activator (tPA) and cholinesterase inhibitors.

In some embodiments, the therapeutic agent is a drug for treatment of Parkinson's disease, e.g. selected from the non-limiting list comprising Carbidopa-levodopa (various forms), Carbidopa-levodopa-entacapone (enteral suspension), Levodopa Inhalation powder, Entacapone, Tolcapone, Opicapone, Carbidopa/Levodopa Entacapone, Pramipexole, Pramipexole (extended release), Ropinirole, Apomorphine (injection), Apomorphine sublingual film, otigotine (transdermal patch), Selegiline, Rasagiline, Safinamide, Amantadine, Istradefylline, Trihexyphenidyl and Benztropine.

In some embodiments, the therapeutic agent is a drug for treatment of ALS, e.g. selected from the non-limiting list of Radicava, Rilutek, Tiglutik, and Nuedexta.

FDA has approved disease modifying therapies for MS which have been found through clinical trials to reduce the number of relapses, delay progression of disability, and limit new disease activity (as seen on MRI). In some embodiments, the therapeutic agent is a drug for treatment of MS, e.g. selected from the non-limiting list of the Injectable medications Avonex® (interferon beta-1a), Betaseron® (interferon beta-1b), Copaxone® (glatiramer acetate), Extavia® (interferon beta-1b), Glatiramer Acetate Injection (glatiramer acetate -generic equivalent of Copaxone 20 mg and 40 mg doses), Glatopa® (glatiramer acetate - generic equivalent of Copaxone 20mg and 40mg doses), Kesimpta® (ofatumumab), Plegridy® (peginterferon beta-1a), Rebif® (interferon beta-1a) and the oral medications Aubagio® (teriflunomide), Bafiertam ™ (monomethyl fumarate), Dimethyl Fumarate (dimethyl fumarate - generic equivalent of Tecfidera), Gilenya® (fingolimod), Mavenclad® (cladribine), Mayzent® (siponimod), Tecfidera® (dimethyl fumarate), Vumerity® (diroximel fumarate), Zeposia® (ozanimod), and Infused medications Lemtrada® (alemtuzumab), Novantrone® (mitoxantrone), Ocrevus® (ocrelizumab) and Tysabri® (natalizumab).

Although current medications cannot cure Alzheimer's or stop it from progressing, they may help lessen symptoms, such as memory loss and confusion, for a limited time. In some embodiments, the therapeutic agent is a drug for treatment of Alzheimer, e.g. selected from cholinesterase inhibitors, e.g. from the non-limiting list of Donepezil (Aricept), Galantamine (Razadyne), Rivastigmine (Exelon), and memantine (Namenda®).

In some embodiments, the therapeutic agent is a drug for treatment of cancer selected from the non-limiting list of examples of the therapeutic agent classes and specific agents:

### Alkylating agents

**Nitrogen Mustards:** Mechlorethamine Hydrochloride *(Mustargen)*

**Nitrosoureas:** Carmustine *(BiCNU),* Streptozocin (Zanosar), Lomustine *(CeeNU)*

**Tetrazines:** Dacarbazine *(DITC-Dome),* Temozolomide *(Temodar)*

**Aziridines:** Thiotepa *(Thioplex),* Mitomycin (Mutamycin), Aziridinylbenzoquinone (AZQ)

***Cisplatins:*** Cisplatin *(Platinol),* Carboplatin *(Paraplatin),* Oxaliplatin *(Eloxatin)*

**Antimetabolites**

**Anti-folates:** Methotrexate *(Otrexup, Rasuvo, Trexall),* Pemetrexed *(Altima)*

**Fluoropyrimidines:** Fluorouracil *(Adrucil),* Capecitabine *(Xeloda)*

**Deoxynucleotide analogues:** Cytarabine *(Cytosar-U),* Decitabine (Dacogen), Azacitidine (Vidaza), Gemcitabine (Gemzar), Fludarabine (Fludara), Nelarabine (Arranon), Pentostatin (Nipent)

**Thiopurines:** Thioguanine (Tabloid), Mercaptopurine (Purinethol, Purixan)

**Anti-microtubule**

***Vinca alkaloids:*** Vinorelbine (Navelbine), Vinicristine (Oncovin, Vincasar Pfs), Vindesine (Eldisine), Vinflunine (Javlor)

**Taxanes:** Paclitaxel or nab-Paclitaxel (Onxol, Abraxane), Cabazitaxel (Jevtana), Docetaxel (Docetaxel, Docefrez)

**Podophyllotoxin:** Etoposide (Eposin, Etoposide), Teniposide (Vumon)

**Topoisomerase inhibitors**

***Topoisomerase I:*** Irinotecan (Onivyde), Topotecan (Act Topotecan, Hycamtin)

**Topoisomerase II:** Doxorubicine (Adriamycin, Caelyx), Mitoxantrone (Novantrone), Teniposide (Vumon), Novobiocin (Novobiocin Sodium), Merbarone, Aclarubicin

**Cytotoxic antibiotics**
**Anthracyclines:** Doxorubicine (Adriamycin, Caelyx), Daunorubicin (Cerubidine, DaunoXome), Epirubicin (Ellence), Idarubicin (Idamycin), Bleomycin (Blenoxane), Mitomycin (Mitosol, Mutamycin)

**Immunotherapies**

**CAR-T cell therapy:** Sipuleucel-T (Provenge), Tisangenlecleucel (Kymriah), Axicabtagene ciloleucel (Yescarta)

**Antibody therapies:** Alemtuzumab (Campath CD52), Atezolizumab (Tecentriq PD-L1), Ipilimumab (Yervoy CTLA4), Pembrolizumab (Keytruda PD-1), Durvalumab (Imfinizi IgG1k)

**Oncolytic virus:** *Talimogene laherparepvec (OncoVEX GM-CSFIT-vecIMLYGIC) Ad2/5 dl1520 (Onyx-015), GLV-1h68 (GL-ONC1), CV706*

**Cancer Vaccines:** Oncophage, Sipuleucel-T (Provenge)

**Microglia and/or iNKT cell based therapy**

**Cytokine therapy**

**Interferon:** IFN$\alpha$ (Infergen), IFN$\beta$ (Actimmune)

**Interleukin:** No commercial product, In trials.

**Antimicrobial compounds**
**RNA therapy**

**Cell based therapies;**
Stem Cells, Fibroblasts

In one embodiment, the therapeutic agent is selected from the group of Alkylating agents, Antimetabolites, Anti-microtubules, Topoisomerase inhibitors, Cytotoxic antibiotics, Immunotherapeutic agents and Cytokine therapeutic agents.

It should be clear that therapeutic agents not normally used for treatment of the CNS, because of limited uptake due to the BBB, are within the scope of the invention. When referring to a specific drug, the reference is intended to include any drug comprising the same active ingredient or ingredients and with a corresponding mode of action, such as a generic drug.

Quite often, therapeutic regimes comprise combination therapies in which for instance one or several chemotherapeutic agents are given in conjunction with an immunotherapeutic agent. A preferred option within the current invention is to apply such combination regimens. One such drug combination is the drug combination called PCV. The drugs in the combination are Procarbazine Hydrochloride, Lomustine (CCNU), and Vincristine Sulfate.

The actual dosage amount of the therapeutic agent administered to a subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject. In one embodiment, the dosage of the therapeutic agent is lower than what the dosage would normally be without use of the method of the invention. Preferably more than 20% lower, more preferably more than 40% lower and even more preferably more than 60% lower.

**The Cluster composition:**

In the method of the invention a premixed cluster composition is administered to the subject, in addition to the separate administration of a therapeutic agent. The administered clusters can be activated by ultrasound. The composition of clusters is a premixture of microbubbles (first component) and microdroplets (second component) resulting in small microbubble-microdroplets clusters in a dispersion held together by electrostatic forces. The microdroplets typically comprise an oil component that has a boiling temperature of < 50 °C, and low blood solubility. The cluster composition, i.e. a combination of the first and second components, comprises clusters of gas microbubbles and oil microdroplets, i.e. is a suspension or dispersion of individual microbubbles and microdroplets in the form of stable microbubble-microdroplet clusters. Analytical methodologies for quantitative detection and characterisation of said clusters are described in Example 1. In this text, the term "clusters" refers to groups of microbubbles and microdroplets permanently held together by electrostatic attractive forces, in a single particle, agglomerated entity. The content and size of the clusters in the cluster composition is essentially stable over some time (e.g. > 1h) after combining the first and second components in vitro, i.e. they do not spontaneously disintegrate, form larger aggregates or activate (phase shifts) spontaneously, and are essentially stable over some time after dilution, even during continued agitation. It is hence possible to detect and characterize the clusters in the cluster composition with various analytical techniques that require dilution and/or agitation. Furthermore, the stability of the cluster composition allows for performing the necessary clinical procedures (e.g. reconstitution, withdrawal of dose and administration). The first and second components, and the cluster composition, are prepared according to Good Manufacturing Practice (GMP).

Hence, in one embodiment the cluster composition comprises a suspension of clusters in an aqueous biocompatible medium, where said clusters have a mean diameter in the range 1 to 10 $\mu$m, and a circularity < 0.9 and comprises:

(i) a first component which comprises a gas microbubble and first stabilizer to stabilize said microbubble; and
(ii) a second component which comprises a microdroplet comprising an oil phase and second stabilizer to stabilize said microdroplet, where the oil comprises a diffusible component capable of diffusing into said gas microbubble so as to at least transiently increase the size thereof;

where the microbubbles and microdroplets of said first and second components have opposite surface charges and form said clusters via attractive electrostatic interactions.

After combining the two components (in vitro), e.g. by reconstituting a lyophilized microbubble component with a microdroplet component in the form of an emulsion, the prepared cluster composition according to the invention display an in-use stability which is suitable for its intended use and display stable characteristics for a suitable time window for administration, such as more than 1 hour or preferably more than 3 hours from combining the components. The cluster composition is to be administered to the subject within this time window.

Each cluster in the cluster composition comprises at least one microbubble and one microdroplet, typically 2-20 individual microbubbles/microdroplets, and a cluster typically has a mean diameter in the range of 1 to 10 $\mu$m and can hence flow freely in the vasculature. They are further characterized and separated from individual microbubbles and microdroplets by a circularity parameter. The circularity of a two-dimensional form (e.g. a projection of a microbubble, microdroplet or microbubble-microdroplet cluster) is the ratio of the perimeter of a circle with the same area as the form, divided by the actual perimeter of the form. Accordingly, a perfect circle (i.e. a two-dimensional projection of a spherical microbubble or microdroplet) has a theoretical circularity value of 1, and any other geometrical form (e.g. projection of a cluster) has a circularity of less than 1. Said clusters of the invention have a circularity < 0.9. The definition of circularity parameter is further provided in WO2015/047103.

According to the invention, compositions comprising clusters with a mean size in the range of 1-10 $\mu$m, and particularly 3-10 $\mu$m, and defined by a circularity of < 0.9 are considered particularly useful, as demonstrated by Figure 2. In one embodiment the mean cluster diameter is in the range of 3-10 $\mu$m, and preferably 4-9 $\mu$m, more preferably 5-7 $\mu$m. Clusters in this size range are free-flowing in the vasculature before activation, they are readily activated by US insonation and they produce activated bubbles that are large enough to deposit and lodge temporarily in the microvasculature, such as in the blood vessels that vascularize the CNS. The microbubbles in the clusters permit efficient energy transfer of ultrasound energy in the diagnostic frequency range (1-10 MHz), i.e. upon activation, and allow vaporisation (phase shift) of the emulsion microdroplets at low MI and diffusion of the vaporized liquid into the microbubbles and/or fusion between the vapour bubble and the microbubble. The activated bubble then expands further from the inwards diffusion of matrix gases (e.g. blood gases) to reach a volume weighted, median diameter of more than 10 $\mu$m, but less than 40 $\mu$m and typically 20 $\mu$m.

The formation of theses clusters, i.e. by preparing a cluster composition from a first component of microbubbles and a second component of microbubbles prior to administration, is a prerequisite for an efficient phase shift event and their number and size characteristics are strongly related to the efficacy of the composition, i.e. its ability to form large, activated (i.e. phased shifted) bubbles *in-vivo,* and has been found to be a pre-requisite for its intended functionality *in-vivo.* The number and size characteristics can be controlled through various formulation parameters such as, but not limited to; the strength of the attractive forces between the microbubbles in the first component and the microdroplets in the second component (e.g. the difference in surface charge between the microbubbles and microdroplets): the size distribution of microbubbles and microdroplets: the ratio between microbubbles and microdroplets: and the composition of the aqueous matrix (e.g. pH, buffer concentration, ionic strength). When the cluster composition has been prepared and is to be administered, the mean circular equivalent diameter of the clusters formed should preferably be larger than 3 $\mu$m, more preferably between 5 to 7 $\mu$m, but smaller than 10 $\mu$m.

The concentration of clusters between 3 to 10 $\mu$m in the combined preparation (cluster composition) should preferably be more than 10 million/mL, more preferably more than 20 million/mL. As shown in Example 1, a cluster composition for use according to the invention had a cluster concentration of 40 - 44 million/mL with a mean diameter of 5.8-6.2 $\mu$m measured 0-3 hours after mixing of the first and second components. In one embodiment, the composition for administration should comprise at least 3 million/ml of clusters with a diameter between 5-10 um. In another embodiment, the cluster concentration, of clusters in size range 1-10 $\mu$m, is at least around 10 million/mL.

It should be appreciated that, whereas the direct mechanism of action, i.e. the produced mechanical and/or thermal bio-effect increases delivery and enhancing distribution of the therapeutic agent, the nature of these bio-mechanical effects is a direct result of the chemical attributes of the cluster composition, i.e. a result of the chemical composition and properties of the clusters. For example, the longevity of a gas bubble in an aqueous matrix is inversely proportional to the solubility and diffusion coefficient of the gas in the matrix, and proportional to the density of the gas. Hence, a bubble made from a heavy gas with low solubility and diffusivity will grow bigger and last longer than a bubble made from a light gas with high solubility and diffusivity. As an example, a 5 $\mu$m microbubble of perfluorobutane will last 500 times longer in water than a 5 $\mu$m microbubble of air. The chemical composition of the microdroplet component will hence govern the longevity of the activated bubbles in-vivo and, hence, the level of bio-mechanical force that can be induced and the therapeutic effect level that can be achieved with the ACT procedure. From this, perfluorated oils will be particularly useful for use in microdroplets of the second component, as gases from such are very low in water solubility and diffusivity, and high in density.

If comparing the compositions and methods of the invention with methods wherein free-flowing, regular contrast microbubbles (e.g. Sonazoid) are used, the large phase shift microbubbles generated *in vivo* from the administered clusters of the current invention are entrapped in a segment of the vessels and the activated bubble surface is in close contact with the endothelium. In addition, the volume of an activated bubble is typically 1000 times that of a regular microbubble. At equal Mechanical Index (MI), insonated at a frequency close to resonance for both bubble types it has been shown that the absolute volume displacement (i.e. biomechanical force exerted) during oscillations are three orders of magnitude larger with the phase shift bubbles than with a regular contrast microbubble. Hence, insonation of phase shift bubbles will produce completely different levels of bio-mechanical effects, with significantly larger effect size and penetration depth than during insonation of regular contrast microbubbles. The bio-effects observed with free-flowing, regular contrast microbubbles are likely dependent upon cavitation mechanisms, with ensuing safety concerns such as micro-haemorrhage and irreversible vascular damage. The larger phase shift bubbles from the clusters however, can be oscillated in a softer manner (lower MI), avoiding cavitation mechanisms, but still inducing sufficient mechanical force to enhance the uptake of drug from the vasculature and into the target tissue. The trapping of the large phase shift bubbles will also act as a deposit tracer. This further allows quantification of the number of activated clusters and perfusion of the tissue and allows contrast agent imaging of the tissue vasculature to identify the spatial extent of the pathology to be treated.

The chemical composition of the administered clusters, and the processes taking place during activation of the clusters, are crucial for the effect of the clusters. For instance, chemistry of the encapsulated oil droplet influences the amount of activated bubbles that deposits upon US insonation as well as their longevity in-vivo. Physicochemical attributes of the oil, such as vapour pressure, boiling point and water solubility all correlate with the amount of activated bubbles that deposit and the time they remain deposited. For a C4-C6 homologue, perfluorinated hydrocarbon chain, the amount of activated bubbles and their longevity increase with the length of the chain, as the water solubility and vapour pressure decrease and the boiling point increases. Further it should be noted that the activated large bubbles of the clusters act mechanically on the cells of the vasculature, potentially generating biochemical signals, leading to an increased uptake of the therapeutic agent.

The cluster composition is engineered to cluster and phase shift in a controlled manner. When exposed to ultrasound, e.g. standard medical imaging frequency and intensity, at the targeted pathology, the microbubble of the administered cluster composition transfers acoustic energy to the attached oil microdroplets and may act as an evaporation seed, or merge with the microbubble so that the oil undergoes a liquid-to-gas phase shift (vaporisation). The resulting bubble undergoes an initial rapid expansion due to vaporisation of the oil, followed by a slower expansion due to inward diffusion of blood gases, and temporarily blocks the microcirculation (metarteriole and capillary network) for approximately 1 minute or more, preferably 2-3 minutes or more, most preferably 3-6 minutes or more. In the method of the invention, or in the pharmaceutical composition for use, a therapeutic agent is further administered to the subject, such as being co-administered or pre-administered or post-administered with the cluster composition. The clusters are activated to produce large bubbles by application of external ultrasound energy, and these are trapped in the microvasculature of the CNS (e.g. in the brain). Further application of low frequency ultrasound (step iv) after trapping facilitates extravasation of the therapeutic agent to the targeted tissue of the brain. Hence, the major limitation of existing technology in effectively circumventing the BBB and its function of limiting vessel permeability can be overcome by the technology of the current invention, as it has been found that the accessibility to the CNS for the therapeutic agent is considerably increased. The large, activated bubbles are hence temporarily embedded in the microvasculature of the insonated tissue and facilitate drug uptake to the CNS target tissue by further application of low power, low frequency ultrasound. The activated phase shift bubbles, being approximately 10 times larger in diameter than typical microbubbles, result in:

- transient deposition/trapping of activated bubbles in the microvasculature of the targeted (i.e. insonated) pathology;
- close contact between the activated bubbles and the endothelium;
- compared to regular contrast microbubbles; orders of magnitude larger biomechanical effects during post activation ultrasound treatment (enhancement step), avoiding inertial cavitation mechanisms.

The first component (the microbubble) of the cluster composition:
The first component comprises a gas microbubble and a first stabilizer to stabilize the microbubble. The microbubbles may be similar to conventional ultrasound contrast agents that are on the market and approved for use for several clinical applications such as Sonazoid, Optison, Definity or Sonovue, or similar agents used for preclinical application such as Micromarker and Polyson L. Any biocompatible gas may be present in the gas dispersion, the term "gas" as used herein including any substances (including mixtures) at least partially, e.g. substantially or completely in gaseous (including vapour) form at the normal human body temperature of 37 °C. The gas may thus, for example, comprise air; nitrogen; oxygen; carbon dioxide; hydrogen; an inert gas such as helium, argon, xenon or krypton; a sulphur fluoride such as sulphur hexafluoride, disulphur decafluoride or trifluoromethylsulphur pentafluoride; selenium hexafluoride; an optionally halogenated silane such as methylsilane or dimethylsilane; a low molecular weight hydrocarbon (e.g. containing up to 7 carbon atoms), for example an alkane such as methane, ethane, a propane, a butane or a pentane, a cycloalkane such as cyclopropane, cyclobutane or cyclopentane, an alkene such as ethylene, propene, propadiene or a butene, or an alkyne such as acetylene or propyne; an ether such as dimethyl ether; a ketone; an ester; a halogenated low molecular weight hydrocarbon (e.g. containing up to 7 carbon atoms); or a mixture of any of the foregoing. Preferably, the gas is a halogenated gas, and more preferably a perfluorinated gas. Advantageously at least some of the halogen atoms in halogenated gases are fluorine atoms; thus, biocompatible halogenated hydrocarbon gases may, for example, be selected from bromochlorodifluoromethane, chlorodifluoromethane, dichlorodifluoro-methane, bromotrifluoromethane, chlorotrifluoromethane, chloropenta-fluoroethane, dichlorotetrafluoroethane, chlorotrifluoroethylene, fluoroethylene, ethylfluoride, 1,1-difluoroethane and perfluorocarbons. Representative perfluorocarbons include perfluoroalkanes such as perfluoromethane, perfluoroethane, perfluoropropanes, perfluorobutanes (e.g. perfluoro-n-butane, optionally in admixture with other isomers such as perfluoro-iso-butane), perfluoropentanes, perfluorohexanes or perfluoroheptanes; perfluoroalkenes; perfluoroalkynes; and perfluorocycloalkanes.

The use of perfluorinated gases, for example sulphur hexafluoride and perfluorocarbons such as perfluoropropane, perfluorobutanes, perfluoropentanes and perfluorohexanes, are particularly advantageous in view of the recognised high stability in the bloodstream of microbubbles containing such gases. In one embodiment, the gas of the first component is selected from the group of sulphur fluorides and halogenated low molecular weight hydrocarbons (e.g. containing up to 7 carbon atoms). Other gases with physicochemical characteristics which cause them to form highly stable microbubbles in the bloodstream may likewise be useful. Most preferably, the dispersed gas comprises either of sulphur hexafluoride, perfluoropropane, perfluorobutane, perfluoropentane, perflurohexane (i.e. a C3-6 perfluorocarbon) or a mix thereof. Even more preferably, the dispersed gas comprises sulphur hexafluoride, perfluoropropane, or perfluorobutane, or mixture there. And even more preferably, the dispersed gas is perfluorobutane.

The dispersed gas may be in any convenient form, for example using any appropriate gas-containing ultrasound contrast agent formulation as the gas-containing component such as Sonazoid, Optison, Sonovue or Definity or preclinical agents such as Micromarker or PolySon L. The first component will also contain material in order to stabilize the microbubble dispersion, in this text termed 'first stabilizer'. Representative examples of such formulations include microbubbles of gas stabilized (e.g. at least partially encapsulated) by a first stabilizer such as a coalescence-resistant surface membrane (for example gelatin), a filmogenic protein (for example an albumin such as human serum albumin), a polymer material (for example a synthetic biodegradable polymer, an elastic interfacial synthetic polymer membrane, a microparticulate biodegradable polyaldehyde, a microparticulate N-dicarboxylic acid derivative of a polyamino acid - polycyclic imide), a non-polymeric and non-polymerisable wall-forming material, or a surfactant (for example a polyoxyethylene-polyoxypropylene block copolymer surfactant such as a Pluronic, a polymer surfactant, or a film-forming surfactant such as a phospholipid). Preferably, the dispersed gas is in the form of phospholipid-, protein- or polymer-stabilized gas microbubbles. Hence, in one embodiment, the first stabilizer is selected from the group of phospholipids, proteins and polymers. A particularly useful first stabilizer is selected from the group of phospholipids, particularly comprising molecules with net overall negative charge, such as naturally occurring (e.g. soya bean or egg yolk derived), semisynthetic (e.g. partially or fully hydrogenated) and synthetic phosphatidyl-serines, phosphatidylglycerols, phosphatidylinositols, phosphatidic acids and/or cardiolipins. Alternatively, the phospholipids applied for stabilization may carry an overall neutral charge and be added a negative surfactant such as a fatty acid, e.g. phosphatidylcholine added palmitic acid, or be a mix of differently charged phospholipids, e.g. phosphatidylethanolamines and/or phosphatidylcholine and/or phosphatidic acid. For the first stabilizer, i.e. stabilizing the microbubble of the first component, different examples are demonstrated in WO2015/047103, Example 5, and Tables 9 and 10, wherein various microbubble formulations with different excipients have been tested. The results demonstrate that the ACT concept used in the current invention is applicable to a wide variety of microbubble formulations, also with regards to the composition of the stabilizing membrane.

The microbubble size of the dispersed gas component should preferably be less than 7 $\mu$m, more preferably less than 5 $\mu$m and most preferably less than 3 $\mu$m in order to facilitate unimpeded passage through the pulmonary system, even when in a microbubble-microdroplet cluster.

The second component (microdroplet) of the cluster composition:

The second component comprises a microdroplet comprising an oil phase and a second stabilizer to stabilize said microdroplet, where the oil comprises a diffusible component. This diffusible component is capable of diffusing into the gas microbubble of the first component so as to at least transiently increase the size thereof. For the second component the "diffusible component" is suitably a gas/vapour, volatile liquid, volatile solid or precursor thereof capable of gas generation, e.g. upon administration, the principal requirement being that the component should either have or be capable of generating a sufficient gas or vapour pressure *in vivo* (e.g. at least 50 torr and preferably greater than 100 torr) so as to be capable of promoting inward diffusion of gas or vapour molecules into the dispersed gas. The 'diffusible component' is preferably formulated as an emulsion (i.e. a stabilized suspension) of microdroplets in an appropriate aqueous medium, since in such systems the vapour pressure in the aqueous phase of the diffusible component will be substantially equal to that of pure component material, even in very dilute emulsions.

The diffusible component in such microdroplets is advantageously a liquid at processing and storage temperature, which may for example be as low as -10 °C if the aqueous phase contains appropriate antifreeze material, while being a gas or exhibiting a substantial vapour pressure at body temperature. Appropriate compounds may, for example, be selected from emulsifiable low boiling liquids. Specific examples of emulsifiable diffusible components include aliphatic ethers such as diethyl ether; polycyclic oils or alcohols such as menthol, camphor or eucalyptol; heterocyclic compounds such as furan or dioxane; aliphatic hydrocarbons, which may be saturated or unsaturated and straight chained or branched,; cycloaliphatic hydrocarbons such as cyclobutane, cyclobutene, methylcyclopropane or cyclopentane; and halogenated low molecular weight hydrocarbons, e.g. containing up to 7 carbon atoms. Representative halogenated hydrocarbons include dichloromethane, methyl bromide, 1,2-dichloroethylene, 1,1-dichloroethane, 1-bromoethylene, 1-chloroethylene, ethyl bromide, ethyl chloride, 1-chloropropene, 3-chloropropene, 1-chloropropane, 2-chloropropane and t-butyl chloride. Advantageously at least some of the halogen atoms are fluorine atoms, for example as in dichlorofluoromethane, trichlorofluoromethane, 1,2-dichloro-1,2-difluoroethane, 1,2-dichloro-1,1,2,2-tetrafluoroethane, 1,1,2-trichloro-1,2,2-trifluoroethane, 2-bromo-2-chloro-1,1,1-trifluoroethane, 2-chloro-1,1,2-trifluoroethyl difluoromethyl ether, 1-chloro-2,2,2-trifluoroethyl difluoromethyl ether, partially fluorinated alkanes (e.g. pentafluoropropanes such as 1H, 1H,3H-pentafluoropropane, hexafluorobutanes, nonafluorobutanes such as 2H-nonafluoro-t-butane, and decafluoropentanes such as 2H,3H-decafluoropentane), partially fluorinated alkenes (e.g. heptafluoropentenes such as 1H,1H,2H-heptafluoropent-1-ene, and nonafluorohexenes such as 1H,1H,2H-nonafluorohex-1-ene), fluorinated ethers (e.g. 2,2,3,3,3-pentafluoropropyl methyl ether or 2,2,3,3,3-pentafluoropropyl difluoromethyl ether) and, more preferably, perfluorocarbons. Examples of perfluorocarbons include perfluoroalkanes such as perfluorobutanes, perfluoropentanes, perfluorohexanes (e.g. perfluoro-2-methylpentane), perfluoroheptanes, perfluorooctanes, perfluorononanes and perfluorodecanes; perfluorocycloalkanes such as perfluorocyclobutane, perfluorodimethyl-cyclobutanes, perfluorocyclopentane and perfluoromethyl-cyclopentane; perfluoroalkenes such as perfluorobutenes (e.g. perfluorobut-2-ene or perfluorobuta-1,3-diene), perfluoropentenes (e.g. perfluoropent-1-ene) and perfluorohexenes (e.g. perfluoro-2-methylpent-2-ene or perfluoro-4-methylpent-2-ene); perfluorocycloalkenes such as perfluorocyclopentene or perfluoro-cyclopentadiene; and perfluorinated alcohols such as perfluoro-t-butanol. Hence, the oil (the diffusible component) of the second component may be selected from the group of aliphatic ethers, heterocyclic compounds, aliphatic hydrocarbons, halogenated low molecular weight hydrocarbons and perfluorocarbons. In one embodiment, the oil phase of the second component comprises a perfluorocarbon.

Particularly useful in the current invention are diffusible components with an aqueous solubility below $1 \cdot 10^{-4}$ M, more preferably below $1 \cdot 10^{-5}$ M. It should be noted, however, that if a mixture of diffusible components and/or co-solvents are used, a substantial fraction of the mixture may contain compounds with a higher water solubility. Based on the water solubility, examples of suitable oils (diffusible components) are: perfluorodimethylcyclobutane, perfluoromethylcylopentane, 2-(trifluoromethyl)perfluoropentane and perfluorhexane. It will be appreciated that mixtures of two or more diffusible components may if desired be employed in accordance with the invention; references herein to "the diffusible component" are to be interpreted as including such mixtures.

The second component will also contain material in order to stabilize the microdroplet dispersion, in this text termed 'second stabilizer'. The second stabilizer may be the same as or different from any materials(s) used to stabilize the gas dispersion, e.g. a surfactant, such as a phospholipid, a polymer or a protein. The nature of any such material may significantly affect factors such as the rate of growth of the dispersed gas phase. In general, a wide range of surfactants may be useful as stabilizers. Representative examples of useful surfactants (stabilizers) include fatty acids (e.g. straight chain saturated or unsaturated fatty acids, for example containing 10-20 carbon atoms) and carbohydrate and triglyceride esters thereof, phospholipids (e.g. lecithin), fluorine-containing phospholipids, proteins (e.g. albumins such as human serum albumin), polyethylene glycols, and polymer such as a block copolymer surfactants (e.g. polyoxyethylene-polyoxypropylene block copolymers such as Pluronics, extended polymers such as acyloxyacyl polyethylene glycols, for example polyethyleneglycol methyl ether 16-hexadecanoyloxy-hexadecanoate, e.g. wherein the polyethylene glycol moiety has a molecular weight of 2300, 5000 or 10000), and fluorine-containing surfactants (e.g. as marketed under the trade names Zonyl and Fluorad. Particularly useful surfactants include phospholipids, and particularly phospholipids comprising molecules with overall neutral charge, e.g. distearoyl-sn-glycerol-phosphocholine (DSPC). For the second component, a range of different stabilizers may be used to stabilize the microdroplet. Further, a wide range of ionic, preferably cationic, substances may be used in order to facilitate a suitable surface charge.

It will be appreciated that, to facilitate attractive electrostatic interactions to achieve clustering between the microbubbles in the first component and the emulsion microdroplets in the second component, these should be of opposite surface charge. Hence, if the microbubbles of the first component are negatively charged, the microdroplets of the second component should be positively charged, or vice versa. In a preferred embodiment, the surface charge of the microbubbles of the first component is negative, and the surface charge of the microdroplets of the second component is positive. In order to facilitate a suitable surface charge for the oil microdroplets a cationic surfactant may be added to the stabilizing structure. A wide range of cationic substances may be used, for example at least somewhat hydrophobic and/or substantially water-insoluble compounds having a basic nitrogen atom, e.g. primary, secondary or tertiary amines and alkaloids. A particularly useful cationic surfactant is stearylamine. In one embodiment, the second stabilizer is a neutral phospholipid added a cationic surfactant, such as a DSPC-membrane with stearylamine.

In one embodiment, the first stabilizer and the second stabilizer each independently comprises a phospholipid, a protein, a polymer, a polyethyleneglycol, a fatty acid, a positively charged surfactant, or a negatively charged surfactant or mixtures thereof. More particularly, the first stabilizer comprises a phospholipid, a protein, or a polymer having a negatively charge, and the second stabilizer comprises a phospholipid, protein, or a polymer and a positively charged surfactant.

In one embodiment, the first component comprises a dispersed gas selected from the group of sulphur hexafluoride, perfluoropropane, perfluorobutane, perfluoropentane and perflurohexane or a mix thereof, stabilized by a first stabilizer selected from the group of phospholipids, proteins and polymers; the second component comprises a diffusible component selected from the group of perfluorocarbons, e.g. a perfluorocycloalkane, stabilized with a second stabilizer selected from the group of surfactants, e.g. including phospholipids, polymers and proteins. More specifically, either of the stabilizers are selected from phospholipids.

The first and second components are combined shortly before the intended use, to prepare the cluster composition, and for use in an appropriate time window. It will also be appreciated that the mixing of the first and second components can be achieved in various manners depended on the form of the components; e.g. mixing two fluid components, reconstitution of one component in dry powder form with one component in fluid form, mixing two components in dry form prior to reconstitution with fluid (e.g. water for injection or buffer solution). Hence, in one embodiment of the invention, the method comprises a step of preparing the microbubble-microdroplet cluster composition prior to the administration step (step i). In a preferred embodiment, the microbubble-microdroplet cluster composition is prepared by reconstitution of the first component (microbubbles) in dry powder form with the second component (microdroplets) in fluid form. More particularly a first vial comprising the first component is reconstituted with the second component withdrawn from a second vial, using a sterile, single use syringe and needle. The content of the syringe is to be added through a stopper of the first vial and the resulting cluster composition is homogenised, e.g. by manual mixing.

It will be appreciated that also other components may influence the ability of the microbubbles and microdroplets to form clusters upon mixing including, but not limited to; the level of surface charge of the microbubbles/microdroplets, the concentration of the microbubbles/microdroplets in the two components, the size of the microbubbles/microdroplets, the composition and concentration of ions in the liquid matrix, the pH, the composition and concentration of excipients (e.g. buffer or tonicity components) etc. (see WO2015/047103, Example 1). Such characteristics of the components and the composition may also influence the size and stability (both in-vitro and in-vivo) of the clusters generated and may be important factors influencing biological attributes (e.g. efficacy and safety profile). It is also appreciated that not all of the microbubbles/microdroplets in the cluster composition may be present in clustered form, but that a substantial fraction of the microbubbles and/or microdroplets may be present together in a free (non-clustered) form together with a population of microbubble-microdroplet clusters. In addition, the way the two components are mixed may influence these aspects, including, but not limited to; shear stress applied during homogenization (e.g. soft manual homogenization or strong mechanical homogenization) and time range for homogenization. The cluster composition is to be administered to the subject during a time window wherein the characteristics of the clusters are substantially unchanged, such as within 5 hours, such as within 3 hours, from combining the two components. In-use stability studies of the applicant show that the clusters display stable characteristics for at least 3 hours, cf. Example 1.

**Administration routes:**

The cluster composition is administered to said mammalian subject parenterally, preferably intravenously. The route of administration might also be selected from the intra-arterial, intramuscular, intraperitoneal, intratumoral or subcutaneous administration. For administration to the subject, the therapeutic agent is pre-, and/or co- and/or post administered separate to the cluster composition as a separate composition. The therapeutic agent is administered according to the respective approved Summary of Product Characteristics. Typically, the route is selected from the group comprising, but not limited to, intravenous, intraperitoneal, intratumoral, nasal and intramuscular administration. In one embodiment, the therapeutic agent is administered from a surgically implanted drug depo in the brain. The two compositions, i.e. the cluster composition (a) and the therapeutic agent composition(s) (b) may hence be administered via the same or via different routes of administration.

**Treatment schedules:**

It will be appreciated that the composition for use, the method for treatment, and/or the method for delivery of drugs, of the invention, may e.g. be employed as part of a multi-drug treatment regimen. In one embodiment, the pharmaceutical composition for use according to the invention, includes the use of more than one therapeutic agent.

Furthermore, in one embodiment, several ACT treatments can be performed during the period of administering the therapeutic agents, e.g. as exemplified in Figure 13. In one embodiment, the method of treatment includes 1 to 5, such as 2 to 4, ACT treatments. The "ACT treatment" or "ACT procedure" includes at least the administration of a cluster composition, the activation of the clusters by regular medical imaging US insonation and the following, low frequency US insonation to induce enhanced uptake.

Figure 13 provides a graph of possible ACT treatments performed during treatment with the combination regimen comprising the Standard of Care combination immunotherapy plus chemotherapy regimen for treatment of cancer; pembrolizumab followed by paclitaxel and cisplatin. Panel A: ACT procedure comprising a.: injection of the cluster composition, b.: activation step with e.g. 60 second of ultrasound insonation, and c.: enhancement step with e.g. 5 minutes of ultrasound insonation. Panel B: y-axis showing plasma concentration of the administered therapeutic agents in percent of peak and x-axis showing time in minutes. In this example three ACT procedures are performed at approximately 160 minutes, 200 minutes and 240 minutes in order to cover all three drugs and provide treatment of the entire region of interest.

The inventors have found that it is beneficial to repeat the ACT procedure, multiple times, even when a single therapeutic agent is administered. Using US imaging during activation of ACT, the inventors have observed a notable effect in deposition of ACT bubbles in tumours. A strong variance in the deposition pattern from injection to injection in the same animal was observed; the density of deposited ACT bubbles differed between various segments of the tumour, and this pattern changed between injections. Although not fully elucidated, it is hypothesised that these effects are due to a temporal variation in perfusion for various tumour segments. Based on these observations, in order to reach as much of a tumour volume as possible, the inventors have found it useful to apply the ACT procedure several consecutive times, such as three times, back-to-back. This also points to the benefit of applying several ACT procedures during clinical use, as noted above for the regimen visualized in Figure 13.

Hence, in one embodiment, more than one therapeutic agent, such as 1 to 5 therapeutic agents, are administered simultaneously or sequentially over a certain time span, such as over up to 3 hours, wherein at least one, such as 1 to 5, ACT treatments are performed during the same period.

In one embodiment, the following ACT procedure is provided:
The administration, such as an intravenous administration, of a cluster composition is followed by local US insonation of the target area with regular medical imaging US (activation), followed by low frequency US insonation to induce extravasation, distribution and uptake of drug, and these steps are performed 2-5 consecutive times, such as 3 consecutive times. Hence, the steps (i) to (iv) of the ACT treatment are repeated one to four times. This is performed in conjunction with administration of therapeutic agents. The activation, i.e. the initial US insonation, should start immediately after each administration of the cluster composition, such as within one minute, and lasts for e.g. 30-120 seconds. The insonation with low frequency ultrasound follows the activation step and should typically last for 3 to 10 minutes, such as for about 5 minutes. There is preferably an immediate start of step (iv) after step (iii). A dual frequency transducer may beneficially be used in the treatment, for both the activation step and the enhancement step, irrespective of insonation approach. By using such, the switch from the activation insonation in step (iii) to the enhancement insonation in step (iv) can be made without any delay. Application of the enhancement field immediately after activation may be important for the resulting therapeutic benefit. In this respect it would be beneficial to apply both the activation and the enhancement insonation using a broad band or dual frequency US transducer. I.e. a transducer capable of delivering sufficient US pressure (i.e. MI) over all frequencies required by the stated preferred ranges should be used. E.g. a transducer capable of delivering MIs of up to 0.4 at both 1 to 10 MHz and at 0.1 to 1 MHz should be used.

In another embodiment, the method comprises a multidrug regimen. Hence, several therapeutic drugs can be used, and several ACT procedures can be applied during the treatment regimen. In a preferred embodiment, the ACT procedure is performed when the active therapeutic molecule displays maximum or close to maximum concentration in the blood after administration. Hence, the timing of the ACT treatment(s) may vary dependent upon the pharmacokinetics of the therapeutic agent.

The therapeutic agent(s) are pre-, and/or co- and/or post administered separate to the cluster composition. In a preferred embodiment, a therapeutic agent is administered after the administration of one of the at least one cluster compositions. Surprisingly, it has been found that performing the ACT treatment, i.e. the administration and insonation of the clusters, before administration of the therapeutic agent showed similar effect size as if the ACT treatment was initiated after administration of the therapeutic agent (i.e. when the therapeutic agent is in the blood stream). This finding indicates that the bio-mechanical effects induced by ACT, i.e. the increased permeability of the vascular barrier, last for some time after the US procedure has terminated. This may be beneficial in clinical practice, as the ACT treatment may be performed prior to starting the therapeutic administration and treatment. Hence, in one embodiment, a therapeutic agent is administered after the cluster composition has been administered and US insonated *in-vivo*. In another embodiment, the cluster composition is administered either immediately prior to or after administration of therapeutic agent(s).

Further disclosed herein but not encompassed by the wording of the claims is a microbubble-microdroplet cluster composition for use in a method of localised delivery of at least one therapeutic agent to the CNS of a subject, wherein the method comprises Acoustic Cluster Therapy (ACT) treatment wherein at least one step of ultrasound insonation is performed either non-invasively towards the CNS, invasively towards the CNS or towards the heart or a spinal or carotid artery outside the CNS, wherein the method comprises the steps of:

(i) administering a microbubble-microdroplet cluster composition to the subject; wherein the least one therapeutic agent is pre-, and/or co- and/or post administered separate to the cluster composition;
(ii) optionally imaging the clusters of the cluster composition using ultrasound imaging to identify a region of interest in the CNS for treatment within said subject;
(iii) activating a phase shift of a diffusible component of the microdroplet of the cluster composition from step (i) by ultrasound insonation at a first frequency and a first mechanical index; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column; and
c) for the insonation towards the heart or a spinal or carotid artery, this takes place in the heart or in a spinal or carotid artery outside the CNS;

(iv) further insonating with ultrasound at a second frequency and a second mechanical index facilitating extravasation of the at least one therapeutic agent administered in step (i) at the region of interest in the CNS; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column;
c) when activating according to c) in step (iii), further insonation in this step takes place either non-invasively or invasively according to step (iv) a) or step (iv) b).

Further disclosed herein but not encompassed by the wording of the claims is a microbubble-microdroplet cluster composition for use in a method of increasing the permeability of the blood-brain barrier of a subject, such as for a therapeutic agent, wherein the method comprises Acoustic Cluster Therapy (ACT) treatment wherein at least one step of ultrasound insonation is performed either non-invasively towards the CNS, invasively towards the CNS or towards the heart or a spinal or carotid artery outside the CNS, wherein the method comprises the steps of:

(i) administering a microbubble-microdroplet cluster composition to the subject;
(ii) optionally imaging the clusters of the pharmaceutical composition using ultrasound imaging to identify a region of interest in the CNS for treatment within said subject;
(iii) activating a phase shift of a diffusible component of the microdroplet of the cluster composition from step (i) by ultrasound insonation at a first frequency and a first mechanical index; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column; and
c) for the insonation towards the heart or a spinal or carotid artery, this takes place in the heart or in a spinal or carotid artery outside the CNS;

(iv) further insonating with ultrasound at a second frequency and a second mechanical index facilitating extravasation of the at least one therapeutic agent administered in step (i) at the region of interest in the CNS; wherein

a) for the non-invasive insonation, this takes place from at least one transducer positioned outside the brain skull or vertebral column of the subject for insonation through this; and
b) for the invasive insonation, this takes place from at least one surgically implanted transducer in the subject's brain skull or vertebral column;
c) when activating according to c) in step (iii), further insonation in this step takes place either non-invasively or invasively according to step (iv) a) or step (iv) b).

Hence, in some embodiments, only the cluster composition without a therapeutic agent is administered to a subject. This may be for the preparation of a subject for a subsequent administration of a therapeutic agent. In such embodiments, the administration of the cluster composition is such that the administration is not a treatment, but a preparation for a treatment, such as a preparation for a treatment with a therapeutic agent and to allow for safe passage of such to a target location of the CNS.

The invention shall not be limited to the shown embodiments and examples. While various embodiments of the present disclosure are described herein, it will be obvious to those skilled in the art that such embodiments are provided by way of example only. Numerous modifications and changes to, and variations and substitutions of, the embodiments described herein will be apparent to those skilled in the art without departing from the disclosure. It is to be understood that various alternatives to the embodiments described herein can be employed in practicing the disclosure.

It is to be understood that every embodiment disclosed for one aspect equally well apply for the other aspects. Hence, for example the features disclosed for the composition for use in therapy are also useful for the method of localised delivery and for the method of increasing the permeability of the BBB, and for the method of treatment which are not encompassed by the wording of the claims.

It is to be understood that every embodiment of the disclosure can optionally be combined with any one or more of the other embodiments described herein.

It is to be understood that each component, compound, or parameter disclosed herein is to be interpreted as being disclosed for use alone or in combination with one or more of each and every other component, compound, or parameter disclosed herein. It is further to be understood that each amount/value or range of amounts/values for each component, compound, or parameter disclosed herein is to be interpreted as also being disclosed in combination with each amount/value or range of amounts/values disclosed for any other component(s), compound(s), or parameter(s) disclosed herein, and that any combination of amounts/values or ranges of amounts/values for two or more component(s), compound(s), or parameter(s) disclosed herein are thus also disclosed in combination with each other for the purposes of this description. Any and all features described herein, and combinations of such features, are included within the scope of the present invention provided that the features are not mutually inconsistent.

It is to be understood that each lower limit of each range disclosed herein is to be interpreted as disclosed in combination with each upper limit of each range disclosed herein for the same component, compound, or parameter. Thus, a disclosure of two ranges is to be interpreted as a disclosure of four ranges derived by combining each lower limit of each range with each upper limit of each range. A disclosure of three ranges is to be interpreted as a disclosure of nine ranges derived by combining each lower limit of each range with each upper limit of each range, etc. Furthermore, specific amounts/values of a component, compound, or parameter disclosed in the description or an example is to be interpreted as a disclosure of either a lower or an upper limit of a range and thus can be combined with any other lower or upper limit of a range or specific amount/value for the same component, compound, or parameter disclosed elsewhere in the application to form a range for that component, compound, or parameter.

## Examples:

### Example 1. Cluster preparation, analytical tools and basic characteristics

The microbubble-microdroplet clusters formed upon combining C1 and C2, i.e. present in DP, are crucial to the critical quality attributes of the composition, i.e. its functionality for delivery of drugs. Hence, analytical methodology to characterize and control the clusters formed with regards to concentration and size, is an imperative tool to assess the current invention as well as for medicinal Quality Control (QC). We have identified three different analytical tools that can be applied for this purpose; Coulter counting, Flow Particle Image Analysis (FPIA) and Microscopy/Image analysis.

In addition to these techniques, applied for characterization of the clusters in the cluster composition, analytical methodology has been developed to study the activation of the clusters in vitro, i.e. the generation of large, activated bubbles upon ultrasound irradiation. This methodology; "Sonometry" is detailed in E1-6 of WO2015/047103. Primary report responses from the Sonometry analysis are attenuation spectra and number and volume of activated bubbles and their size distribution, both vs. time after activation. Activation responses may also be explored by Microscopy/Image analysis as detailed in E1-5 of WO2015/047103.

### Components and compositions:

The 1st component (C1) in the compositions investigated in the included example consisted of per-fluorobutane (PFB) microbubbles stabilized by a hydrogenated egg phosphatidyl serine-sodium (HEPS-Na) membrane and embedded in lyophilized sucrose. HEPS-Na carries a negatively charged head group with an ensuing negative surface charge of the microbubbles. Each vial of C1 contains approximately 16 $\mu$L or $2.10^9$ microbubbles, with a mean diameter of approximately 2.0 $\mu$m. The freeze-dried formulation displays long shelf life, more particularly 3 years, stored at ambient room temperature.

The 2nd component (C2) in the compositions investigated in this example consisted of perfluoromethyl-cyclopentane (pFMCP) microdroplets stabilized by a 1,2-Distearoyl-sn-glycerol-3-phosphocholine (DSPC) membrane with 3% mol/mol stearylamine (SA) added to provide a positive surface charge. The microdroplets in the C2 were dispersed in 5 mM TRIS buffer. The standard formulation of C2 investigated in these studies contains approximately 4 $\mu$L or $0.8 \cdot 10^9$ microdroplets per mL, with a mean diameter of approximately 1.8 $\mu$m. The 2nd component displays long shelf life, more particularly 18 months or more, stored refrigerated.

In some cases, to elucidate effects on cluster characteristics, a variety of formulation variables such as SA content, microdroplet size, microdroplet concentration, TRIS concentration and pH was varied in a controlled manner. In case such samples have been used, these aspects are detailed in the text.

The cluster composition (DP) was prepared aseptically by reconstituting a vial of C1 with 2 mL of C2 followed by 30 seconds of manual homogenisation. 2 mL was withdrawn from a vial of C2 using a sterile, single use syringe and needle. The content of the syringe was added through the stopper of a vial of C1 and the resulting DP was homogenised preparing the composition for administration.

As shown in WO2015/047103, the first and second components, i.e. the microbubble formulation and the microdroplet formulation, can be varied. E.g. as shown in tables 9 and 10 of WO2015/047103 both the gas and the stabilising membrane of the first component can be varied, to prepare clusters with suitable properties, expected to be useful in treatment according to the invention.

**Stability of clusters in the cluster composition during analysis:**

The clusters in the DP are formed and held together by the electrostatic attraction between the microbubbles and the microdroplets. These forces are finite, and the clusters may break up after formation through various routes/influences such as mechanical stress or thermal (Brownian) motion. For precise and accurate characterization, it is important that the clusters remain stable during the time of analysis. This stability has been investigated with all the methodologies described above. To evaluate stability, 3 to 5 analyses where repeated on a single DP sample covering a timespan of > 5 minutes. No significant change in neither concentration nor size has been observed cross these replicates, proving that the microbubbles, microdroplets and clusters are stable for > 5 minutes under the analytical conditions stated, i.e. after dilution in PBS or water and under continuous homogenization (stirring).

**Formulation aspects:**

A number of different formulation aspects can be explored for controlling the cluster content and size in the DP and for targeting optimal properties. Parameters that can be used to engineer cluster content and size distribution include, but are not limited to; the difference in surface charge between the microbubbles and the microdroplets e.g. SA%: the microdroplet size of C2: the pH: the concentration of TRIS in C2: and the concentration of microbubbles and microdroplets. In addition, chemical degradation of the components, e.g. during prolonged storage at high temperatures, may influence the ability of C1 and C2 to form clusters during preparation of the DP.

From *in-vitro* characterisation of 30 different compositions, as reported in WO2015/047103, several important correlations that elucidate the nature and characteristics of the system can be extracted. We found that the size of the clusters formed is also strongly connected to the Reactivity of the system. Only small clusters (i.e. 1- 5 um) and medium sized clusters (i.e. 5-10 $\mu$m) are formed at relatively low levels of Reactivity (e.g. < 20%). With increasing Reactivity, larger clusters start to form; at R > approx. 20%, 10-20 $\mu$m clusters start to form and at R > approx. 50%, 20-40 $\mu$m clusters start to form. When larger clusters form, it is at the expense of smaller and medium sized clusters; we found a clear optimum in content vs. Reactivity for cluster concentration 1- 5 $\mu$m and 5 -10 $\mu$m. We found that formation of larger clusters (i.e. larger than 10 um, or larger than 20 um) is detrimental to the efficacy of the composition and that the clustering potential must be balanced accordingly.

Based on applicant's experiments, and the results shown in Tables 5 and 6 of WO2015/047103, the efficacy (linear enhancement in Grey Scale units (GS)) of the cluster composition is correlated with the cluster mean size and the concentration of clusters (million/ml). Grey Scale enhancement is the increase in brightness (contrast) observed by US imaging after administration and activation of the cluster composition *in-vivo* and is a measure of the amount of activated bubbles in the imaged tissue. The results reported there are from a multivariate, principal component analysis (PCA) of the contribution of clusters in various size classes to the linear enhancement in the ultrasound signal from dog myocardium (Grey Scale units) upon i.v. administration of the cluster composition and activation in the left ventricle, please see Example 2 of WO2015/047103. The PCA was performed on data for 30 samples detailed in Tables 5 and 6 of this. The results demonstrate that small and medium sized clusters (< 10 $\mu$m) contribute significantly to the efficacy of the cluster composition whereas larger clusters (> 10 $\mu$m) do not. These results and conclusion also apply for the current invention. The results from the PCA analysis are stated in Table 1 below. Figure 2 shows a visualization of cluster size versus product efficacy based on the data in Table 1, demonstrating that clusters having a mean diameter in the range of 3 to 10 $\mu$m have an optimal efficacy. Hence, in Figure 2 product efficacy vs. cluster diameter is provided. Y-axis shows correlation coefficient to Grey Scale enhancement from US imaging of dog myocardium after injection and activation of clusters in the left ventricle and reflects the amount of activated bubbles deposited. X-axis shows cluster diameter in $\mu$m. Grey boxes represent the different cluster size bins evaluated: 1 to 5 $\mu$m, 5 to 10 $\mu$m, 10 to 20 $\mu$m and 20 to 40 $\mu$m. Solid line represents the continuous function of efficacy vs. cluster diameter. Error bars are standard error. Figure 2 is an alternative visualization of Figure 12 (left side) of WO2015/047103. As can be observed, the mean cluster diameter should be in the range of 3-10 $\mu$m, and preferably 4-9 $\mu$m, more preferably 5-7 $\mu$m.

**Table 1: Efficacy of clusters vs. mean diameter as reported in WO2015/047103.**

| Channel Group | Mean Channel Diameter ($\mu$m) | Efficacy Coefficient |
|---|---|---|
| 1 to 5 $\mu$m | 3 | 0.4 |
| 5 to 10 $\mu$m | 7.5 | 0.55 |
| 10 to 20 $\mu$m | 15 | 0.12 |
| 20 to 40 $\mu$m | 30 | -0.15 |

The cluster concentration and mean diameter of the cluster composition, prepared according to Example 1, was analysed and found to have a cluster concentration of about 40-44 million per mL and with a cluster mean diameter of about 5.8-6.2 $\mu$m, for several hours. The results are shown in Table 2 below and are consistent with the results of Table 6 of WO2015/047103. The data of Table 2 shows that the prepared cluster composition has an acceptable stability, and that an optimal size and concentration of clusters can be achieved.

**Table 2: Cluster concentration and mean diameter at various timepoints after preparation of the cluster composition.**

| Time (hours) | Cluster Concentration (millions/mL) | Cluster Mean Diameter ($\mu$m) |
|---|---|---|
| 0h | 44 $\pm$ 2 | 6.0 $\pm$ 0.2 |
| 1h | 43 $\pm$ 1 | 5.8 $\pm$ 0.2 |
| 2h | 44 $\pm$ 5 | 6.2 $\pm$ 0.1 |
| 3h | 40 $\pm$ 1 | 6.0 $\pm$ 0.2 |

Applying the concept of the present invention, i.e. by preparing a cluster composition from C1 and C2 prior to administration, hence forming microbubble-microdroplet clusters, as opposed to co-injection of the two components as taught by WO/9953963, enable a > 10-fold increase in efficacy. The formation of microbubble-microdroplet clusters upon combination of the 1st component and 2nd component, and administering these pre-made clusters, is a pre-requisite for its intended functionality *in-vivo.* The cluster composition is to be administered to the subject during a time window wherein the characteristics of the clusters are substantially unchanged, such as within 3 hours from combining the two components.

**Example 2. Frequency and Mechanical Index for the Enhancement step.**

As earlier noted, the application of further US insonation after activation of the large ACT bubbles, i.e. the Enhancement step, leads to an increase in extravasation of drug from the vascular compartment to the targeted tissue interstitium.

However, the attributes of this Enhancement field with regards to frequency and MI may strongly influence the efficacy of the procedure. A certain minimum level of biomechanical effects is needed in order for increased permeability to be induced but, if too strong, inertial cavitation mechanisms may be induced with ensuing vascular damage and a reduction in efficacy.

The level of bubble oscillations will be depended on several parameters, most importantly the US frequency and pressure, the latter defined by the mechanical index (MI). In order to study the effect of frequency and MI on the nature of the induced bubble oscillations and on the efficacy of the ACT procedure, 5 studies have been performed:

- The attenuation spectrum of a population of activated bubbles was measured. The radial oscillations of a typical activated bubble with a resting diameter of 20 $\mu$m, induced during the Enhancement step, have been modelled using the modified Rayleigh-Plesset model [Postema and Schmitz, Ultrasonic bubbles in medicine: influence of the shell, Ultrason Sonochem, 2007. 14(4): p. 438-44] for a series of frequencies and MIs.
- The tissue uptake of a drug mimicking chromophore (Evans Blue) has been explored as a function of MI with an Enhancement step US frequency of 0.5 MHz. For this study, the bubble oscillations were modelled using the modified Rayleigh-Plesset model.
- The therapeutic efficacy, treating prostate cancer in mice with nab-paclitaxel $\pm$ ACT, has been explored with an Enhancement MI of 0.2 at both 0.5 and 0.9 MHz.

- The therapeutic efficacy, treating breast cancer in mice with nab-paclitaxel ± ACT, has been explored with an Enhancement frequency of 0.5 MHz at MIs of 0.1 and 0.2.

**Methods:**

The cluster composition investigated in these studies was as detailed in Example 1.

The attenuation spectrum of a population of activated bubbles was measured by Sonometry, as detailed in E1-6 of WO2015/047103.

Modelling of bubble oscillations as a function of frequency and MI was performed by solving the partial differential modified Rayleigh-Plesset equation in MATLAB 2020b (MathWorks, Natick, MA, USA). Specifically, a 20 μm diameter bubble was modelled in blood using a negligible shell stiffness and C4F10 gas properties. A linear ultrasound pulse was mimicked using a sinusoidal wave with a 3 cycle start and end gaussian ramp.

To investigate the effect of MI variance of the US Enhancement field, tumour specific uptake of Evans Blue (EB, fluorescent dye) has been investigated in a subcutaneous prostate cancer model (PC3) in mice. Five groups with Enhancement insonation MIs of 0, 0.1, 0.2, 0.3 and 0.4 were investigated (N=3 animals per group). Immediately after i.v. injection of EB, a single dose of cluster composition (2 mL/kg, (i.v.)) was given followed by 45 sec Activation US (2.25 MHz, MI 0.4) and 5 min Enhancement US (0.5 MHz, variable MI), focused to the tumor volume. 30 min after treatment, the tumors were excised and the amount of EB was measured by spectrophotometry at 620 nm.

The therapeutic effect of nab-paclitaxel (Abraxane®, ABR) ± ACT with 2 mL cluster dispersion/kg, for treatment of human prostate cancer in mice, was investigated in a subcutaneous prostate cancer model (PC3). ABR (12 mg/kg, i.v.) was administered weekly for 4 weeks, each time immediately followed by three, back to back, ACT procedures. N=9 to 12 animals per group. In addition, a saline control group was performed (N=4). Activation US consisted of 45s insonation at 2.5 MHz, MI=0.4 and Enhancement US consisted of 5 minutes at 0.5 or 0.9 MHz, both with MIs of 0.2. End point was overall survival. Animals were culled when the tumor reached a maximum volume of 1000 mm$^3$.

The therapeutic effect of nab-paclitaxel (Abraxane®, ABR) ± ACT with 2 mL cluster dispersion/kg, for treatment of human breast cancer in mice, was investigated in a subcutaneous breast cancer model (Ca-MDA-MB231). ABR (12 mg/kg, i.v.) was administered weekly for 4 weeks, each time immediately followed by three, back-to-back, ACT procedures at MIs of 0.1 or 0.2. In addition, a drug alone group was investigated. N=9 to 12 animals per group. Activation US consisted of 45s insonation at 8 MHz MI=0.33 and Enhancement US consisted of 5 minutes at 0.5 MHz with MIs of 0.1 or 0.2. End point was tumor volume measured with caliper (each day), normalized by tumor volume at first day of treatment. Animals were culled when the tumor reached a maximum volume of 1000 mm$^3$.

**Results:**

**Attenuation spectrum of activated bubbles.**

The attenuation spectrum of a typical population of activated ACT bubbles was measured and results are visualized in Figure 3. As can be noted, the resonance frequency was determined to approx. 0.3 MHz. Essentially, the attenuation spectrum describes the coupling between the bubble population and the incident US field; at or close to the resonance frequency, the bubbles are at their most effective in attenuating, hence, at their most effective in transforming the incident US energy to volume oscillations and biomechanical effects. Notably then, the spectrum demonstrate that the bubbles will respond very limited to frequencies outside a range from approx. 0.15 MHz to 0.6 MHz. However, this is the situation when the bubbles are dispersed in a practically infinite matrix, which is not the case when they are lodged in a micro vessel. In this case, the contact with the vessel wall will dampen the bubble response and shift the attenuation spectrum upwards, depending on the diameter and elasticity of the vessel. It is difficult to provide a precise modelling of such dampening effect, however, based on experience, a shift in resonance frequency to approx. 0.5 MHz is a reasonable estimate. Hence, an optimal coupling between the ACT bubbles, with ensuing optimal generation of biomechanical effects, is expected to occur between approx. 0.4 to 0.6 MHz. This then represents the preferred frequency range for the enhancement step under the current invention.

**Modelling of bubble oscillations as a function of frequency and MI.**

Modelling of bubble oscillations for 300, 400, 600 and 900 kHz at MIs of 0.1, 0.2, 0.3 and 0.4 are visualised in Figure 4A. Strong oscillations are evidence for induction of increased biomechanical effects. However, sharp sawtooth responses indicate onset of non-linear and/or inertial cavitation behaviour. As can be noted, at 900 kHz, for all MIs, the radial oscillations are small. In line with the prediction from the attenuation spectrum above, and likely too limited to induce the necessary biomechanical effects necessary to produce a significant therapeutic benefit. On the other hand, for 300 kHz, even for low MIs, the radial oscillations are very strong and non-linear, likely to lead to some level of inertial cavitation and to induce vascular damage. For frequencies between 400 and 600 kHz, however, the radial oscillations seem to meet with the requirement to induce sufficient biomechanical work to induce a therapeutic effect, while at the same time avoiding too much non-linear behaviour and vascular damage.

A particular issue with US mediated delivery of therapeutic agents to the CNS is that the applied US field needs to go through bone (e.g. skull) and that this structure represents a significant and frequency dependent barrier for the incident sound. E.g. the attenuation when passing US across a mouse skull is approx. 20% at 0.5 MHz, but as high as 40% at 2.7 MHz. As the skull represents a significant barrier to US, the use of low frequency fields is optimal for CNS applications, and common to most commercial US devices for CNS insonation. Modelling of bubble oscillations for 0.250 MHz at MIs of 0.025, 0.05 and 0.1 are visualised in Figure 4B. As can be noted, the radial oscillations can be controlled within the desired range, i.e. inducing sufficient oscillations for optimal biomechanical effects but still avoiding excessive inertial cavitation and damage to CNS tissue, even at this low frequency, if combined with a lower MI.

**Tissue uptake of Evans blue and bubble oscillations as a function of MI.**

Results are visualized in Figure 5. As can be noted, the tissue uptake increases from no US (MI=0) to MI=0.1 and further with MI=0.2, but then drops again with MI=0.3 and further with M=0.4. At the same time, from the embedded bubble oscillation panels, maximum radial oscillation increases from approx. 3 $\mu$m at MI=0.1, to approx. 6 $\mu$m at MI=0.2, to approx. 10 $\mu$m at MI=0.3 and more than to 20 $\mu$m at MI=0.4. Importantly, then onset of a reduction in tissue uptake (from MI=0.2 to MI=0.3) is concurrent with the onset of significant non-linear behaviour, where inertial cavitation starts to occur.

**Therapeutic effect of nab-paclitaxel $\pm$ ACT for treatment of prostate cancer - effect of US frequency during the Enhancement step**

The results showing overall survival vs. time are visualized in Figure 6. As can be noted, for the ACT (0.5 MHz group), 100% animals survived until end of study, with 80% of the animals in stable, complete remission (i.e. cancer free). While the ACT (0.9 MHz) group also showed therapeutic benefit vs. drug alone, the effect is significantly inferior to ACT (0.5 MHz). With 0.9 MHz, most tumours started to regrow with only 25% in stable, complete remission and 57% survival at end of study. These results demonstrate that a certain, minimum radial oscillation is needed to induce an optimal therapeutic effect.

As a pilot investigation to this study, in order to evaluate if higher MIs could be applied, an MI of 0.40 was also tested at 0.9 MHz. However, at this MI, clear evidence of superficial haemorrhaging was observed.

**Therapeutic effect of nab-paclitaxel $\pm$ ACT for treatment of breast cancer - effect of MI during the Enhancement step**

The results showing tumour growth rate vs. time are visualized in Figure 7. As can be noted, for the ACT (MI 0.1) group, a marginal but insignificant reduction in tumour growth rate is observed, with tumour growth inhibition vs drug alone at Day 31 of only 8%. For the ACT (MI 0.2) group, however, a strong and significant reduction in tumour growth rate is observed, with tumour growth inhibition vs drug alone at Day 31 of 52%. Again, these results demonstrate that a certain, minimum radial oscillation is needed to induce therapeutic benefit.

**Conclusion:**

Based on the results generated in these four examples, it has been demonstrated that the functionality of the ACT concept is quite sensitive to variance in frequency and MI applied during the Enhancement step. Based on these studies, it is concluded that a preferred frequency range is between 0.4 to 0.6 MHz in combination with an applied MI between 0.1 to 0.3. For CNS applications, frequencies between 0.2 to 0.4 MHz, in combination with MIs between 0.025 and 0.15 also represent a preferred embodiment. With lower frequencies and higher MI applied during the Enhancement step, it has surprisingly been demonstrated that the activated bubble oscillations induced are too strong, leading to a significant loss of efficacy and vascular damage. On the other hand, with higher frequencies and lower MIs, the bubble oscillations induced are too small, leading to a lack of sufficient biomechanical effects and hence a significant loss in therapeutic efficacy.

**Example 3. Delivery of nano-drugs across the Blood-Brain Barrier (BBB).**

The current example investigates the ability of ACT to deliver large, nano-constructs across the BBB, applying US fields according to the current invention.

**Materials and Methods:**

The ACT cluster composition investigated was as detailed in Example 1.

The nanoparticles investigated were core-crosslinked polymeric micelles (CCPMs) from Cristal Therapeutics (Maastricht, The Netherlands). These CCPMs are 70 nm in diameter, labelled with rhodamine B Cy7 for imaging purposes, and the formulation contained 44 mg/ml polymer and 40 nmol/ml Cy7.

The extravasation of CCPM in healthy mouse brains was measured using near infrared fluorescence (NIRF) imaging and the micro-distribution of the CCPM in brain sections was imaged by confocal laser scanning microscopy (CLSM).

Thirteen female albino BL6 mice, purchased at 6-8 weeks of age (Janvier labs, France), were housed in groups of five in individually ventilated cages under conditions free of specific pathogens. Cages were enriched with housing, nesting material and gnaw sticks, and were kept in a controlled environment (20-23°C, humidity of 50-60%) at a 12-hour night/day cycle. Animals had free access to food and sterile water. All experimental procedures were approved by the Norwegian Food Safety Authority.

Illustration of the ultrasound set-up is shown in Figure 8. A custom-built dual frequency transducer (centre frequency 0.5 MHz and 2.7 MHz) [Andersen et al., A Harmonic Dual-Frequency Transducer for Acoustic Cluster Therapy, Ultrasound Med Biol 2019 Sep; 45(9): 2381-2390] was mounted on top of a custom-made cone filled with degassed water. The transducer has a diameter of 42 mm and the -3dB width of the beam profile of the 0.5 and 2.7 MHz were 16 and 6 mm at a distance of 220 mm from the transducer surface. Signals were generated with a signal generator (33500B, Agilent Technologies, USA) and amplified with a 50 dB RF amplifier (2100L, E&I, USA). The amplifier is connected to the switch box which allows for switch from the Activation to the Enhancement US fields. The bottom of the cone was covered with an optically and acoustically transparent plastic foil (Jula Norge AS, Norway), forming a bag. The animal was positioned in prone position on top of an acoustically absorbing material (Aptflex F28, Precision Acoustics, UK), with ultrasound gel for coupling between the acoustic absorber, the animals head and the acoustically transparent foil.

The ACT procedure used comprised an activation and an enhancement step. The attenuation through the murine skull was measured to be approximately $21 \pm 17\%$ and $42 \pm 21\%$ for the 0.5 MHz and 2.7 MHz frequencies, respectively. These numbers where used to calculate the in situ acoustic pressures/MIs. The following ultrasound parameters were used for each step:

- Activation: Centre frequency of 2.7 MHz, average in situ acoustic pressure of corresponding to mechanical index (MI) of 0.18, 8 cycles pulse length, pulse repetition frequency of 1 kHz and insonation time of 60 s.
- Enhancement: Centre frequency of 0.5 MHz, average in situ acoustic pressure corresponding to MI of 0.15, 4 cycles pulse length, pulse repetition frequency of 1 kHz and insonation time of 300 s.

One round of ACT consisted of a bolus intravenous injection of 2 mL/kg of cluster composition prior to the 360 s insonation. Each animal received 3 rounds of ACT, resulting in a total of 75 $\mu$l ACT formulation and 18 min ultrasound. CCPM was injected i.v. immediately prior to the first ACT procedure.

Animals were anaesthetized using 2% isoflurane in medical air (78%) and oxygen (20%) (Baxter, USA) after which their lateral tail vein was cannulated. Hair was removed with a hair trimmer and depilatory cream (Veet, Canada). During the ACT procedure, animals were anaesthetized using 1.5-2% isoflurane in medical air. Respiration rate was monitored using a pressure sensitive probe (SA instruments, USA) and body temperature was maintained with external heating. Each animal received 3 ACT rounds directly after injection of CCPM. Control animals were handled in the same way as the ACT receiving animals but received 3 times a 50 $\mu$l saline injection instead of the cluster composition with 6 minutes interval.

Two timepoints were investigated: 1 and 24 hours after ended ACT treatment. At these timepoints, animals were euthanized by an intraperitoneal injection of pentobarbital (200$\mu$l) and kept under anaesthesia until their breathing halted. Thereafter they were transcardially perfused with 30 ml of PBS after which the brain was excised and imaged with the NIRF imager. Groups were; control/1 hour N=3, control/24 hours N=3, ACT/1 hour N=5 and ACT/24 hours N=2.

Excised brains were placed in a NIRF imager (Pearl Impulse Imager, LI-COR Biosciences Ltd., USA) to assess accumulation of CCPM® in the brain. Brains were excited at 785 nm and fluorescence emission was detected at 820 nm. Images were analysed with ImageJ (ImageJ 1.51j, USA). A Region of Interest (ROI) was drawn around the brain and the total fluorescence intensity of the brain was acquired and normalized to the wet weight of the brain. A standard curve was used to convert the total fluorescence intensity to the percentage of the injected dose per gram of brain tissue (% ID/g). Results were plotted per timepoint and treatment group.

For confocal microscopy, excised brains were mounted transversely on a piece of cork with Optimum Cutting Temperature Tissue Tek (Sakura, The Netherlands) before submerging the sample slowly in liquid nitrogen. Of the frozen brains, the first 500 $\mu$m from the top was removed after which 5 x 10 $\mu$m thick sections and 5 x 25 $\mu$m thick sections were cut transversely. This was repeated every 800 $\mu$m throughout the brain.

**Results:**

To study whether the increased permeability would facilitate extravasation of CCPMs, excised brains were imaged in a NIRF imager. Representative NIRF-images of controls and animals injected with the nanoparticles are shown in Figure 9 (upper panels). As can be noted, clear accumulation could be observed in brains which received ACT opposed to the control brains.

Quantitative analysis of the NIRF-images revealed a statistically significant increase in accumulation (% ID/g) between the ACT and control animals at both timepoints (Figure 9, left lower panel). Vs. control, with ACT the median % ID/g increased from 0.9 % ID/g to 2.6 % ID/g 1 hour and from 0.8 % ID/g to 2.2 % ID/g 24 hours after ACT. Respectively, a 2.9-fold and 2.8-fold increase in % ID/g was observed.

To verify the increased accumulation of the CCPM in brain tissue after ACT treatment, and to study the location of CCPM with respect to blood vessels, brain sections were imaged by CLSM. Tilescans of ACT-treated brains showed several 'clouds' of fluorescence which were not observed in brains of control animals. 24 hours post ACT, tilescans of ACT-treated brains showed similar cloud patterns as the 1 hour treatment group. From thresholded tilescans of both control and ACT-treated animals, the number of pixels representing CCPM were extracted and normalized by the size of the ROI used to outline the hemispheres. As can be noted from Figure 9 (right lower panel), A clear and statistically significant 4.7-fold increase can be observed in the 1h post ACT-treated sections opposed to the control brains.

High magnification CLSM images at different locations in both the control brains and the ACT-treated brains were acquired to study the location of the CCPM with respect to the blood vessels. In ACT-treated brains, CCPM had clearly extravasated whereas in control brains CCPM were mainly observed intravascularly or minimally displaced from the blood vessel staining.

**Conclusion:**

ACT clearly increased the permeability of the BBB for large nanoparticle constructs like the 70nm CCPM compound investigated, when applying the two-step insonation approach of the current invention. ACT resulted in an improved accumulation, extravasation, and penetration of CCPM into the brain parenchyma. The study demonstrates the great potential of ACT for improving the delivery of drugs and nanoparticles temporarily and safely across the BBB. Indirectly, it also demonstrates the ability of ACT to delivery large drug molecules or constructs across any vascular barrier of the body.

**Example 4. Intravital Microscopy**

A prerequisite for the ACT concept is that large, activated bubbles are deposited in the micro vasculature of a region of interest and that they are in relatively close contact with the endothelial wall. In order to further elucidate and demonstrate the characteristics of large bubbles produced after activation in-vivo, a study directly observing individual activated phase shift bubbles within the microcirculation via microscopy of rat mesentery was performed.

**Methods:**

The ACT cluster composition investigated was as detailed in Example 1.

Male Wistar rats were used in the study. General anaesthesia was administered and maintained with i.v. and i.m. pentobarbital sodium. The rats were intubated, and the tail vein or carotid vein was cannulised for administration of the test formulation. Ultrasound was applied to activate the clusters in the mesentery. The abdomen was opened by means of a vertical midline incision, the rats were then placed in the lateral position on a plastic plate incorporating a round window of cover glass, and the exteriorized mesenteries were placed on the cover glass window. The spread mesenteries were perfused with Krebs-Ringer buffer at 37°C. Ultrasound was applied directly onto the exteriorised mesentery under the objective lens of the microscope. An ultrasound scanner (Elegra; Siemens, Seattle, WA) equipped with a linear probe (7.5L40) with a centre frequency of 7.5 MHz was used for ultrasound exposure. Sonar gel was applied between ultrasound transducer and chest wall or the mesentery. Images were recorded on S-VHS or DV tape for subsequent review.

**Results:**

No ultrasound activation was tested in two animals and no large ACT bubbles were observed in the mesentery microcirculation after the 6 injections performed.

Ultrasound activation was applied for three animals. Large, activated bubbles were observed after all injections. The growth phase of the activated phase shift bubbles could be observed in real-time. The nucleus of the activated bubble grew within a few seconds along with micro vessel blood flow obstruction. Over a time period of typically 5-10 minutes, the activated bubbles gradually shrank and intermittently advanced in the micro vessels. All activated phase shift bubbles were larger than red blood cells and lodged in the micro vessels and transiently blocked blood flow. All activated bubbles were non-spherical and appeared ellipsoidal in shape, forming against a section of the micro vessel.

Figure 10 shows video frames of an activated phase shift bubble in the mesentery (indicated by black arrows) at; (top left) pre-injection, (top right) 17 seconds post-injection in a micro vessel, blocking blood flow; (second row, left) at 1 minutes and 9 seconds; (second row, right) at 5 minutes and 9 seconds; (third row, left) at 8 minutes and 19 seconds; (third row, right) at 8 minutes and 45 seconds and (bottom, left) at 8 minutes and 56 seconds, respectively. The activated phase shift bubble (indicated by the arrow) gradually shrinks and advances in the micro vessel by intermittent lodging and dislodging, before it has cleared completely after 8 minutes and 56 seconds. Figure 10 (bottom, right) shows a scale bar with minor units of 10 $\mu$m, to illustrate the size of the bubble.

## Conclusion:

As demonstrated in this example, after activation, large bubbles of typically 20 $\mu$m are formed and transiently retained in the larger micro capillaries. As shown in Figure 10, the activated phase shift bubble gradually shrinks and advances further down in the capillary three by intermittent lodging and dislodging, before it clears completely after typically 5 to 10 minutes. This process entails that even when activating in the heart or feeding vessels to the CNS, the intermittent lodging and dislodging assures that capillaries of all sizes less than the bubble diameter are actually receiving the biomechanical effect and that a single bubble will lead to enhanced permeability at several places in the capillary bed.

## Example 5. Deposition of Activated Bubbles in the CNS after Activation in the Heart

In order to demonstrate the feasibility of depositing large ACT bubbles in the brain after activation outside the CNS, a dog study where activation was performed in the heart was performed. Deposition of activated bubbles was measured by ultrasound imaging of the brain.

## Material and Methods:

The ACT cluster composition investigated was as detailed in Example 1.

A schematic of the ultrasound set up is visualized in Figure 11. In brief, the procedure consisted of the following steps; i.v. injection of 50 $\mu$L cluster composition per kg followed by immediate activation insonation of the heart for 60 seconds, using an ATL HDI-5000 scanner with a P4-2 phased array transducer with a 3 MHz centre frequency and an MI of 1. The scanner was then switched to imaging of the brain using a P5-3 transducer with a 4 MHz centre frequency and an MI of 0.1 to assure the absence of potential local bubble activation. For the same reason, emission of ultrasound was suspended between image acquisition episodes be freezing the scanner display and heart-rate gated imaging with triggering on each fourth cycle was used to minimise overall ultrasound exposure. Single images of the brain tissue were acquired before each injection, and at 2, 4, 6, 8, 10 and 12 minutes after the injection. Brain imaging was towards a larger proximal region of brain tissue not containing strong reflecting elements at baseline. Calculations of echo intensities from images, expressed in dB, was performed by custom written MathLab software.

The procedure was repeated in two mongrel dogs. Anaesthesia was induced with pentobarbital (12 - 25 mg/kg i.v.) and fentanyl (1.5 - 2.5 $\mu$g/kg) and an endotracheal tube was inserted. The animal was transferred to the operating table and put on volume-controlled mechanical room air ventilation (New England mod. 101 Large Animal Ventilator). The animal is kept in general anaesthesia by a continuous i.v. infusion of fentanyl (20 $\mu$g/kg h) controlled by a syringe infusion pump (IVAC model P2000), and pentobarbital (10 mg/kg h) by drip line. The depth of anaesthesia is monitored by physiological recordings (heart rate, blood pressure) and by general observation of the animal (signs of muscular activity, breathing efforts, reflexes).

## Results:

Results for contrast enhancement (dB) in the brain are stated in Table 3 below and visualized in Figure 12. As can be noted, a strong increase in the echo signal was observed at the first imaging time point (2 minutes). The contrast signal displayed a close to exponential decay back to baseline values within 8 minutes, similar to the bubble dynamics observed in Example 4.

Table 3 Echo contrast (dB) in brain after activation of the cluster composition in the heart.

| Animal # | Baseline | 2 min | 4 min | 6 min | 8 min | 10 min | 12 min |
|---|---|---|---|---|---|---|---|
| 1 | 4,3 | 11,4 | 7,9 | 5,7 | 4,3 | 4,2 | 4,2 |

(continued)

| Animal # | Baseline | 2 min | 4 min | 6 min | 8 min | 10 min | 12 min |
|---|---|---|---|---|---|---|---|
| 2 | 6,0 | 13,5 | 10,6 | 8,1 | 6,2 | 5,5 | 5,4 |

<u>**Conclusion:**</u>

The results clearly demonstrate the deposition of large, activated bubbles in the brain after activation in the heart.

**Example 6. Real time intravital imaging of Acoustic Cluster Therapy (ACT®) induced vascular effects in a healthy murine brain**

Real-time observations using intravital microscopy has emerged as a powerful approach to investigate immediate responses associated with microbubble mediated BBB permeabilization (Caskey et al. 2007; Chen et al. 2011; Helfield et al. 2016; Raymond et al. 2007). To deepen our understanding of ACT® induced enhanced BBB permeability, we used multiphoton intravital microscopy to study the real-time vascular response to ACT®. For this purpose, cranial windows aligned with a ring transducer centred around an objective were mounted to the skull of mice. Fluorescein isothiocyanate (FITC) labelled dextrans were injected to delineate the blood vessels and to visualise extravasation. Histological evaluation of brain sections was performed to assess ACT® induced tissue damage. Furthermore, brains were treated with ACT® using a single element dual frequency transducer, and frozen brain sections were stained and imaged by confocal microscopy to assess changes in perfusion and immune response. The study revealed novel knowledge on the mechanism behind ACT® induced enhanced BBB permeability *in vivo* which will be important considering treatment optimization for a safe and efficient clinical translation of ACT®.

Activated ACT® bubbles were observed to alter the blood flow inducing transient and local increase in fluorescence intensity and subsequent extravasation in the form of outpouchings. The observations indicate that ACT® induces widening of the capillaries without causing substantial damage to the vessels in the brain.

**MATERIAL AND METHODS**

The ACT cluster composition investigated was as detailed in Example 1. More specifically, the cluster dispersion was prepared by reconstituting freeze dried suspension of microbubbles of perfluorobutane (PFB) (16 μl/vial) stabilised by a monomolecular phospholipid membrane of hydrogenated egg phosphatidylserine (H-EPS) which is negatively charged, embedded in an amorphous sucrose structure, with a microdroplet emulsion of perfluoromethylcyclopentane (PFMCP) (6.8 mg/mL) stabilized with a monomolecular distearoylphosphatidylcholine (DSPC) phospholipid membrane containing 3% (mol/mol) stearyl amine making the overall surface positively charged, dispersed in a 5 mM tris(hydroxymethyl) aminomethane (TRIS) buffer solution, forming a 2 mL dispersion. The reconstituted ACT® formulation consisted of $6 \times 10^7$ clusters/mL with a median diameter of 5 μm.

Animals received 1-3 ACT® treatments, depending on the individual treatment responses, with 10 to 15 minutes in between treatments. One treatment consisted of a bolus injection of 50 μL ACT® clusters prior to 60 sec activation ultrasound and 300 sec enhancement ultrasound. The ultrasound settings used for the activation and enhancement exposure are given in Table 44.

Table 4. Ultrasound settings for ACT® using the cranial ring transducer

| | ACT® Activation (60 sec) | ACT® Enhancement (300 sec) |
|---|---|---|
| Frequency | 0.84 MHz | 0.5 MHz |
| Peak Negative Pressure (PNP) | 0.25 - 0.3 MPa | ~0.15 MPa |
| Mechanical index (MI) | ~0.3 | ~0.2 |
| Number of cycles | 8 | 8 |
| Pulse Repetition Frequency (PRF) | 1 kHz | 1 kHz |

**Fluorescent tracer**

To visualize the brain vasculature, 50 μL of 2 MDa FITC-dextran (100 mg/mL in saline; Sigma-Aldrich, Germany) were administered intravenously prior to the first ACT® treatment. A 30 μL reboost was given before every following ACT®

treatment.

## Ring transducer and ultrasound profile

A custom-built, single-element, ring-shaped piezoelectric ultrasound transducer (10 mm outer diameter, 1.4 mm thickness, 1.2 mm height) glued to a glass coverslip (ø 13 mm, #1 thickness, Thermo Fisher Scientific, USA) for direct attachment to the skull (Yddal et al., 2016) was used for ACT® treatment. A transducer assembly jig was 3D-printed to facilitate the alignment of the glass coverslip and the ring transducer. The transducer was driven with a signal generator (33500B, Agilent Technologies, USA) and the signal was amplified with a 50 dB power amplifier (2100L, E&I, USA).

The ultrasound profile was characterized by attaching the individual ring transducers (n = 3) to extracted mouse skulls and by placing them in a water tank. A hydrophone (Onda, HGL-0200) connected to a pre-amplifier (AG-2010) and mounted to a motorized 3D stage (Onda, AIMS III) was positioned at approximately 1-2 mm distance from the centre of the transducer. The acoustic signals measured by the hydrophone were sent to an oscilloscope (PicoScope, 5244A) and post-processed on a PC (Soniq Software) (Table 4). The focal size of the activation ultrasound and enhancement ultrasound was extracted from the ultrasound beam profile. At approximately 1-2 mm distance, the focal diameter of the activation ultrasound (0.84 MHz) and enhancement ultrasound (0.5 MHz) were both ø 1-2 mm.

## Multiphoton microscope

A multiphoton microscope (*in vivo* SliceScope, Scientifica, UK) with a 20× water dipping objective (XLUMPLFLN 20XW, Olympus; numerical aperture (NA) = 1; working distance 2 mm) was used for real-time imaging. The pulsed MaiTai DeepSee (Spectra-Physics Mountain View CA, USA) laser was used with an excitation wavelength of 790 nm. The laser power was set to 11 %, corresponding to a power of 20 mW as determined by LabMax-TOP Laser Power/Energy Meter (Coherent, Inc.). A 525/50 bandpass filter in front of the GaAsp detector was used to detect FITC dextran. Images (512×512 pixels) were acquired in resonant scanning mode at 31 frames per second (fps) with a field of view of 400×400×1 $\mu$m. During ACT® treatment, an XYT-scan was acquired to assess the induced effect of ACT® in real time. Before and after ACT® treatment, an XYZ-stack (Imaging depth = 100-400 $\mu$m, $\Delta z$ = 1 $\mu$m, 12 averages) was acquired to assess the induced effects in 3D.

## Animals

Female albino BL6 mice (6-8 weeks of age, ~20 g, n = 20) obtained from Janvier Labs, France, were housed in groups of five under conditions free of specific pathogens in a controlled environment (20-23 °C, humidity of 50-60 %) at a 12-hour night/day cycle. Cages were individually ventilated and enriched with housing, nesting material and gnaw sticks in addition to free access to food and sterile water. All animal procedures were approved and in accordance with the guidelines established with the Norwegian Food Safety Authority.

## Experimental procedure - cranial window and ring transducer placement

The experimental procedure including video is described in detail in Poon et al. 2021 (submitted). Briefly, prior to experiments, animals were anaesthetized in an induction chamber using 2-3 % isoflurane (Baxter; USA) in $N_2O$ (0.4 L/min) and $O_2$ (0.2 L/min). The anesthesia carrier gas was used to ensure survival of the animal. Thereafter they were positioned in a stereotaxic frame (Kopf Instruments, USA) and the tail vein was cannulated for easy access to the systemic circulation. During the procedure the animal's physiology was continuously monitored and a body temperature of 37 °C was maintained using external heating. Before the craniotomy, the fur was removed from the top of the head using a trimmer and depilatory cream (Veet, Canada), and systemic (Buprenorphine (0.05-0.1 mg/kg s.c.), Meloxicam (2-3 mg/kg s.c.) and local (Bupivacaine (1 mg/kg s.c.) analgesia was administered. Using a stereo microscope (10-25×, Nikon, Japan), the scalp was removed, and a hand drill was used to drill a 3-4 mm circular hole into the parietal bone, which was covered with a glass coverslip (ø 5 mm, #1 thickness, Thermo Fisher Scientific, USA) and attached to the skull with superglue (Loctite #1363589, Henkel, Germany). Several droplets of 1 % (w/v) agarose were applied on top of the cranial window, and the ring transducer attached to a coverslip was centrally placed on top and glued to the surrounding skull. Thereafter, the stereotaxic frame with the animal was transferred to the microscope stage and aligned with the objective lens of the microscope.

## ACT® treatment and multiphoton imaging

The timeline of the experimental procedure was the following: Animals were injected with 50 $\mu$L of 2 MDa FITC dextran (t=-10 min), and a field of view was selected for an XYZ (pre Z-stack, t=0, -1, -3 min) scan to assess the vasculature prior to ultrasound exposure. Afterwards, an XY plane was selected for XYT-imaging during ACT® treatment. A volume of 50 μL of ACT® clusters was intravenously injected (t=0) and sonication was started. Imaging continued throughout the duration of sonication and up to 2 min after treatment. Then, a post XYZ (post-Z-stack) scan was acquired (t=6, 8, 10 min.). Control animals were handled in the same way, either by injecting 50 μL of ACT® without applying ultrasound or by initiating ultrasound without injecting ACT® clusters. The animals were euthanized by cervical dislocation, and the brains were extracted and prepared for histology.

### Image analysis

The acquired images and videos were analyzed using Fiji (ImageJ 1.51i, USA) and MATLAB (Mathworks, USA). The signal to noise ratio of the acquired videos was enhanced by performing frame averaging (n = 2-4) with Fiji.

### Volume determination of red blood cells and ACT®-bubbles

All videos acquired were visually assessed for the presence of ACT® bubbles. ACT® bubbles were visualised as non-fluorescence voids (black) with predominantly ellipsoid shape surrounded by FITC-dextran fluorescence. Once observed, the size of the determined ACT® bubble was measured over several consecutive frames (n = 2-7) with the measuring tool in Fiji. The size of the red blood cells (RBCs) was calculated based on profile plots acquired with Fiji and further analysis by a custom-made MATLAB script. Volumes of both the ACT® bubbles and RBCs were calculated with the following formula: $(\pi/4)\times(width)2\times length$.

### Size determination of outpouching and corresponding extravasation area and time

Both the length and width of the outpouching that appeared on the vessel wall were determined with the measuring tool 'freehand straight line' in FIJI. For the measurement, the last frame of the video prior to extravasation was used. The size of the corresponding extravasation area was determined with the freehand selection tool in the last frame in which extravasation was still visually observed. The extravasation time was defined as the time between the onset and stop of extravasation.

### Determination of the diameter of blood vessels

In Fiji, a line (approx. 100 μm long) was drawn perpendicularly to the blood vessel of interest. For each frame of the video, the intensity profile plot of this line was obtained. These profile plots were processed in MATLAB using a custom-made script to determine the FWHM of each intensity profile plot.

### Measuring fluorescence intensity in blood vessels

To assess if the presence of ACT® bubbles altered the intravascular FITC-dextran fluorescence intensity, three circular ROIs were placed inside the blood vessels while ACT® bubbles passed through, and the fluorescence intensity was determined in Fiji. One of the ROIs was placed at the location of the transiently stuck ACT® bubble while the other two ROIs were placed just behind and in front of the ROI. Of all three ROIs the average intensity per video frame was determined and plotted.

### Acquired videos from real time imaging

A total of 20 animals have been used to conduct real time intravital microscopy experiments. From successful experiments (n = 6-7 animals) approximately 200 videos were acquired during ACT® treatment and 50-60 videos represent Z-stacks prior and post ACT® treatment. The results presented in this study are based on 30-40 videos, since not all ACT® treatments resulted in measurable effects in the field of view.

### Histology

For histological evaluation, excised brains from real time imaging experiments were pre-cut to include the hemisphere that contained the cranial window and the contralateral hemisphere. The brain tissue was fixed in 4 % formalin and embedded in paraffin. The brains were serially sectioned every 100 μm transversely from the surface until 2 mm into the brain. The 4 μm thick sections were stained with hematoxylin, erythrosine and saffron (HES). Tilescans of the HES sections were acquired using bright field imaging with an LSM800 (Zeiss) and a 20×/0.8 Plan-Apochromat air objective. A senior neuropathologist, blinded to the study, assessed the brain sections for tissue damage induced by cranial window placement or ACT® treatment.

**Immunohistochemistry**

For immunohistochemical analyses, frozen brain sections were stained with the following primary antibodies: Anti-CD31, as endothelial marker to assess vascular changes, anti-Claudin 5, for tight junction complexes, anti-F4/80, as a marker for macrophages, anti-TMEM119 as a microglia specific marker, and anti-Ly6G for neutrophils to assess potential immune responses.

Brain sections were obtained from a separate study (Olsman et al., 2021) where the entire brain was treated with a dual frequency transducer (Andersen et al., 2019) and excised 1 h or 24 h after ACT® treatment. The reason for analyzing these brain sections in addition was the larger ACT® exposed area (-3 dB beamwidth of 6 mm (0.5 MHz) and 16 mm (2.7 MHz)) as well as the two timepoints. Furthermore, the effect craniotomy had on the brain could be excluded by analyzing these sections. Before euthanization of the animals, FITC-labelled Lycopersicon esculentum (tomato) lectin (FITC-lectin) (Vector Laboratories, USA) was intravenously injected to stain for functional vessels at that time point.

Briefly, 10 $\mu$m or 25 $\mu$m thick sections from 1-2 brain depth levels were thawed at room temperature (RT) and rehydrated in phosphate buffered saline (PBS, pH 7.4). The sections were fixed in acetone/methanol (V/V) at -20 °C for 90 sec. Non-specific staining was blocked for 1 h at RT using 0.2 % bovine serum albumin (BSA) in PBS supplemented with 0.2 % Triton X-100 for permeabilization. Sections were then incubated for 1 h at RT using the primary antibodies (anti-CD31; anti-Claudin 5; anti-F4/80; anti-TMEM119; anti-Ly6G) in blocking buffer containing 1 % BSA in PBS supplemented with 0.3 % Triton X-100. Thereafter, the sections were washed 3×5 min in PBS.

The following secondary antibodies were used: Alexa Fluor 647-conjugates (AF-647) as well as Cy3 conjugates. The secondary antibodies were diluted in a blocking buffer containing 1 % BSA and 0.3 % Triton X-100 and sections were incubated with the antibodies for 1 h at RT in the dark. Afterwards the sections were washed 3×5 min with PBS and mounted with Vectashield Vibrance (Vector Laboratories, USA), with or without 4',6-Diamidin-2-phenylindol (DAPI) nuclear stain depending on the applied staining and covered with a cover glass.

**Confocal laser scanning microscopy of brain sections and image analysis**

*Tile scans*

Immunohistochemically labelled brain sections were imaged with an LSM 800 from Zeiss. Tile scans of anti-CD31 stained brains (also containing FITC-lectin and rhodamine B labelled polymeric micelles) were acquired using a 20×/0.8 plan Apochromat air objective (16-bit, 1024×1024 pixels, 312 nm pixel size, 2 averages by line, 10 % overlap between consecutive tiles, optical slice thickness of 10 $\mu$m). Tile scans of anti-F4/80 and DAPI stained sections were acquired using a 20×/0.50 Plan-Neofluar air objective (8-bit, 1024×1024, 312 nm pixel size, 2 averages per line, 10 % overlap, optical slice thickness 6 $\mu$m). To minimize fluorescent spill-over, the different channels of the image were captured in a sequential manner. The fluorophores were FITC-lectin, AF-647, Rhodamine B, DAPI. The fraction of perfused vessels was calculated from tile scans by dividing the area fraction of FITC+ blood vessels by the area fraction of CD31+ vessels.

**High magnification imaging**

Regions of interest within sections were imaged using 20×/0.8 plan Apochromat air objective or a 40×/1.2 C-Apochromat water objective (For both objectives: 16-bit, 1024×1024 pixels, 0.156-0.312 $\mu$m/pixel, line average of 4, optical slice thickness 1.9 $\mu$m). Each fluorophore was captured in a different channel and imaging was done sequentially.

**Temperature measurements**

To evaluate if multiphoton imaging caused a temperature increase and potentially activate ACT® clusters, 50 $\mu$L of the ACT® cluster dispersion was deposited on a microscope glass slide (SS XX). The temperature rise was measured using the inbuilt temperature measuring capabilities of a fiber optic needle hydrophone (Precision Acoustics) whilst imaging with a near-infrared laser (Chamelein Vision-S IR pulsed laser, Olympus, Japan) at a wavelength of 790 nm. The laser power was measured at different power levels using a LabMax-TOP Laser Power/Energy Meter (Coherent, Inc.). XYT scans were collected with a Leica TCS SP (10×/0.40 HC PL Apo CS air objective). To assess if the temperature rise induced by the multiphoton laser could affect ACT® bubble activation (bubbles with diameters above $\geq$ 20 $\mu$m were regarded as 'activated'), 15 $\mu$L of the ACT® dispersion was deposited on a microscope glass slide. A white light laser was used for bright-field transmitted light imaging. The effect of the 790 nm excitation laser was evaluated at three different power levels (20 mW, 40 mW, and 60 mW). Repeated XY scanning at fixed power was done at 2.4 $\mu$s/pixel speed for a total of 10 min, capturing transmitted light images (512$\times$512 pixels, 8-bit, 2.27 $\mu$m pixel size) every 2 min.

### Statistics

Statistical analyses of the acquired datasets were performed using GraphPad Prism (v8.0, USA). The type of statistical test used is indicated in the corresponding figure caption. A p-value less than 0.05 indicates statistical significance.

## RESULTS

### Activated ACT® bubbles in brain capillaries

During ACT® treatment, anticipated ACT® bubbles appeared as dark spheres, or ellipsoids, marked with arrows, in the fluorescent vasculature. **Feil! Fant ikke referansekilden.**14 shows snapshots of acquired videos with anticipated ACT® bubbles, with four (A-D) examples of ACT® bubble observations. Snapshots of acquired videos (i) without and (ii) with a suspected ACT® bubble. Scale bar represents 50 $\mu$m. From 64 observations the average volume of the anticipated ACT® bubbles was 965 $\pm$ 1485 $\mu$m$^3$ with the smallest and largest observed ACT® bubble being 185 $\mu$m$^3$ and 9622 $\mu$m$^3$, respectively. The red blood cells (n = 5949) had an average size of 144 $\pm$ 57 $\mu$m$^3$ and differed significantly from the average volume of the anticipated ACT® bubbles (p<0.0001).

### Transient vaso-modulation in response to sonication

During ACT® treatment, variation in the diameter of blood vessels in the field of view was observed. In two consecutive treatments of one animal two vessels responded to the ultrasound (Figure 15, snapshot from 2 videos, two treatments). During the first treatment (Figure 15A), the vessel in the top right ("b" line) started to deform from approximately the moment the ultrasound was turned on. The diameter of the vessel was reduced by 85 % (60 $\mu$m to 10 $\mu$m) before, the diameter increased to 40 $\mu$m again. Another vessel ("c" line) had an initial diameter of around 13 $\mu$m and changed the diameter by approximately 5 $\mu$m (40 % reduction) in a pulsed manner several times. The diameter of two other blood vessels ("a" and "d" line) in the field of view were measured as well but did not change diameter during ultrasound exposure. During the second treatment (Figure 15B), the four blood vessels showed similar behaviour as during the first treatment. In panel A, the "a" line in the graph (Figure 15Aiii) is noisier compared to the corresponding "a" line in panel B due to a difference in contrast between the videos. In addition, some motion artefacts were observed at the start of the video corresponding to panel A. The diameter of the four measured blood vessels during the first and second treatment can be found in Figure 15, panels iii. The observations cannot be directly attributed to ACT® since no lodged ACT® bubbles were observed in the affected vessels in the field of view.

### Fluorescence intensity inside blood vessel

To study if ACT® bubbles cause accumulation of FITC-dextrans while they are going through or temporarily lodged in or obstructed the blood vessel, the fluorescence intensity was measured at different locations in the blood vessel in which an ACT® bubble was observed. When an ACT® bubble appeared in the vasculature and was temporarily lodged in a blood vessel, a drop in fluorescence intensity at the specific location was observed. Blockage resulted in a temporary increase/decrease in the fluorescence intensity in the ROIs located upstream/downstream of the lodged ACT® bubble. In one example the ACT® bubble seemed to be lodged for a longer time since the change in the fluorescence intensity was prolonged. In yet another example, the situation was different since several ACT® bubbles flew through the same blood vessel and got lodged, which resulted in fluctuations in the fluorescence intensity, marked by decreasing and increasing intensities.

### Extravasation during ACT® treatment

During ultrasound exposure, the growth of eight outpouchings was observed, as indicated by the circles in Figure 16. The images and videos obtained showed how the outpouchings, appearing as small saccular outpouchings, developed during ACT®. Eventually outpouchings expanded and extravasation of FITC-dextrans occurred. No clear extravasation was observed at other locations. The outpouching shown in Figure 16H originated upon expansion of the outpouching shown in Figure 16C. The same applies to the outpouching shown in Figure 16F which originated from the outpouching shown in Figure 16E. The maximum size of the outpouchings was determined and is presented in Table 5 below. The size of the outpouchings prior to expansion varied largely, with the smallest one being 43 $\mu$m$^2$ while the largest

one was 100 times larger, 5950 $\mu$m$^2$. The maximum penetration depth observed varied between 10 and 200 $\mu$m. Seven of the eight outpouchings expanded while imaging. The corresponding extravasation area and time were determined (see Table 5). A relation between the size of the aneurysm prior to rupturing and the area of extravasation was found. The extravasation time was independent of the area of extravasation.

Table 1. Information about the diameter of the blood vessel from which the outpouching originates, size of the outpouching, extravasation area, maximum penetration depth of extravasation and extravasation time of the outpouchings shown in **Feil! Fant ikke referansekilden.**16.

| Outpouching | A | B | C | D | E | F | G | H* |
|---|---|---|---|---|---|---|---|---|
| Diameter blood vessel [$\mu$m] | 4.4 | 5.5 | 5.7 | 7.2 | 5.5 | 5.5 | 5.7 | 5.7 |
| Length [$\mu$m] | 12 | 10 | 19 | 20 | 44 | 105 | 26 | 24 |
| Width [$\mu$m] | 7 | 7 | 17 | 18 | 34 | 79 | 22 | 19 |
| Area [$\mu$m$^2$] | 58 | 43 | 246 | 286 | 1070 | 5950 | 446 | 376 |
| Area of extravasation [$\mu$m$^2$] | 71.471 | 170 | 950 | 87.527** | 5950 | 9945 | 6107 | - |
| Maximum penetration depth [$\mu$m] | 200 | 10 | 25 | 183 | 30 | 61 | 61 | - |
| Extravasation time [s] | ~36 | ~22 | ~4 | ~50 | ~44 | ~10 | ~35 | - |

\* Outpouching did not rupture
\*\* Area of extravasation hard to determine due to overlap with area of extravasation of outpouching A

**Vascular observations post ACT® treatment**

Most information about the underlying mechanism of ACT® was obtained by real-time imaging of the murine brain during ACT® treatment. Prior and after ACT® treatment a Z-stack was obtained to compare and assess for ACT®-induced effects at different depths not recorded during real-time imaging. Prior to ACT® treatment the vasculature in the pre Z-stack appeared normal (Figure 17-i), whereas a large elliptical structure with a length and diameter of approximately 75 and 50 $\mu$m was found in the post Z-stack (Figure 17-ii). After approximately 6 minutes the structure had disappeared leaving a heterogenous fluorescence intensity within the blood vessel (Figure 17-iii). In addition, the vasculature outside the field of view was assessed for ACT® induced effects post treatment. Two post Z-stacks resemble the fluorescence intensity pattern observed upon the formation of the outpouchings during real time imaging. However, due to the missing pre Z-stack the structures cannot be directly attributed to ACT®.

**After ACT® - Blood vessels still functional post leakage of the outpouchings**

In addition to the real time imaging during ACT®, a pre and a post Z-stack was recorded. The maximum projection image of the Z-stacks is shown in Figures 18A and B, wherein projection image A is taken before treatment and B is after treatment. Scale bar represents 50 $\mu$m. The lines mark blood vessels of which the diameter is measured pre and post ACT® treatment. The difference in diameter pre and post ACT® treatment are shown in Table 6 below. Arrows mark the left-overs of the aneurysms. The asterisks mark blood vessels which were observed in the pre XYZ-stack but not in the post XYZ-stack. The latter is either due to slight misalignment between the pre and post XYZ-stack, movement of the blood vessels with respect to each other, movement of the animal or the vessels not being functional during the time recording. The image hence show the blood vessels in which the expansion of the outpouching occurred still contained flowing FITC-dextran, indicating they are still functional. Furthermore, it can be observed that several blood vessels are slightly dilated (1.2-2.1 $\mu$m) with respect to their initial size. There are small misalignments both in XY-direction and in Z-direction, and some blood vessels clearly observable in the pre Z-stack are not as clear in the post Z-stack. This is most likely due the movement of blood vessels with respect to each other, movement of the animal during ACT®, or the focus was changed when ultrasound was turned on. In addition, the high local concentration of extravasated FITC-dextran will reduce the light penetration depth. Furthermore, some vessels might have a rapid or prolonged impact from ACT®.

Table 6: The difference in diameter pre and post ACT treatment

| Diameter blood vessel [$\mu$m] | | | |
|---|---|---|---|
| | Pre-treatment (A) | Post-treatment (B) | Δ |
| i | 7.2 | 9.5 | 1.3 |

(continued)

| Diameter blood vessel [μm] | | | |
|---|---|---|---|
| | Pre-treatment (A) | Post-treatment (B) | Δ |
| ii | 5.4 | 6.9 | 1.5 |
| iii | 5.3 | 7.4 | 2.1 |
| iv | 6.0 | 7.7 | 1.7 |
| V | 4.3 | 5.5 | 1.2 |

**Brain perfusion after ACT® treatment**

To further assess vessel functionality, blood vessels were immunofluorescently labelled with the endothelial marker CD31 and compared to the FITC-lectin labelled functional vasculature in control and ACT® treated animals. No significant difference in the lectin staining relative to the CD31 staining was detected for the 1 h and 24 h time point for the treated compared to the non-treated brains indicating that there was no systemic change in perfusion in the brain.

Anti-Claudin 5 staining of brain sections showed a linear staining pattern along the entire endothelial cell lining in regions without extravasated polymeric micelles. Vessels seldomly showed a discontinuous staining pattern with only speckles along the vessel structure in ACT® treated brains, predominantly observed in the vicinity of areas with extravasated polymeric micelles but the pattern was also seen in control brains. Visual assessment showed no difference in the distribution of Claudin 5 in control brains compared to ACT® treated brains over the entire brain.

High resolution images of anti-CD31 and anti-claudin 5 stained vessels of local areas where polymeric micelles extravasated showed in some cases a loss of lectin staining in ACT® treated animals, indicating non-functional vessels during the time of lectin injection. Assuming that polymeric micelles extravasated from vessels affected by ACT® bubble activity, simultaneous detection of claudin-5 and lectin staining also allowed to identify non perfused vessels.

**Histological evaluation following ACT® treatment**

Microscopic examination of 4 μm HES stained sections from 5 different depth levels of extracted brains showed no signs of vascular damage in the non and cranial window containing hemisphere. A small amount of vacuoles was detected in the area of the cranial window in the case of one animal. HES sections of ACT® treated animals showed no acute lesions, no damage or destructed blood vessels compared to control animals. Furthermore, there was no increase in polymorphonuclear leukocytes or other leukocytes and no detectable assembly of fluid in the sonicated regions.

**Immune cell presence in the brain following ACT® treatment**

To evaluate the presence of immune cells after ACT® treatment, brain sections were stained for F4/80+ (macrophages, microglia) and Ly6G+ (polymorphonuclear neutrophils) cells and imaged by CLSM. In tilescans of ACT® treated and control sections, F4/80+ cells were detected in the perivascular space, parenchymal tissue and in the cerebral ventricles. The majority of Ly6G+ cells were observed as clusters intravascular or in perivascular spaces, while a few were seen as single cells or fragmented cell structures on the capillary wall. Semi-quantification of sections from two depth levels (2.1 mm and 2.9 mm from the brain surface) showed a slight increase in the amount of pixels representing F4/80+ cells in % brain area in ACT® treated animals 1 h (20,5 %; p = ns) and 24 h (33.3 %, p = ns) after treatment compared to control animals. The number of Ly6G+ cells was too low for an adequate quantification.

High magnification images showed that some F4/80+ cells were situated in different compartments in the brain such as perivascular spaces of the arteriolar and venular vasculature as well as arteries and veins. These cells represent either vessel associated microglia or perivascular macrophages. F4/80+ cells present in the brain parenchymal tissue were morphologically distinct from the perivascular cells. Those cells showed a ramified morphology and were positively co-stained with the microglial marker (TMEM119), classifying them as brain resident parenchymal microglia. Some double labelled cells had an amoeboid morphology indicating that they could be in an activated state. Immunofluorescence verified histological findings of no serious infiltration of immune cells after ACT® treatment.

Summary of findings from study of Ex. 6:

- Large ACT® bubbles induced a transient and local increase in FITC dextran-fluorescence intensity inside the blood vessels.
- Extravasation occurred from ACT® induced outpouchings in the vessel wall
- Outpouchings varied in size and showed a heterogenous distribution pattern of FITC dextrans in the brain parenchyma (comparable to cloud patterns as observed in the study of Example 5)

- Immunohistochemical analysis assessing blood vessel functionality after ACT® showed no change in overall brain perfusion
- Real-time observations as well as histology showed no haemorrhage. The vessels observed remained functional. No acute inflammatory response was detected.
- ACT® can extravasate drug to a larger area, have observed an area of 9945 $\mu m^2$
- ACT® does to our knowledge not open large pores or junction that allow bleeding, pore size is less than 6 $\mu$m. No sign for haemorrhages
- Open skull example
- Ring transducer applying two frequencies

  ○ Activation: 2.7 MHz, PNP 0.21 MPa, 60 sec
  ○ Enhancement: 0.5 MHz, PNP 0.1 MPa, 300 sec

The in vivo response of ACT® treatment was evaluated using real time imaging of the vasculature in the brain through a cranial window. In addition, vascular changes, possible tissue damage and immune responses to ACT® were histologically evaluated ex vivo. ACT® led to an increase in the fluorescence intensity of coinjected FITC-dextrans inside blood vessels, expansion in vessel diameter and subsequent extravasation, without causing severe damage to the vasculature or a distinct inflammatory response. The extravasation occurred from ACT®-induced outpouchings.

## Claims

1. A pharmaceutical composition comprising a microbubble-microdroplet cluster composition and at least one therapeutic agent for use in a method of treatment of diseases, disorders or injuries of the central nervous system (CNS) of a subject, wherein the use comprises Acoustic Cluster Therapy (ACT) treatment wherein at least one step of ultrasound insonation is performed either non-invasively towards the CNS, invasively towards the CNS or towards the heart or a spinal or carotid artery outside the CNS, comprising the steps of:

   (i) administering the pharmaceutical composition to the subject; wherein the at least one therapeutic agent is pre-, and/or co- and/or post administered separate to the cluster composition;
   (ii) optionally imaging the clusters of the pharmaceutical composition using ultrasound imaging to identify a region of interest in the CNS for treatment within said subject;
   (iii) activating a phase shift of a diffusible component of the microdroplet of the cluster composition from step (i) by ultrasound insonation at a first frequency and a first mechanical index; wherein

      a) for the non-invasive insonation, this takes place from at least one transducer positioned outside the brain skull or vertebral column of the subject for insonation through this; and
      b) for the invasive insonation, this takes place from at least one surgically implanted transducer in the subject's brain skull or vertebral column; and
      c) for the insonation towards the heart or a spinal or carotid artery, this takes place in the heart or in a spinal or carotid artery outside the CNS;

   (iv) further insonating with ultrasound at a second frequency which is lower than the first frequency, and a second mechanical index, facilitating extravasation of the at least one therapeutic agent administered in step (i) at the region of interest in the CNS; wherein

      a) for the non-invasive insonation, this takes place from at least one transducer positioned outside the brain skull or vertebral column of the subject for insonation through this; and
      b) for the invasive insonation, this takes place from at least one surgically implanted transducer in the subject's brain skull or vertebral column;
      c) when activating according to c) in step (iii), further insonation in this step takes place either non-invasively or invasively according to step (iv) a) or step (iv) b).

2. The pharmaceutical composition of claim 1 for use according to claim 1, wherein:

   step (iii) is performed non-invasively towards the CNS using the first frequency in a range of 0.2-3 MHz with the first MI of 0.1-0.4; and/or

step (iv) is performed non-invasively towards the CNS using the second frequency in a range of 0.2-0.4 MHz with the second MI in a range of 0.025-0.15, or using the second frequency in a range of 0.4-0.6 MHz with a second MI in a range of 0.1-0.3.

3. The pharmaceutical composition of claim 1, for use according to claim 2, wherein the ultrasound insonation is performed non-invasively either by an extracranial, hemispheric focused US array, an extracranial, focused mono-element US transducer or from a nasal or ocular transducer.

4. The pharmaceutical composition of claim 1 for use according to claim 1, wherein:

the ultrasound insonation is performed invasively towards the CNS in the step (iii) using the first frequency in a range of 1-10 MHz with the first MI in a range of 0.1-0.4; and/or
step (iv) is performed invasively towards the CNS using the second frequency in a range of 0.2-0.4 MHz with the second MI in a range of 0.025 to 0.15, or using the second frequency in a range of 0.4-0.6 MHz with the second MI in a range of 0.1 to 0.3.

5. The pharmaceutical composition of claim 1, for use according to claim 4 wherein the ultrasound insonation is performed through the skull or vertebral column by one or more surgically implanted US transducers.

6. The pharmaceutical composition of claim 1, for use according to claim 1, wherein the ultrasound insonation in step (iii) is performed towards the heart or spinal or carotid arteries outside the CNS with the first frequency in a range of 1-10 MHz with the first MI in a range of 0.1-0.4, and wherein the ultrasound insonation of step iv) is performed either non-invasively or invasively towards the CNS using the second frequency in a range 0.2-0.4 MHz with the second MI in a range of 0.025 to 0.15, or using the second frequency in a range of 0.4-0.6 MHz with the second MI in a range of 0.1 to 0.3.

7. The pharmaceutical composition of claim 1, for use according to any of the claims 1 to 6, wherein:

the insonation of step (iii) starts immediately after step (i) and is immediately followed by the insonation of step (iv); and optionally
the insonation of step (iii) lasts for 30-120 seconds, followed by the insonation of step (iv) which lasts for 3-10 minutes.

8. The pharmaceutical composition of claim 1, for use according to any of the claims 1 to 7, wherein 1 to 5 therapeutic agents are administered simultaneously or sequentially over a certain time span wherein at least one, such as 1 to 5, ACT treatments are performed during the same period.

9. The pharmaceutical composition of claim 1, for use according to any of the claims 1 to 8, wherein the same broad band or dual frequency US transducer is used in both the activation insonation of step (iii) and the enhancement insonation of step (iv).

10. The pharmaceutical composition of claim 1, for use according to any of the claims 1 to 9, wherein:

the clusters have a mean diameter in the range 3-10 $\mu$m, and preferably in the range 4-9 $\mu$m; and optionally
the cluster concentration of clusters in the size range 1-10 $\mu$m is at least 25 million/ml.

11. The pharmaceutical composition of claim 1 or 10, for use according to any one of claims 1 to 9, wherein:

a gas of the microbubbles of the microbubble-microdroplet clusters comprises sulphur hexafluoride or a C3-6 perfluorocarbon or mixtures thereof; and optionally
the gas of the microbubble is selected from the group of sulphur hexafluoride, perfluoropropane, perfluorobutane, perfluoropentane and perflurohexane or a mix thereof, the microbubble is stabilized by a first stabilizer selected from the group of phospholipids, proteins and polymers; and
the oil phase of the microdroplets comprises a diffusible component selected from the group of perfluorocarbons, e.g. a perfluorocycloalkane, stabilized with a second stabilizer selected from the group of surfactants, e.g. including phospholipids, polymers and proteins.

12. The pharmaceutical composition of claim 1, 10 or 11, for use according to any one of claims 1 to 14, wherein an oil phase of the microdroplet of the microbubble-microdroplet clusters comprises a partly or fully halogenated hydrocarbon or a mixture thereof.

13. The pharmaceutical composition of any of claims 1 and 10-12, for use according to any one of the claims 1 to 14, wherein the therapeutic agent is selected from the group of the drug classes chemotherapeutic agents, immunotherapeutic agents, immune oncology agents, immunomodulatory drugs, anti-B cell drugs, antiinflammatory drugs, antimicrobial drugs, anti-angiogenic drugs, antidepressants, anticonvulsants, cannabinoid drugs, tumour necrosis factor-$\alpha$ (TNF) inhibitors, dopamine precursors, catechol-o-methyl transferase inhibitors, dopamine agonists, monoamine oxidase B inhibitors, mantadine, anticholinergics, anticoagulants, antiplatelet drugs, tissue plasminogen activator (tPA) and cholinesterase inhibitors.

14. The pharmaceutical composition of any of the claims 1 and 10-13, for use according to any one of the claims 1 to 9, wherein the therapeutic agent is formulated in a vehicle, such as included in the form of liposomes, micelles, conjugates, nanoparticles, core-crosslinked polymeric micelles (CCPMs) or microspheres, or selected from the group of genes, antimicrobial peptides, stem cells and aptamers; and optionally
wherein the therapeutic agent, or a formulated form of the therapeutic agent, has a molecular weight of more than 500 Daltons.

15. The pharmaceutical composition of any of the claims 1 and 10-14, for use according to any one of the claims 1 to 9, wherein the cluster composition is administered in a time window of 3 hours from combining a first component of microbubbles with a second component of microdroplets preparing the microbubble-microdroplet cluster composition.

**Patentansprüche**

1. Pharmazeutische Zusammensetzung, umfassend eine Mikrobläschen-Mikrotröpfchen-Cluster-Zusammensetzung und mindestens ein therapeutisches Mittel zur Verwendung in einem Behandlungsverfahren für Erkrankungen, Störungen oder Verletzungen des Zentralnervensystems (ZNS) eines Subjekts, wobei die Verwendung eine Behandlung mit Akustik-Cluster-Therapie (ACT) umfasst, wobei mindestens ein Schritt einer Ultraschallbeschallung entweder nicht-invasiv in Richtung des ZNS, invasiv in Richtung des ZNS oder in Richtung des Herzens oder einer Spinal- oder Karotisarterie außerhalb des ZNS durchgeführt wird, umfassend die Schritte:

(i) Verabreichen der pharmazeutischen Zusammensetzung an das Subjekt; wobei das mindestens eine therapeutische Mittel vorher, und/oder zusammen und/oder nachher getrennt von der Cluster-Zusammensetzung verabreicht wird;
(ii) optional Abbilden der Cluster der pharmazeutischen Zusammensetzung unter Verwendung von Ultraschallbildgebung, um einen Bereich von Interesse in dem ZNS für die Behandlung innerhalb des Subjekts zu identifizieren;
(iii) Aktivieren einer Phasenverschiebung einer diffundierbaren Komponente des Mikrotröpfchens der Cluster-Zusammensetzung von Schritt (i) durch Ultraschallbeschallung bei einer ersten Frequenz und einem ersten mechanischen Index; wobei

a) für die nicht-invasive Beschallung diese von mindestens einem Wandler erfolgt, der außerhalb des Hirnschädels oder der Wirbelsäule des Subjekts positioniert ist, für Beschallung dadurch; und
b) für die invasive Beschallung diese von mindestens einem chirurgisch implantierten Wandler in dem Hirnschädel oder in der Wirbelsäule des Subjekts erfolgt; und
c) für die Beschallung in Richtung des Herzens oder einer Spinal- oder Karotisarterie diese in dem Herzen oder in einer Spinal- oder Karotisarterie außerhalb des ZNS erfolgt;

(iv) weiteres Beschallen mit Ultraschall bei einer zweiten Frequenz, die niedriger als die erste Frequenz ist, und einem zweiten mechanischen Index, wobei die Extravasation des mindestens einen therapeutischen Mittels, das in Schritt (i) verabreicht wird, an dem Bereich von Interesse in dem ZNS ermöglicht wird; wobei

a) für die nicht-invasive Beschallung diese von mindestens einem Wandler erfolgt, der außerhalb des Hirnschädels oder der Wirbelsäule des Subjekts positioniert ist, für Beschallung dadurch; und
b) für die invasive Beschallung diese von mindestens einem chirurgisch implantierten Wandler in dem Hirnschädel oder in der Wirbelsäule des Subjekts erfolgt;

c) wenn gemäß c) in Schritt (iii) aktiviert wird, weitere Beschallung in diesem Schritt entweder nicht-invasiv oder invasiv gemäß Schritt (iv) a) oder Schritt (iv) b) erfolgt.

2. Pharmazeutische Zusammensetzung nach Anspruch 1 zur Verwendung nach Anspruch 1, wobei:

Schritt (iii) nicht-invasiv in Richtung des ZNS unter Verwendung der ersten Frequenz in einem Bereich von 0,2-3 MHz mit dem ersten MI von 0,1-0,4 durchgeführt wird; und/oder
Schritt (iv) nicht-invasiv in Richtung des ZNS unter Verwendung der zweiten Frequenz in einem Bereich von 0,2-0,4 MHz mit dem zweiten MI in einem Bereich von 0,025-0,15 oder unter Verwendung der zweiten Frequenz in einem Bereich von 0,4-0,6 MHz mit einem zweiten MI in einem Bereich von 0,1-0,3 durchgeführt wird.

3. Pharmazeutische Zusammensetzung nach Anspruch 1 zur Verwendung nach Anspruch 2, wobei die Ultraschallbeschallung nicht-invasiv entweder durch ein extrakranielles, hemisphärisch fokussiertes US-Array, einen extrakraniellen, fokussierten Mono-Element-US-Wandler oder von einem nasalen oder okularen Wandler durchgeführt wird.

4. Pharmazeutische Zusammensetzung nach Anspruch 1 zur Verwendung nach Anspruch 1, wobei:

die Ultraschallbeschallung invasiv in Richtung des ZNS in dem Schritt (iii) unter Verwendung der ersten Frequenz in einem Bereich von 1-10 MHz mit dem ersten MI in einem Bereich von 0,1-0,4 durchgeführt wird; und/oder
Schritt (iv) invasiv in Richtung des ZNS unter Verwendung der zweiten Frequenz in einem Bereich von 0,2-0,4 MHz mit dem zweiten MI in einem Bereich von 0,025 bis 0,15 oder unter Verwendung der zweiten Frequenz in einem Bereich von 0,4-0,6 MHz mit dem zweiten MI in einem Bereich von 0,1 bis 0,3 durchgeführt wird.

5. Pharmazeutische Zusammensetzung nach Anspruch 1 zur Verwendung nach Anspruch 4, wobei die Ultraschallbeschallung durch den Schädel oder die Wirbelsäule durch einen oder mehrere chirurgisch implantierte US-Wandler durchgeführt wird.

6. Pharmazeutische Zusammensetzung nach Anspruch 1 zur Verwendung nach Anspruch 1, wobei die Ultraschallbeschallung in Schritt (iii) in Richtung des Herzens oder der Spinal- oder Karotisarterien außerhalb des ZNS mit der ersten Frequenz in einem Bereich von 1-10 MHz mit dem ersten MI in einem Bereich von 0,1-0,4 durchgeführt wird, und wobei die Ultraschallbeschallung von Schritt iv) entweder nicht-invasiv oder invasiv in Richtung des ZNS unter Verwendung der zweiten Frequenz in einem Bereich von 0,2-0,4 MHz mit dem zweiten MI in einem Bereich von 0,025 bis 0,15 oder unter Verwendung der zweiten Frequenz in einem Bereich von 0,4-0,6 MHz mit dem zweiten MI in einem Bereich von 0,1 bis 0,3 durchgeführt wird.

7. Pharmazeutische Zusammensetzung nach Anspruch 1 zur Verwendung nach einem der Ansprüche 1 bis 6, wobei:

die Beschallung von Schritt (iii) unmittelbar nach Schritt (i) beginnt und unmittelbar von der Beschallung von Schritt (iv) gefolgt wird; und optional
die Beschallung von Schritt (iii) 30-120 Sekunden dauert, gefolgt von der Beschallung von Schritt (iv), die 3-10 Minuten dauert.

8. Pharmazeutische Zusammensetzung nach Anspruch 1 zur Verwendung nach einem der Ansprüche 1 bis 7, wobei 1 bis 5 therapeutische Mittel gleichzeitig oder sequenziell über eine bestimmte Zeitspanne verabreicht werden, wobei mindestens eine, wie 1 bis 5, ACT-Behandlungen während desselben Zeitraums durchgeführt werden.

9. Pharmazeutische Zusammensetzung nach Anspruch 1 zur Verwendung nach einem der Ansprüche 1 bis 8, wobei derselbe Breitband- oder Zweifrequenz-US-Wandler sowohl bei der Aktivierungsbeschallung von Schritt (iii) als auch bei der Verstärkungsbeschallung von Schritt (iv) verwendet wird.

10. Pharmazeutische Zusammensetzung nach Anspruch 1 zur Verwendung nach einem der Ansprüche 1 bis 9, wobei:

die Cluster einen mittleren Durchmesser in dem Bereich von 3-10 $\mu$m und vorzugsweise in dem Bereich von 4-9 $\mu$m aufweisen; und optional
die Cluster-Konzentration von Clustern in dem Größenbereich von 1-10 $\mu$m mindestens 25 Millionen/ml beträgt.

11. Pharmazeutische Zusammensetzung nach Anspruch 1 oder 10 zur Verwendung nach einem der Ansprüche 1 bis 9, wobei:

ein Gas der Mikrobläschen der Mikrobläschen-Mikrotröpfchen-Cluster Schwefelhexafluorid oder ein C3-6-Perfluorcarbon oder Mischungen davon umfasst; und optional

das Gas der Mikroblase aus der Gruppe von Schwefelhexafluorid, Perfluorpropan, Perfluorbutan, Perfluorpentan und Perfluorhexan oder einer Mischung davon ausgewählt ist, wobei die Mikroblase durch einen ersten Stabilisator stabilisiert ist, der aus der Gruppe von Phospholipiden, Proteinen und Polymeren ausgewählt ist; und die Ölphase der Mikrotröpfchen eine diffundierbare Komponente, die aus der Gruppe von Perfluorcarbonen, z. B. einem Perfluorcycloalkan, stabilisiert mit einem zweiten Stabilisator, ausgewählt aus der Gruppe von Tensiden, z. B. einschließlich Phospholipiden, Polymeren und Proteinen, ausgewählt ist.

12. Pharmazeutische Zusammensetzung nach Anspruch 1, 10 oder 11 zur Verwendung nach einem der Ansprüche 1 bis 14, wobei eine Ölphase des Mikrotröpfchens der Mikrobläschen-Mikrotröpfchen-Cluster einen teilweise oder vollständig halogenierten Kohlenwasserstoff oder eine Mischung davon umfasst.

13. Pharmazeutische Zusammensetzung nach einem der Ansprüche 1 und 10 bis 12 zur Verwendung nach einem der Ansprüche 1 bis 14, wobei das therapeutische Mittel aus der Gruppe der Arzneimittelklassen chemotherapeutische Mittel, immuntherapeutische Mittel, immunonkologische Mittel, immunmodulatorische Arzneimittel, Anti-B-Zell-Arzneimittel, entzündungshemmende Arzneimittel, antimikrobielle Arzneimittel, antiangiogene Arzneimittel, Antidepressiva, Antikonvulsiva, Cannabinoidarzneimittel, Tumornekrosefaktor-$\alpha$-Inhibitoren (TNF-Inhibitoren), Dopaminvorläufer, Catechol-O-Methyltransferase-Inhibitoren, Dopaminagonisten, Monoaminoxidase-B-Inhibitoren, Mantadin, Anticholinergika, Antikoagulanzien, Anti-Plättchen-Arzneimittel, Gewebe-Plasminogenaktivator (tPA)- und Cholinesterase-Inhibitoren ausgewählt ist.

14. Pharmazeutische Zusammensetzung nach einem der Ansprüche 1 und 10 bis 13 zur Verwendung nach einem der Ansprüche 1 bis 9, wobei das therapeutische Mittel in einem Trägerstoff, wie eingeschlossen in der Form von Liposomen, Mizellen, Konjugaten, Nanopartikeln, kernvernetzten polymeren Mizellen (CCPMs) oder Mikrohohlkugeln, oder ausgewählt aus der Gruppe von Genen, antimikrobiellen Peptiden, Stammzellen und Aptameren, formuliert ist; und optional wobei das therapeutische Mittel, oder eine formulierte Form des therapeutischen Mittels, ein Molekulargewicht von mehr als 500 Dalton aufweist.

15. Pharmazeutische Zusammensetzung nach einem der Ansprüche 1 und 10 bis 14 zur Verwendung nach einem der Ansprüche 1 bis 9, wobei die Cluster-Zusammensetzung in einem Zeitfenster von 3 Stunden ab Kombinieren einer ersten Komponente von Mikrobläschen mit einer zweiten Komponente von Mikrotröpfchen zum Herstellen der Mikrobläschen-Mikrotröpfchen-Cluster-Zusammensetzung verabreicht wird.

## Revendications

1. Composition pharmaceutique comprenant une composition de grappes de microbulles-microgouttelettes et au moins un agent thérapeutique pour utilisation dans une méthode de traitement de maladies, de troubles ou de lésions du système nerveux central (SNC) d'un sujet, dans laquelle l'utilisation comprend un traitement de thérapie par grappes acoustiques (ACT) dans laquelle au moins une étape d'insonation aux ultrasons est mise en œuvre soit de façon non invasive en direction du SNC, soit de façon invasive en direction du SNC, soit en direction du cœur ou d'une artère spinale ou carotide à l'extérieur du SNC, comprenant les étapes consistant à :

(i) administrer la composition pharmaceutique au sujet ; dans laquelle l'au moins un agent thérapeutique est pré-, et/ou co- et/ou post-administré séparément de la composition de grappes ;
(ii) imager facultativement les grappes de la composition pharmaceutique à l'aide d'une imagerie par ultrasons pour identifier une région d'intérêt dans le SNC pour un traitement au sein dudit sujet ;
(iii) activer un déphasage d'un composant diffusible de la microgouttelette de la composition de grappes provenant de l'étape (i) par insonation aux ultrasons à une première fréquence et un premier indice mécanique ; dans laquelle

a) pour l'insonation non invasive, celle-ci se déroule à partir d'au moins un transducteur positionné à l'extérieur du crâne cérébral ou de la colonne vertébrale du sujet pour insonation à travers celui-ci ou celle-ci ; et

b) pour l'insonation invasive, celle-ci se déroule à partir d'au moins un transducteur implanté chirurgicalement dans le crâne cérébral ou la colonne vertébrale du sujet ; et

c) pour l'insonation en direction du cœur ou d'une artère spinale ou carotide, celle-ci se déroule dans le cœur ou dans une artère spinale ou carotide à l'extérieur du SNC ;

(iv) effectuer une insonation supplémentaire avec des ultrasons à une seconde fréquence qui est inférieure à la première fréquence, et à un second indice mécanique, facilitant une extravasation de l'au moins un agent thérapeutique administré à l'étape (i) au niveau de la région d'intérêt dans le SNC ; dans laquelle

a) pour l'insonation non invasive, celle-ci se déroule à partir d'au moins un transducteur positionné à l'extérieur du crâne cérébral ou de la colonne vertébrale du sujet pour insonation à travers celui-ci ou celle-ci ; et

b) pour l'insonation invasive, celle-ci se déroule à partir d'au moins un transducteur implanté chirurgicalement dans le crâne cérébral ou la colonne vertébrale du sujet ;

c) lors d'une activation selon c) à l'étape (iii), l'insonation supplémentaire à cette étape se déroule soit de façon non invasive soit de façon invasive selon l'étape (iv) a) ou l'étape (iv) b).

2. Composition pharmaceutique selon la revendication 1 pour utilisation selon la revendication 1, dans laquelle :

l'étape (iii) est mise en œuvre de façon non invasive en direction du SNC à l'aide de la première fréquence dans une plage de 0,2 à 3 MHz avec le premier indice mécanique, MI, de 0,1 à 0,4 ; et/ou

l'étape (iv) est mise en œuvre de façon non invasive en direction du SNC à l'aide de la seconde fréquence dans une plage de 0,2 à 0,4 MHz avec le second MI dans une plage de 0,025 à 0,15, ou à l'aide de la seconde fréquence dans une plage de 0,4 à 0,6 MHz avec un second MI dans une plage de 0,1 à 0,3.

3. Composition pharmaceutique selon la revendication 1, pour utilisation selon la revendication 2, dans laquelle l'insonation aux ultrasons est mise en œuvre de façon non invasive soit par un réseau à ultrasons extra-crânien à focalisation hémisphérique, soit par un transducteur à ultrasons extra-crânien à mono-élément focalisé, soit par un transducteur nasal ou oculaire.

4. Composition pharmaceutique selon la revendication 1 pour utilisation selon la revendication 1, dans laquelle :

l'insonation aux ultrasons est mise en œuvre de façon invasive en direction du SNC dans l'étape (iii) à l'aide de la première fréquence dans une plage de 1 à 10 MHz avec le premier MI dans une plage de 0,1 à 0,4 ; et/ou

l'étape (iv) est mise en œuvre de façon invasive en direction du SNC à l'aide de la seconde fréquence dans une plage de 0,2 à 0,4 MHz avec le second MI dans une plage de 0,025 à 0,15, ou à l'aide de la seconde fréquence dans une plage de 0,4 à 0,6 MHz avec un second MI dans une plage de 0,1 à 0,3.

5. Composition pharmaceutique selon la revendication 1, pour utilisation selon la revendication 4 dans laquelle l'insonation aux ultrasons est mise en œuvre à travers le crâne ou la colonne vertébrale par un ou plusieurs transducteurs à ultrasons chirurgicalement implantés.

6. Composition pharmaceutique selon la revendication 1, pour utilisation selon la revendication 1, dans laquelle l'insonation aux ultrasons à l'étape (iii) est mise en œuvre en direction du cœur ou des artères spinales ou carotides à l'extérieur du SNC avec la première fréquence dans une plage de 1 à 10 MHz avec le premier MI dans une plage de 0,1 à 0,4, et dans laquelle l'insonation aux ultrasons de l'étape iv) est mise en œuvre soit de façon non invasive soit de façon invasive en direction du SNC à l'aide de la seconde fréquence dans une plage de 0,2 à 0,4 MHz avec le second MI dans une plage de 0,025 à 0,15, ou à l'aide de la seconde fréquence dans une plage de 0,4 à 0,6 MHz avec le second MI dans une plage de 0,1 à 0,3.

7. Composition pharmaceutique selon la revendication 1, pour utilisation selon l'une quelconque des revendications 1 à 6, dans laquelle :

l'insonation de l'étape (iii) démarre immédiatement après l'étape (i) et est suivie immédiatement par l'insonation de l'étape (iv) ; et facultativement

l'insonation de l'étape (iii) dure pendant 30 à 120 secondes, suivie par l'insonation de l'étape (iv) qui dure pendant 3 à 10 minutes.

**8.** Composition pharmaceutique selon la revendication 1, pour utilisation selon l'une quelconque des revendications 1 à 7, dans laquelle 1 à 5 agents thérapeutiques sont administrés simultanément ou séquentiellement sur un certain intervalle de temps dans laquelle au moins un, tel que 1 à 5, traitements ACT sont mis en œuvre pendant la même période.

**9.** Composition pharmaceutique selon la revendication 1, pour utilisation selon l'une quelconque des revendications 1 à 8, dans laquelle le même transducteur à ultrasons à large bande ou à double fréquence est utilisé à la fois dans l'insonation d'activation de l'étape (iii) et l'insonation de renforcement de l'étape (iv).

**10.** Composition pharmaceutique selon la revendication 1, pour utilisation selon l'une quelconque des revendications 1 à 9, dans laquelle :

les grappes ont un diamètre moyen dans la plage de 3 à 10 $\mu$m, et de préférence dans la plage de 4 à 9 $\mu$m ; et facultativement
la concentration en grappes des grappes dans la plage de taille de 1 à 10 $\mu$m est d'au moins 25 millions/ml.

**11.** Composition pharmaceutique selon la revendication 1 ou 10, pour utilisation selon l'une quelconque des revendications 1 à 9, dans laquelle :

un gaz des microbulles des grappes de microbulles-microgouttelettes comprend de l'hexafluorure de soufre ou un hydrocarbure perfluoré en C3-6 ; et facultativement
le gaz de la microbulle est choisi dans le groupe d'hexafluorure de soufre, perfluoropropane, perfluorobutane, perfluoropentane et perfluorohexane ou un mélange de ceux-ci, la microbulle est stabilisée par un premier stabilisant choisi dans le groupe phospholipides, protéines et polymères ; et
la phase huileuse des micro-gouttelettes comprend un composant diffusible choisi dans le groupe d'hydrocarbures perfluorés, par exemple un perfluorocycloalcane, stabilisé avec un second stabilisant choisi dans le groupe d'agents tensioactifs, par exemple comportant des phospholipides, des polymères et des protéines.

**12.** Composition pharmaceutique selon la revendication 1, 10 ou 11, utilisable selon l'une quelconque des revendications 1 à 14, dans laquelle une phase huileuse de la microgouttelette des grappes de microbulles-microgouttelettes comprend un hydrocarbure partiellement ou complètement halogéné ou un mélange de ceux-ci.

**13.** Composition pharmaceutique selon l'une quelconque des revendications 1 et 10 à 12, pour utilisation selon l'une quelconque des revendications 1 à 14, dans laquelle l'agent thérapeutique est choisi dans le groupe des classes de médicament agents chimiothérapeutiques, agents immunothérapeutiques, agents d'immuno-oncologie, médicaments immunomodulateurs, médicaments anti-cellules B, médicaments anti-inflammatoires, médicaments antimicrobiens, médicaments anti-angiogéniques, antidépresseurs, anticonvulsifs, médicaments cannabinoïdes, inhibiteurs de facteur de nécrose tumorale $\alpha$ (TNF), précurseurs de dopamine, inhibiteurs de catéchol-O-méthyltransférase, agonistes de dopamine, inhibiteurs de monoamine oxydase B, mantadine, anticholinergiques, anticoagulants, antiagrégants plaquettaires, activateur tissulaire de plasminogène (tPA) et inhibiteurs de cholinestérase.

**14.** Composition pharmaceutique selon l'une quelconque des revendications 1 et 10 à 13, pour utilisation selon l'une quelconque des revendications 1 à 9, dans laquelle l'agent thérapeutique est formulé dans un véhicule, tel qu'inclus sous la forme de liposomes, micelles, conjugués, nanoparticules, micelles polymère à noyau réticulé (CCPM) ou microsphères, ou est choisi dans le groupe de gènes, peptides antimicrobiens, cellules souches et aptamères ; et facultativement
dans laquelle l'agent thérapeutique, ou une forme formulée de l'agent thérapeutique, a une masse moléculaire de plus de 500 Daltons.

**15.** Composition pharmaceutique selon l'une quelconque des revendications 1 et 10 à 14, pour utilisation selon l'une quelconque des revendications 1 à 9, dans laquelle la composition de grappes est administrée dans une fenêtre temporelle de 3 heures à partir de la combinaison d'un premier composant de microbulles avec un second composant de micro-gouttelettes préparant la composition de grappes de microbulles-microgouttelettes.

Figure 1

A

B

129694

C

D

E

F

Figure 2

Figure 3

Figure 4 A

Figure 4 B

Figure 5

Figure 6

Figure 7

Figure 8

Figure 9

Figure 10

**Pre-Injection**

(a)

**Post-Injection: 17 sec**

(b)

Figure 10 continued

**Post-Injection: 1 min 9 sec**

(c)

**Post-Injection: 5 min 9 sec**

(d)

Figure 10 continued

**Post-Injection: 8 min 19 sec**

(e)

**Post-Injection: 8 min 45 sec**

(f)

Figure 10 continued

**Post-Injection: 8 min 56 sec**

(g)

(h)

Figure 11

Figure 12

Figure 13

Figure 14

Figure 15

Figure 16

Figure 17

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015047103 A

- WO 9953963 A

### Non-patent literature cited in the description

- **CHEN, K-TING et al.** Theranostic Strategy of Focused Ultrasound Induced Blood-Brain Barrier Opening for CNS Disease Treatment. *Frontiers in Pharmacology*, 2019, vol. 10
- **ÅSLUND et al.** Efficient enhancement of blood-brain barrier permeability using Acoustic Cluster Therapy (ACT). *Theranostics*, 2017, vol. 7, 23
- Acoustic Output Measurement Standard for Diagnostic Ultrasound Equipment. American Institute of Ultrasound in Medicine, 1998

- **BECCARIA, K. et al.** Blood-brain barrier disruption with low-intensity pulsed ultrasound for the treatment of paediatric brain tumours: a review and perspectives. *Neurosurg Focus*, 01 January 2020, vol. 48 (1)
- **POSTEMA** ; **SCHMITZ**. Ultrasonic bubbles in medicine: influence of the shell. *Ultrason Sonochem*, 2007, vol. 14 (4), 438-44
- **ANDERSEN et al.** A Harmonic Dual-Frequency Transducer for Acoustic Cluster Therapy. *Ultrasound Med Biol*, September 2019, vol. 45 (9), 2381-2390